# EUROPEAN PATENT APPLICATION

(11) **EP 2 423 685 A1**
(43) Date of publication of application: **29.02.2012**
(21) Application number: 11172241.9
(22) Date of filing: 05.12.2008
(51) Int. Cl.: G01N 33/53, C07K 14/705, C07K 16/28, A61K 45/00, A61P 25/28

(54) **Agent and method for diagnosis on the occurrence of Alzheimer's disease**

(30) Priority: 12.12.2007 US 13284 P
(62) Divisional of application: 08858648.2
(71) Applicant: Immuno-Biological Laboratories Co., Ltd., Takasaki-shi, Gunma 370-0831 (JP); National University Corporation Hokkaido University, Sapporo-shi, Hokkaido 060-0808 (JP)
(72) Inventor: Maeda, Masahiro, Takasaki-shi Gunma, 370-0831 (JP); Seito, Tsutomu, Takasaki-shi Gunma, 370-0831 (JP); Suzuki, Toshiharu, Sapporo-shi Hokkaido, 0600812 (JP); Hata, Saori, Sapporo-shi Hokkaido, 0600810 (JP)
(74) Representative: Ford, Hazel

(57) **Abstract**

Disclosed are: an agent, an apparatus and a method for the diagnosis of a disease associated with the abnormality in γ-secretase; a method for providing information for the diagnosis of a disease associated with the abnormality in γ-secretase; a method for monitoring the condition or the degree of progression of a disease associated with the abnormality in γ-secretase; and a method for determining the therapeutic effect of a therapeutic agent on a disease associated with the abnormality in γ-secretase. More specifically disclosed are: a diagnostic agent for a disease associated with the abnormality in γ-secretase, which comprises an antibody capable of recognizing a digestion product of an alcadein with α-secretase or γ-secretase or a fragment of the antibody; and others.

## Description

### Technical Filed

The present invention relates to the field of diagnosis of Alzheimer's disease. More specifically, the present invention relates to a method for detecting a mutated cleavage product of an alcadein peptide in a body fluid.

### Background Art

Alzheimer's disease (AD) is a neurodegenerative disease involving progressive deterioration of various cognitive functions. The pathology of Alzheimer's disease and a mechanism responsible therefor have become more evident, and the disease-modifying therapy has been developing. The progress in the development of therapy has increased the need for the early diagnosis/detection of Alzheimer's disease. In recent years, as a diagnostic marker for Alzheimer's disease, attention has been focused on an amyloid beta peptide (hereinafter, referred to as "A-beta") which is generated by cleavage of an amyloid beta protein precursor (hereinafter, referred to as "APP") by beta-secretase (beta-site APP-cleaving enzyme; hereinafter, referred to as "BACE") and cleavage of a C-terminal fragment of the beta-secretase cleaved products by gamma-secretase. The secreted A-beta can be detected from human cerebrospinal fluids and has been expected to be useful as a biomarker for Alzheimer's disease or as an endophenotype for the diagnosis or classification of Alzheimer's disease (see Non-Patent Documents 1, 2, and 3). Particularly, A-beta42, which terminates at alanine at residue 42, has been thought to be useful in the diagnosis of Alzheimer's disease, because of its increased production in familial Alzheimer's disease.

It has been reported that the concentration of A-beta 42 in the serum of Alzheimer's disease patients is elevated over the normal level before the onset and/or during the mild cognitive impairment stage of the disease (Non-Patent Document 4), which is then decreased to the normal level or lower due to the uptake of A-BETA peptides by cerebral vascular amyloid and cerebral parenchyma plaques (see Non-Patent Documents 2 and 5). Since the level of A-BETA42 seems to biphasically change according to the degree of progression of the disease, it has been known that A-BETA42 have a stability problem as a diagnostic marker for Alzheimer's disease.

Alcadeins (hereinafter, referred to as "Alc") are the family of evolutionarily conserved type I membrane proteins and are mainly expressed in neurons. The Alc family proteins were individually identified as a neuron-specific adaptor protein X11L-binding protein (see Non-Patent Document 6) and as a postsynaptic Ca²⁺-binding protein (also called calsyntenin) (see Non-Patent Document 7). In mammals, the following 4 Alc isomers have been reported: Alcₐₗₚₕₐ₁ (971 amino acids in humans), Alcₐₗₚₕₐ₂ (981 amino acids in humans), Alc_{beta} (956 amino acids in humans), and Alc_{gamma} (955 amino acids in humans) (see Non-Patent Document 6). Alcₐₗₚₕₐ, Alc_{beta}, and Alc_{gamma} are encoded by independent genes, whereas Alcₐₗₚₕₐ₁ and Alcₐₗₚₕₐ₂ are splicing mutants of the Alcₐₗₚₕₐ gene.

APP is colocalized with Alcₐₗₚₕₐ in brain neurons containing dystrophic neurites of senile plaques (see Non-Patent Document 6) and is a type I membrane protein that has been thought to play an important role in the onset of Alzheimer's disease (see Non-Patent Documents 8 and 9). APP successively undergoes processing by alpha-secretase or beta-secretase at the juxtamembrane site and processing by a gamma-secretase complex at the intermembrane site. Since A-BETA formed by the cleavage of APP by the combination of beta-secretase and gamma-secretase has been considered as a leading cause of Alzheimer's disease, the cleavage of APP has been studied continuously (see Non-Patent Documents 8 and 9).

Via interaction with X11L, Alc is stabilized and indirectly forms a complex with APP. Both Alc and APP in the trimer with X11L exhibit resistance to proteolytic metabolism (Non-Patent Document 6). The level of endogenous APP metabolites containing A-BETA is elevated in X11L-deficient mouse brains, suggesting that the interaction between APP and X11L physiologically takes place in the brain *in vivo* (see Non-Patent Document 10). Alc in the Alc-X11L-APP complex exhibits resistance to proteolytic digestion, whereas uncomplexed Alc has been reported to be cleaved by successive proteolytic digestions to form a short-chain peptide (see Non-Patent Document 11). It has been reported that: Alc is primarily cleaved in the extracellular juxtamembrane region to release the extracellular domain of Alc (soluble Alc: sAlc); the cell-associated carboxyl terminal fragment (hereinafter, referred to as "CTF") of an alcadein (Alc) after cleavage is cleaved by protease cleaving in intermembrane space; and both Alc cleavages coordinate with the corresponding reactions of APP (see Non-Patent Document 11).

ADAM10 and ADAM17 have been identified as alpha-secretases that destroy the A-BETA domain by cleaving the bond between lysine at residue 612 and leucine at residue 613 in APP₆₉₅. This cleavage in the juxtamembrane region has been reported to induce secondary juxtamembrane cleavage that secretes a 3-kDa non-amyloidogenic peptide (p3) consisting of 24 to 26 amino acids (see Non-Patent Document 8). However, no report has been made on an enzyme primarily cleaving Alc.

Since an Alcₐₗₚₕₐ-derived short-chain peptide corresponding to the APP p3 domain (hereinafter, this peptide is referred to as "p3-Alcₐₗₚₕₐ") is secreted by the cleavage of AlcCTF by gamma-secretase, presenilin (hereinafter, referred to as "PS")-dependent gamma-secretase has been reported to participate in the secondary cleavage of Alc (cleavage of AlcCTF). It has been known that presenilin is an enzyme component of the gamma-secretase complex, and that some mutations in PS1 and PS2 genes cause familial Alzheimer's disease. Some PS1 mutations linked to familial Alzheimer's disease have been known to change the cleavage site in APPCTF to promote the formation of A-BETA species extended at the C-terminus, such as A-BETA42 (see Non-Patent Documents 12, 13, 14, and 15).

As cleavage sites in Alc, it has been reported that: primary cleavage sites in Alcₐₗₚₕₐ are the peptide bond between methionine at residue 815 and alanine at residue 816, the peptide bond between glutamine at residue 820 and phenylalanine at residue 821, and the peptide bond between alanine at residue 838 and asparagine at residue 839; and secondary cleavage sites in Alcₐₗₚₕₐ are the peptide bond between proline at residue 842 and phenylalanine at residue 843, the peptide bond between phenylalanine at residue 843 and alanine at residue 844, and the peptide bond between threonine at residue 851 and valine at residue 852 (see Patent Document 1). It has also been reported that in cells expressing presenilin 1 (hereinafter, referred to as "PS1") having a substitution of leucine at residue 166 by proline as a mutation, the secondary cleavage sites were changed to the peptide bond between valine at residue 853 and valine at residue 854, the peptide bond between valine at residue 854 and isoleucine at residue 855, the peptide bond between isoleucine at residue 855 and valine at residue 856, and the peptide bond between valine at residue 856 and valine at residue 857 (see Patent Document 1). However, none of other PS1 mutants has been reported to change the cleavage sites, although a plurality of PS1 mutants have been reported. Antibodies capable of specifically recognizing p3'-Alc are difficult to prepare. Thus, in Patent Document 1, a FLAG-modified artificial peptide (AlcalphaΔE) was used in the identification of gamma-cleavage sites in Alc, instead of assay on p3-Alc. In addition, the successful collection of p3-Alc peptides has not been reported so far, and the structures of the peptides have been unknown.
Patent Document 1: International Publication No. WO 2005/044847
Non-Patent Document 1: Kanai, et al., (1998) Ann. Neurol. 44, 17-26.
Non-Patent Document 2: Graff-Radford, et al., (2007) Arch. Neurol. 64, 354-362.
Non-Patent Document 3: Kauwe, et al., (2007) Ann. Neurol., 61, 446-453.
Non-Patent Document 4: Mayeux, et al., (1999) Ann. Neurol. 46, 412-416.
Non-Patent Document 5: Sunderland (2003) JAMA 289, 2094-103.
Non-Patent Document 6: Araki, et al., (2003) J. Biol. Chem. 278, 49448-49458.
Non-Patent Document 7: Vogt, et al., (2001) Mol. Cell. Neurosci. 17, 151-166.
Non-Patent Document 8: Gandy, S (2005) J. Clin. Invest. 115, 1121-1129.
Non-Patent Document 9: Small, et al., (2006) Neuron 52, 15-31.
Non-Patent Document 10: Sano, et al., (2006) J. Biol. Chem. 281, 37853-37860.
Non-Patent Document 11: Araki, et al., (2004) J. Biol. Chem. 279, 24343-24354.
Non-Patent Document 12: Sherrington, et al., (1995) Nature 375, 754-760.
Non-Patent Document 13: Kwok, et al., (1997) Neuro Rep. 8, 1537-1542.
Non-Patent Document 14: Moehlmann, et al., (2002) Proc. Natl. Acad. Sci. USA, 99, 8025-8030.
Non-Patent Document 15: Devi, et al., (2000) Arch. Neurol. 57, 1454-1457.

### Disclosure of the Invention

It has been thought that ADAM10 is most likely to be an enzyme cleaving APP at the alpha-site (alpha-secretase) (Lammich, et al., (1999) Proc. Natl. Acad. Sci. USA 96, 3922-3927). Thus, the present inventors examined whether ADAM10 cleaves Alc family proteins Alcₐₗₚₕₐ, Alc_{beta}, and Alc_{gamma}. As a result, it was found that ADAM10 is a main enzyme digesting Alcₐₗₚₕₐ, Alc_{beta}, and Alcgₐₘₘₐ. It has been reported that another alpha-secretase ADAM17 cleaves APP (Buxbaum et al., (1998) J. Biol. Chem. 273, 27765-27767; Lammich et al., (1999) Proc. Natl. Acad. Sci. USA 96, 3922-3927; and Allinson et al., (2004) Eur. J. Biochem. 271, 2539-2547). Accordingly, the present inventors examined whether ADAM17 cleaves Alc family proteins Alcₐₗₚₕₐ. Alc_{beta}, and Alc_{gamma}. As a result, it was found that ADAM17 cleaves Alcₐₗₚₕₐ, Alc_{beta}, and Alc_{gamma}, as with APP. Specifically, the present inventors found that all of these 3 Alc family proteins (Alcₐₗₚₕₐ, Alc_{beta}, and Alc_{gamma}) are cleaved by ADAM10 and ADAM17 known as alpha-secretases of APP. Since Alc is mainly expressed in neurons, Alc and APP, when released from the complex with X11L, were demonstrated to undergo primary cleavage by the same enzymes in neurons.

The present inventors successfully prepared antibodies capable of specifically recognizing the juxtamembrane region of each Alc by variously manipulating the juxtamembrane region of the Alc as an antigen. By using the antibodies, the present inventors successfully collect, for the first time, gamma-secretase digestion products p3-Alcₐₗₚₕₐ, p3-Alc_{beta}, and p3-Alc_{gamma} from culture solutions of HEK293 cells expressing Alcₐₗₚₕₐ, Alc_{beta}, or Alc_{gamma}. Furthermore, the present inventors successfully identified primary and secondary cleavage sites in Alcₐₗₚₕₐ, Alc_{beta}, and Alc_{gamma} and determined the sequences of p₃-Alcₐₗₚₕₐ, p3-Alc_{beta}, and p3-Alc_{gamma} by analyzing the collected peptides by MALDI-MS/MS.

Likewise, the present inventors determined the sequence and length of p3-Alc in the presence of a PS1 mutation linked to familial Alzheimer's disease to confirm whether or not this mutation changes the cleavage sites and peptides formed thereby. Specifically, the present inventors determined the sequences and cleavage sites of the p3-Alcₐₗₚₕₐ, p3-Alc_{beta}, and p3-Alc_{gamma} peptides in culture solutions of cells expressing wild-type PS1 or each of 6 pathogenic PS1 mutants and in cerebrospinal fluids obtained from elderly humans without dementia and patients affected by sporadic Alzheimer's disease. In the cell culture experiment, the expressions of wild-type PS1 and some pathogenic PS1 mutants caused cleavage that forms p3-Alc species extended or shortened. Thus, the mutant PS1 was found to cause the speciation of the C-terminal fragments of p3-Alcₐₗₚₕₐ, p3-Alc_{beta}, and p3-Alc_{gamma}, in the same way as the speciation of A-BETA. The ratios of the abnormal p3-Alc species to normal p3-Alc were compared with the ratio of A-BETA42 to A-BETA40. As a result, it was found that the proportion of the secreted abnormal p3-Alc species increases in a coordinate manner with the ratio of A-BETA42 to A-BETA40. From these findings, the present inventors found that incorrect cleavage by gamma-secretase occurs not only in APP but also in Alc in familial Alzheimer's disease patients having mutant presenilin.

Thus, assuming that p3-Alc species in cerebrospinal fluids serve as surrogate markers for gamma-secretase functions and offer novel findings about sporadic Alzheimer's disease, the present inventors assayed p3-Alcₐₗₚₕₐ, p3-Alc_{beta}, and p3-Alc_{gamma} species in human cerebrospinal fluids. As a result, it was found that the speciation of p3-Alcₐₗₚₕₐ in sporadic Alzheimer's disease patients is different from that in healthy humans, and this change is close to that observed in the culture solutions of cells expressing mutant PS1 associated with familial Alzheimer's disease. From these results, the present inventors found that: the dysfunction of gamma-secretase is characteristic of both familial and sporadic Alzheimer's diseases; Alzheimer's disease can be diagnosed by assaying a gamma-secretase digestion product of Alc; and a therapeutic or preventive agent, particularly, a gamma-secretase-targeting therapeutic or preventive agent, for Alzheimer's disease can be evaluated for its efficacy by assaying a gamma-secretase digestion product of Alc.

The present inventors further successfully prepared an antibody which can specifically recognize a particular p3-Alc species (e.g., p3-Alcₐₗₚₕₐ35) among p3-Alc species having common sequences each other. The use of this antibody enables the particular p3-Alc species to be quantitatively assayed. Based on these findings or techniques, the present invention has been completed.

Thus, in one aspect, the present invention relates to a method for determining a likelihood of a patient having Alzheimer's disease or having the tendency to develop Alzheimer's disease, comprising determining the presence or absence of fragment(s) of an abnormally cleaved Alc protein in a body fluid analyte derived from the patient, wherein the presence of one or more of the fragment(s) means that the patient has Alzheimer's disease or has the tendency to develop Alzheimer's disease. In another aspect, the present invention relates to a diagnostic agent for detecting the presence or absence of a fragment of an abnormally cleaved Alc protein in a body fluid analyte derived from a patient.

In specific aspects, the present invention relates to the following embodiments (1) to (17):
(1) A diagnostic agent for a disease associated with the abnormality in gamma-secretase, comprising an antibody or a fragment thereof which recognizes an alpha-secretase and gamma-secretase digestion product of an alcadein.
(2) The diagnostic agent for a disease associated with the abnormality in gamma-secretase of (1), wherein the diagnostic agent is used for assaying the alpha-secretase and gamma-secretase digestion product of an alcadein in the body fluid of a subject.
(3) The diagnostic agent for a disease associated with the abnormality in gamma-secretase of (1) or (2), wherein the digestion product of an alcadein with alpha-secretase and gamma-secretase is a peptide consisting of the amino acid sequence of any one of SEQ ID NOs: 11 to 16 and SEQ ID NOs: 18 to 25.
(4) The diagnostic agent for a disease associated with the abnormality in gamma-secretase of any one of (1) to (3), wherein the antibody is an antibody which can recognize the amino acid sequence of SEQ ID NO: 5, the amino acid sequence of SEQ ID NO: 6, the amino acid sequence of SEQ ID NO: 7, the amino acid sequence of SEQ ID NO: 8, the amino acid sequence of SEQ ID NO: 9, the amino acid sequence of SEQ ID NO: 10, the amino acid sequence of SEQ ID NO: 26, or the amino acid sequence of SEQ ID NO: 27 as an epitope.
(5) The diagnostic agent of any one of (1) to (4), wherein the disease associated with the abnormality in gamma-secretase is Alzheimer's disease.
(6) A diagnostic apparatus for a disease associated with the abnormality in gamma-secretase comprising means of separating an alpha-secretase and gamma-secretase digestion product of an alcadein from an analyte and means of detecting information about the amino acid sequence of the separated alpha-secretase and gamma-secretase digestion product of an alcadein.
(7) A peptide consisting of the amino acid sequence of any one of SEQ ID NO selected from SEQ ID NOs: 11 to 16 and SEQ ID NOs: 18 to 25.
(8) An antibody capable of recognizing the amino acid sequence of any one of SEQ ID NO: 5, the amino acid sequence of SEQ ID NO: 6, the amino acid sequence of SEQ
   ID NO: 7, the amino acid sequence of SEQ ID NO: 8, the amino acid sequence of SEQ ID NO: 9, the amino acid sequence of SEQ ID NO: 10, the amino acid sequence of SEQ ID NO: 26, or the amino acid sequence of SEQ ID NO: 27 as an epitope.
(9) A diagnostic method for a disease associated with the abnormality in gamma-secretase, comprising the step of detecting an alpha-secretase and gamma-secretase digestion product of an alcadein in an analyte.
(10) A diagnostic method for a disease associated with the abnormality in gamma-secretase, comprising the following steps:
   a) preparing a sample derived from a subject;
   b) contacting the sample with an antibody or a fragment thereof which recognizes at least one alpha-secretase and gamma-secretase digestion product of an alcadein;
   c) determining the level of the alpha-secretase and gamma-secretase digestion product of an alcadein with by detecting the binding of the antibody or the fragment thereof to the alpha-secretase and gamma-secretase digestion product of an alcadein; and
   d) associating the level of the alpha-secretase and gamma-secretase digestion product of an alcadein with the presence or absence of or the severity of the disease associated with the abnormality in gamma-secretase in the subject.
(11) A diagnostic method for a disease associated with the abnormality in gamma-secretase, comprising the following steps:
   a) preparing a sample derived from a subject;
   b) separating an alpha-secretase and gamma-secretase digestion product of an alcadein;
   c) detecting information about the amino acid sequence of the separated alpha-secretase and gamma-secretase digestion product of an alcadein; and
   d) associating the level of the alpha-secretase and gamma-secretase digestion product of an alcadein with the presence or absence of or the severity of the disease associated with the abnormality in gamma-secretase in the subject.
(12) The diagnostic method for a disease associated with the abnormality in gamma-secretaseof any one of (9) to (11), wherein the alpha-secretase and gamma-secretase digestion product of an alcadein is a peptide consisting of the amino acid sequence of any one of SEQ ID NOs: 11 to 16 or SEQ ID NOs: 18 to 25.
(13) A diagnostic method for a disease associated with the abnormality in gamma-secretase, comprising the following steps:
   a) preparing a sample derived from a subject;
   b) contacting the sample with an antibody or a fragment thereof which recognizes a peptide consisting of the amino acid sequence of SEQ ID NO: 11 or 12 and with an antibody of a fragment thereof which recognizes a peptide consisting of the amino acid sequence of any one of SEQ ID NOs: 18 to 25;
   c) determining the levels of the peptide consisting of the amino acid sequence of SEQ ID NO: 11 or 12 and the peptide consisting of the amino acid sequence of any one of SEQ ID NOs: 18 to 25 by detecting the binding of the antibody or the fragment thereof to the peptide consisting of the amino acid sequence of SEQ ID NO: 11 or 12 and the peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
(14) A diagnostic method for a disease associated with the abnormality in gamma-secretase, comprising the following steps:
   a) preparing a sample derived from a subject;
   b) separating a peptide consisting of the amino acid sequence of SEQ ID NO: 11 or 12 and a peptide consisting of the amino acid sequence of any one of SEQ ID NOs: 18 to 25;
   c) detecting information about the amino acid sequences of the separated peptide consisting of the amino acid sequence of SEQ ID NO: 11 or 12 and the separated peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
(15) The diagnostic method for a disease associated with the abnormality in gamma-secretase of (11) or (12), further comprising the following steps:
   i) detecting amyloid beta42 and amyloid beta40 in the analyte;
   ii) calculating the ratio of the amyloid beta42 to the amyloid beta40 detected;
   iii) calculating the quantitative ratio between the peptide consisting of the amino acid sequence of any one of SEQ ID NOs: 18 to 25 and the peptide consisting of the amino acid sequence of SEQ ID NO: 11; and
   iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the severity of the disease associated with the abnormality in gamma-secretase in the subject.
(16) The diagnostic method for a disease associated with the abnormality in gamma-secretase of any one of (9) to (15), wherein the analyte is the body fluid of the subject.
(17) The diagnostic method for a disease associated with the abnormality in gamma-secretase of any one of (9) to (16), wherein the disease associated with the abnormality in gamma-secretase is Alzheimer's disease.

In another aspect, the present invention relates to the following embodiments (18) to (26):
(18) A method for gathering information for the diagnosis of a disease associated with the abnormality in gamma-secretase, comprising the step of detecting an alpha-secretase and gamma-secretase digestion product of an alcadein in an analyte.
(19) A method for gathering information for the diagnosis of a disease associated with the abnormality in gamma-secretase, comprising the following steps:
   a) preparing a sample derived from a subject;
   b) contacting the sample with an antibody of a fragment thereof which recognizes at least one alpha-secretase and gamma-secretase digestion product of an alcadein;
   c) determining the level of the alpha-secretase and gamma-secretase digestion product of an alcadein by detecting the binding of the antibody or the fragment thereof to the alpha-secretase and gamma-secretase digestion product of an alcadein; and
   d) associating the level of the alpha-secretase and gamma-secretase digestion product of an alcadein with the presence or absence of or the severity of the disease associated with the abnormality in gamma-secretase in the subject.
(20) A diagnostic method for a disease associated with the abnormality in gamma-secretase, comprising the following steps:
   a) preparing a sample derived from a subject;
   b) separating an alpha-secretase and gamma-secretase digestion product of an alcadein;
   c) detecting information about the amino acid sequence of the separated alpha-secretase and gamma-secretase digestion product of an alcadein; and
   c) associating the level of the alpha-secretase and gamma-secretase digestion product of an alcadein with the presence or absence of or the severity of the disease associated with the abnormality in gamma-secretase in the subject.
(21) The method for gathering information for the diagnosis of a disease associated with the abnormality in gamma-secretase of any one of (18) to (20), wherein the alpha-secretase and gamma-secretase digestion product of an alcadein is a peptide consisting of the amino acid sequence of any one of SEQ ID NOs: 11 to 16 or SEQ ID NOs: 18 to 25.
(22) A method for gathering information for the diagnosis of a disease associated with the abnormality in gamma-secretase, comprising the following steps:
   a) preparing a sample derived from a subject;
   b) contacting the sample with an antibody or a fragment thereof which recognizes a peptide consisting of the amino acid sequence of SEQ ID NO: 11 or 12 and with an antibody or fragment thereof which recognizes a peptide consisting of the amino acid sequence of any one of SEQ ID NOs: 18 to 25 or a fragment of the antibody;
   c) determining the levels of the peptide consisting of the amino acid sequence of SEQ ID NO: 11 or 12 and the peptide consisting of the amino acid sequence of any one of SEQ ID NOs: 18 to 25 by detecting the bindings of the antibodies or the fragments to the peptide consisting of the amino acid sequence of SEQ ID NO: 11 or 12 and the peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
(23) A method for gathering information for the diagnosis of a disease associated with the abnormality in gamma-secretase, comprising the following steps:
   a) preparing a sample derived from a subject;
   b) separating a peptide consisting of the amino acid sequence of SEQ ID NO: 11 or 12 and a peptide consisting of the amino acid sequence of any one of SEQ ID NOs: 18 to 25;
   c) detecting information about the amino acid sequences of the separated peptide consisting of the amino acid sequence of SEQ ID NO: 11 or 12 and the separated peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of by SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
(24) The method for gathering information for the diagnosis of a disease associated with the abnormality in gamma-secretase of (22) or (23), further comprising the following steps:
   i) detecting amyloid beta42 and amyloid beta40 in the analyte;
   ii) calculating the ratio of the amyloid beta42 to the amyloid beta40 detected;
   iii) calculating the quantitative ratio between the peptide consisting of the amino acid sequence of any one of SEQ ID NOs: 18 to 25 and the peptide consisting of the amino acid sequence of SEQ ID NO: 11; and iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the severity of the disease associated with the abnormality in gamma-secretase in the subject.
   (25) The method for gathering information for the diagnosis of a disease associated with the abnormality in gamma-secretase of any one of (18) to (24), wherein the analyte is the body fluid of the subject.
(26) The method for gathering information for the diagnosis of a disease associated with the abnormality in gamma-secretase of any one of (18) to (25), wherein the disease associated with the abnormality in gamma-secretase is Alzheimer's disease.

In further alternative aspects, the present invention relates to the following embodiments (27) to (35) :
(27) A method for screening a therapeutic or preventive agent for a disease associated with the abnormality in gamma-secretase, comprising the step of detecting a alpha-secretase and gamma-secretase in an analyte digestion product of an alcadein.
(28) A method for screening a therapeutic or preventive agent for a disease associated with the abnormality in gamma-secretase, comprising the following steps:
   a) contacting a test agent with a cell expressing murated gamma-secretase;
   b) preparing a culture solution of the cell as a sample;
   c) contacting the sample with an antibody of a fragment thereof recognizeds at least one alpha-secretase and gamma-secretase digestion product of an alcadein;
   d) determining the level of the alpha-secretase and gamma-secretase digestion product of an alcadein by detecting the binding of the antibody or the fragment thereof to the alpha-secretase and gamma-secretase digestion product of an alcadein; and
   e) associating the level of the alpha-secretase and gamma-secretase digestion product of an alcadein with the presence or absence of or the degree of the therapeutic or preventive effect of the test agent on the disease associated with the abnormality in gamma-secretase.
(29) A method for screening a therapeutic or preventive agent for a disease associated with the abnormality in gamma-secretase, comprising the following steps:
   a) administering a test agent to an animal or a human having the abnormality in gamma-secretase;
   b) preparing the body fluid of the recipient as a sample;
   c) contacting the sample with an antibody or a fragment thereof which recognizes at least one alpha-secretase and gamma-secretase digestion product of an alcadein;
   d) determining the level of the alpha-secretase and gamma-secretase digestion product of an alcadein by detecting the binding of the antibody or the fragment thereof to the alpha-secretase and gamma-secretase digestion product of an alcadein; and
   d) associating the level of the alpha-secretase and gamma-secretase digestion product of an alcadein with the presence or absence of or the degree of the therapeutic or preventive effect of the test agent on the disease associated with the abnormality in gamma-secretase.
(30) A method for screening a therapeutic or preventive agent for a disease associated with the abnormality in gamma-secretase, comprising the following steps:
   a) contacting a test agent with a cell expressing mutated gamma-secretase;
   b) preparing a culture solution of the cell as a sample;
   c) separating a alpha-secretase and gamma-secretase digestion product of an alcadein;
   d) detecting information about the amino acid sequence of the separated alpha-secretase and gamma-secretase digestion product of an alcadein; and
   e) associating the level of the alpha-secretase and gamma-secretase digestion product of an alcadein with the presence or absence of or the degree of the therapeutic or preventive effect of the test agent on the disease associated with the abnormality in gamma-secretase.
(31) A method for screening a therapeutic or preventive agent for a disease associated with the abnormality in gamma-secretase, comprising the following steps:
   a) administering a test agent to an animal or a human having the abnormality in gamma-secretase;
   b) preparing the body fluid of the recipient as a sample;
   c) separating a alpha-secretase and gamma-secretase digestion product of an alcadein;
   d) detecting information about the amino acid sequence of the separated alpha-secretase and gamma-secretase digestion product of an alcadein; and
   e) associating the level of the alpha-secretase and gamma-secretase digestion product of an alcadein with the presence or absence of or the degree of the therapeutic or preventive effect of the test agent on the disease associated with the abnormality in gamma-secretase.
(32) The method for screening a therapeutic or preventive agent for a disease associated with the abnormality in gamma-secretase of any one of (27) to (31), wherein the alpha-secretase and gamma-secretase digestion product of an alcadein is a peptide consisting of the amino acid sequence of any one of SEQ ID NOs: 11 to 16 or SEQ ID NOs: 18 to 25.
(33) The method for screening a therapeutic or preventive agent for a disease associated with the abnormality in gamma-secretase of (32), wherein the step of associating the level of the alpha-secretase and gamma-secretase digestion product of an alcadein with the presence or absence of or the degree of the therapeutic or preventive effect of the test agent on the disease associated with the abnormality in gamma-secretase comprises the steps of:
   e-1) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e-2) associating the calculated ratio with the presence or absence of or the degree of the therapeutic or preventive effect of the test agent on the disease associated with the abnormality in gamma-secretase.
(34) The method for screening a therapeutic or preventive agent for a disease associated with the abnormality in gamma-secretase of (32) or (33), further comprising the following steps:
   i) detecting amyloid beta42 and amyloid beta40 in the analyte;
   ii) calculating the ratio of the amyloid beta42 to the amyloid beta40 detected;
   iii) calculating the quantitative ratio between the peptide consisting of the amino acid sequence of any one of SEQ ID NOs: 18 to 25 and the peptide consisting of the amino acid sequence of SEQ ID NO: 11; and iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the degree of the therapeutic or preventive effect of the test agent on the disease associated with the abnormality in gamma-secretase. (35) The method for screening a therapeutic or preventive agent for a disease associated with the abnormality in gamma-secretase of any one of (27) to (34), wherein the disease associated with the abnormality in gamma-secretase is Alzheimer's disease.

In the present specification, "alcadein" is a collective name for alcadein family proteins which are type I membrane proteins and also known as neuron-specific adaptor protein X11L-binding proteins or as postsynaptic Ca²⁺-binding proteins (also called calsyntenin), and encompasses Alcₐₗₚₕₐ, Alc_{beta}, and Alc_{gamma}. Alcadein may mean any of Alcₐₗₚₕₐ, Alc_{beta}, and Alc_{gamma} proteins or may mean all of these proteins. For example, alcadein may be Alcₐₗₚₕₐ.

In the present specification, the "alpha-secretase" refers to a protease involved in the primary cleavage of APP. Examples of the alpha-secretase can include ADAM10 and ADAM17. The alpha-secretase cleaves APP and Alc in their extracellular juxtamembrane regions. In the present specification, the site at which APP or Alc is digested with alpha-secretase is referred to as an "alpha-site". Alc is cleaved by alpha-secretase and digested into N-terminal and C-terminal fragments. The N-terminal fragment formed by the digestion of Alc with alpha-secretase is released from cell surface and secreted. In the present specification, the N-terminal fragment is referred to as soluble Alc or sAlc. On the other hand, the C-terminal fragment formed by the digestion with alpha-secretase has a transmembrane site and therefore remains on the cell membrane. In the present specification, this C-terminal fragment is referred to as "CTF".

In the present specification, the "gamma-secretase" refers to a protease involved in the secondary cleavage of APP. Examples of the gamma-secretase can include presenilin. The presenilin encompasses presenilin 1 which is located on chromosome 14 and presenilin 2 which is mapped to chromosome 1 and is responsible for Volga-German families or the like. The presenilin is a membrane protein having 6 to 8 transmembrane domains. Digestion with gamma-secretase is induced by the digestion with alpha-secretase. The gamma-secretase cleaves APP and Alc at their intermembrane sites. In the present specification, the site at which APP or Alc is digested with gamma-secretase is referred to as a "gamma-site". Alc is cleaved by gamma-secretase and digested into N-terminal and C-terminal fragments. The N-terminal fragment formed by the digestion of Alc with gamma-secretase is released from cell surface and secreted. In the present specification, this N-terminal fragment is referred to as p3-Alc.

In the present specification, "alpha-secretase and gamma-secretase digestion product of an alcadein" refers to a digestion product formed by the digestion of Alc with alpha-secretase and gamma-secretase and specifically refers to a peptide resulting from the secondary gamma-secretase cleavage of AlcCTF which is formed by the primary cleavage of Alc by alpha-secretase. The alpha-secretase and gamma-secretase digestion product of an alcadein is synonymous with the p3-Alc. Since a plurality of sites are present as alpha-secretase cleavage and gamma-secretase cleavage sites in an alcadein, the alpha-secretase and gamma-secretase digestion product of an alcadein includes plural types. Specific examples thereof can include: p3-Alcₐₗₚₕₐ peptides having the amino acid sequence of SEQ ID NO: 11, 12, 18, 19, 20, 21, 22, 23, 24, or 25; p3-Alc_{beta} peptides having the amino acid sequence of SEQ ID NO: 13 or 14; and p3-Alc_{gamma} peptides having the amino acid sequence of SEQ ID NO: 15 or 16. In the description below, such various p3-Alc fragments are collectively referred to as "p3-Alc species".

The present invention encompasses an antibody which recognizes an alpha-secretase and gamma-secretase digestion product of an alcadein. In this context, the "antibody which recognizes a alpha-secretase and gamma-secretase digestion product of an alcadein" is not particularly limited as long as the antibody which recognizes p3-Alc. The antibody is preferably an antibody which specifically recognizes p3-Alc, more preferably an antibody which specifically recognizes p3-Alcₐₗₚₕₐ, p3-Alc_{beta}, or p3-Alc_{gamma}, even more preferably an antibody which specifically recognizes p3-Alcₐₗₚₕₐ34, p3-Alcₐₗₚₕₐ35, p3-Alcₐₗₚₕₐ36, p3-Alcₐₗₚₕₐ37, p3-Alcₐₗₚₕₐ38, or p3-Alcₐₗₚₕₐ39.

The p3-Alc recognized by the antibody of the present invention is not particularly limited by its origin and may be mammalian p3-Alc, for example, mouse, rat, hamster, rabbit, or human p3-Alc, preferably human p3-Alc. Examples of the antibody which recognizes a alpha-secretase and gamma-secretase digestion product of an alcadein can include antibodies which recognize the amino acid sequence of SEQ ID NO: 5, the amino acid sequence of SEQ ID NO: 6, the amino acid sequence of SEQ ID NO: 7,
the amino acid sequence of SEQ ID NO: 8, the amino acid sequence of SEQ ID NO: 9, the amino acid sequence of SEQ ID NO: 10, the amino acid sequence of SEQ ID NO: 26, or the amino acid sequence of SEQ ID NO: 27 as an epitope. Examples of the antibody which recognizes a alpha-secretase and gamma-secretase digestion product of an alcadein can include antibodies obtained using, as an antigen, a peptide having the amino acid sequence of SEQ ID-NO: 5, a peptide having the amino acid sequence of SEQ ID NO: 6, a peptide having the amino acid sequence of SEQ ID NO: 7, a peptide having the amino acid sequence of SEQ ID NO: 8, a peptide having the amino acid sequence of SEQ ID NO: 9, a peptide having the amino acid sequence of SEQ ID NO: 10, a peptide having the amino acid sequence of SEQ ID NO: 26, or a peptide having the amino acid sequence of SEQ ID NO: 27.

The antibody of the present invention encompasses non-human animal antibodies, antibodies having the amino acid sequence of a non-human animal antibody and the amino acid sequence of a human-derived antibody, and human antibodies. Examples of the non-human animal antibodies can include mouse, rat, hamster, and rabbit antibodies. Preferably, the non-human animal antibodies are antibodies of animals from which hybridomas can be prepared, more preferably mouse antibodies. Examples of the antibodies having the amino acid sequence of a non-human animal antibody and the amino acid sequence of a human-derived antibody can include: human chimeric antibodies comprising a human antibody having the antigen-binding domain Fv of an animal-derived monoclonal antibody in place of the original one; and humanized antibodies comprising the CDRs (sequences on the Fv domain directly involved in antigen binding) of an animal-derived monoclonal antibody grafted into frameworks of a human antibody. The human antibodies refer to human antibodies as expression products of antibody genes completely derived from humans. The antibody of the present invention is not particularly limited by its immunoglobulin class and may belong to any of IgG, IgM, IgA, IgE, IgD, and IgY immunoglobulin classes, preferably, IgG. Furthermore, the antibody of the present invention encompasses antibodies of any isotype. The antibody of the present invention may be a polyclonal antibody or may be a monoclonal antibody.

The "fragment of the antibody which recognizes a alpha-secretase and gamma-secretase digestion product of an alcadein" is not particularly limited as long as it is a fragment of the "antibody which recognizes an alpha-secretase and gamma-secretase digestion product of an alcadein ". In this context, the "fragment of the antibody" refers to a portion of the antibody (partial fragment) or a peptide containing a portion of the antibody, which maintains the binding effect of the antibody to the antigen. Examples of such a fragment of the antibody can include F(ab')₂, Fab', Fab, single chain Fv (hereinafter, referred to as "scFv"), disulfide-stabilized Fv (hereinafter, referred to as "dsFv"), or polymers thereof, and dimerized V region (hereinafter, referred to as "Diabody"), and a CDR-containing peptide. The F(ab')₂ refers to an antibody fragment of approximately 100,000 in molecular weight having antigen-binding activity among fragments obtained by the protease pepsin treatment of IgG. The Fab' refers to an antibody fragment of approximately 50,000 in molecular weight having antigen-binding activity, which is obtained by cleaving the disulfide bond of the hinge region in the F(ab') ₂ fragment. The sdFv refers to a polypeptide which comprises one VH domain and one VL domain linked via a peptide linker and has antigen-binding activity. The dsFv refers to a fragment which comprises VH and VL domains linked via the disulfide bond between cysteine residues which are replaced and has antigen-binding activity. The Diabody refers to a fragment obtained by dimerizing scFvs. The Diabody of the present invention may be monospecific or may be bispecific (polyspecific antibody). The scFvs in this dimer may be the same as or different from each other. The CDR-containing peptide refers to a peptide containing the amino acid sequence of at least one CDR selected from heavy chain variable region CDR1, CDR2, and CDR3 and light chain variable region CDR1, CDR2, and CDR3.

The "disease associated with the abnormality in gamma-secretase" is not particularly limited as long as the disease is caused by the abnormality in gamma-secretase. The abnormality in gamma-secretase may be dysfunction or may be change in cleavage site (gamma-site). Examples of the disease associated with the abnormality in gamma-secretase can include Alzheimer's disease.

### Brief Description of the Drawings

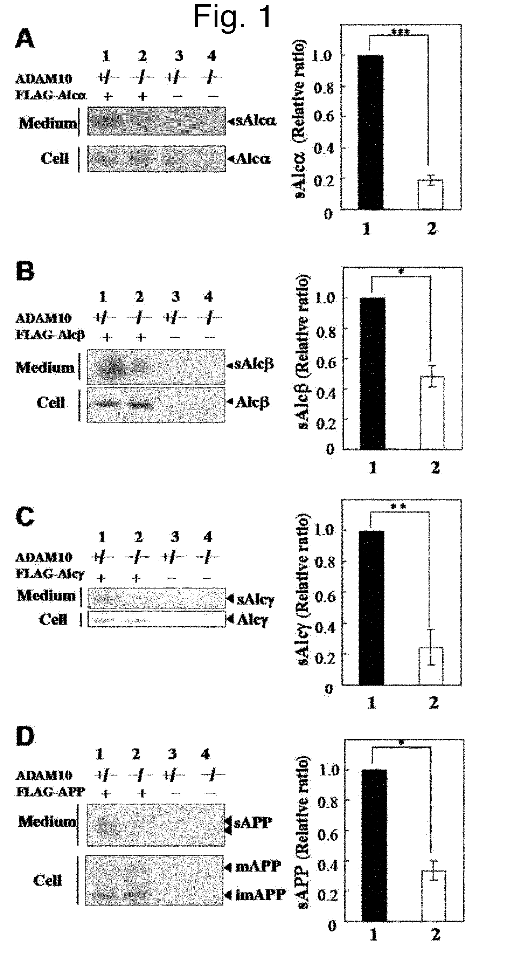

Figure 1 shows western blotting results of FLAG-labeled Alcₐₗₚₕₐ, Alc_{beta}, Alc_{gamma} or APP in culture solutions or cell lysates of ADAM10 gene homozygous knockout mouse (-/-)-derived mouse embryonic fibroblasts and ADAM10 gene heterozygous knockout mouse (+/-)-derived mouse embryonic fibroblasts , and graphs showing the proportion of sAlc (digestion product secreted into the culture solution) in the lane 2 to sAlc (digestion product secreted into the culture solution) in the lane 1 as 1. The western blot images show the results of Alcₐₗₚₕₐ (Figure 1A), Alc_{beta} (Figure 1B), Alc_{gamma} (Figure 1C), and APP (Figure 1D). sAlc and sAPP represent digestion products of Alc and APP secreted after cleavage respectively. "Medium" represents sAlc or sAPP secreted into the culture solution, and "Cell" represents intracellular Alc or APP.

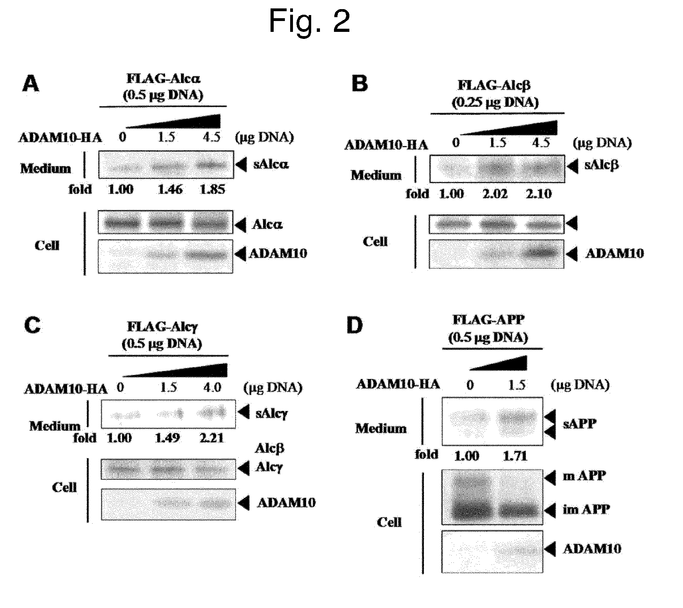

Figure 2 shows western blotting results of Alcₐₗₚₕₐ, Alc_{beta}, Alc_{gamma}, or APP in culture solutions or cell lysates of ADAM10 gene homozygous knockout mouse (-/-)-derived mouse embryonic fibroblasts expressing various levels of ADAM10. Figure 2A shows the results of Alcₐₗₚₕₐ, Figure 2B shows the results of Alc_{beta}, Figure 2C shows the results of Alc_{gamma}, and Figure 2D shows the results of APP. "Medium" represents sAlc or sAPP secreted into the culture solution, and "Cell" represents intracellular Alc or APP and intracellular ADAM10 expression. "Fold" represents the ratio of amount of sAlc or sAPP secreted by the ADAM10-HA gene-transfected cells to the amount of sAlc or sAPP secreted by ADAM10-HA gene-untransfected cells (0 microg DNA) as 1.

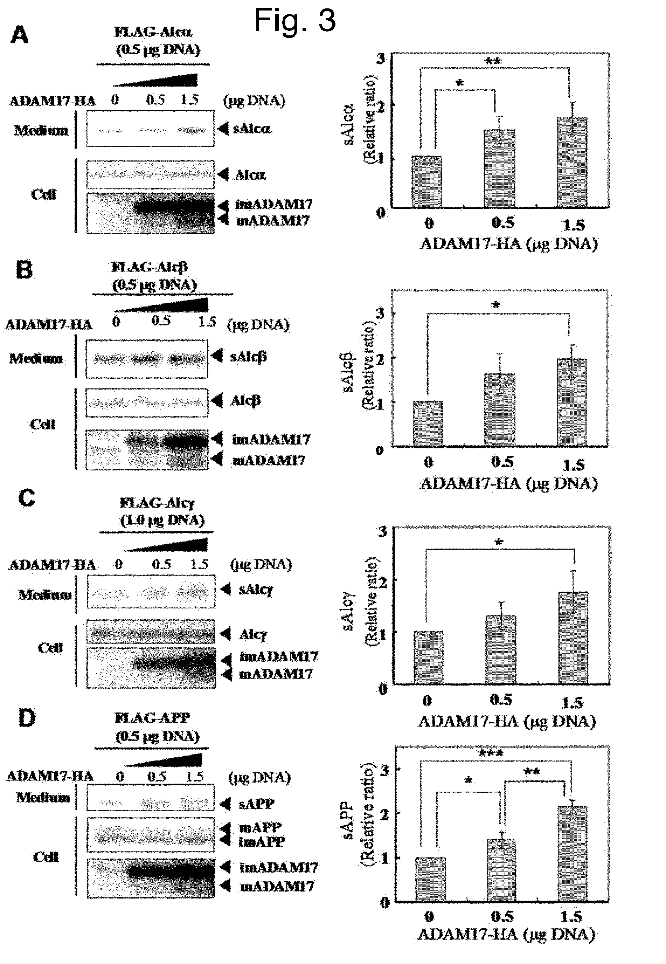

Figure 3 shows western blotting results of Alcₐₗₚₕₐ, Alc_{beta}, Alc_{gamma}, or APP in culture solutions or cell lysates of N2a cells expressing various levels of ADAM17 (left diagrams), amd graphs showing the ratio of amount of sAlc or sAPP secreted by the ADAM17-HA gene-transfected cells to the amount of sAlc or sAPP secreted by ADAM17-HA gene-untransfected cells (0 microg DNA) as 1 (right diagrams). Figure 3A shows the results of Alcₐₗₚₕₐ, Figure 3B shows the results of Alc_{beta}, Figure 3C shows the results of Alc_{gamma}, and Figure 3D shows the results of APP. "Medium" represents sAlc or sAPP secreted into the culture solution, and "Cell" represents intracellular Alc or APP and intracellular ADAM17 expression.

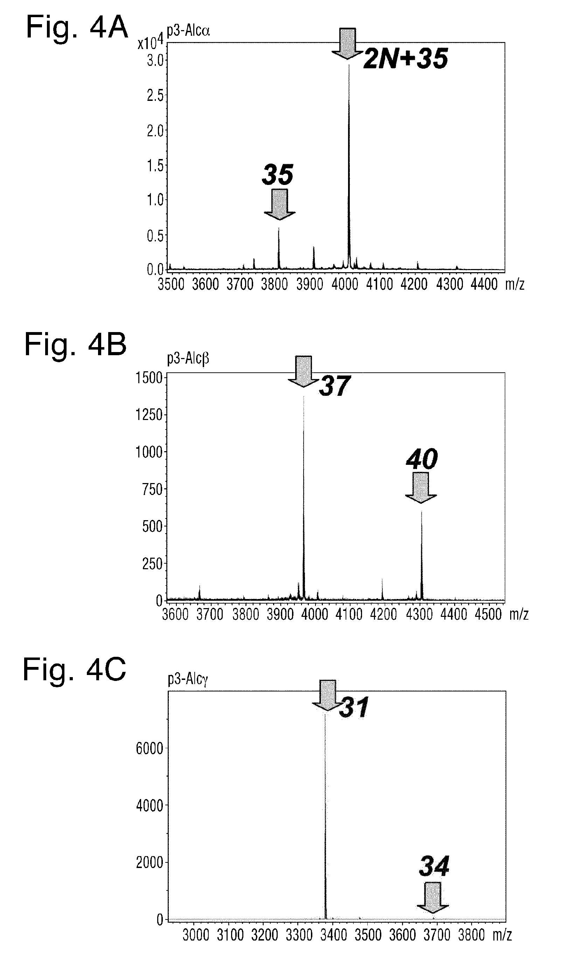

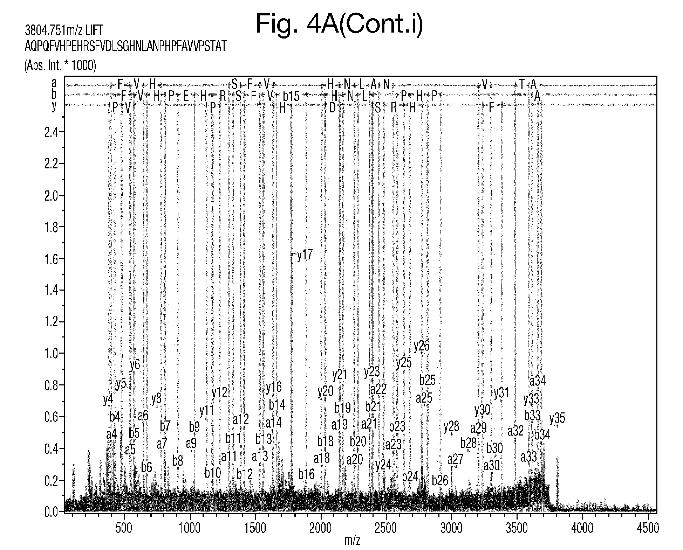

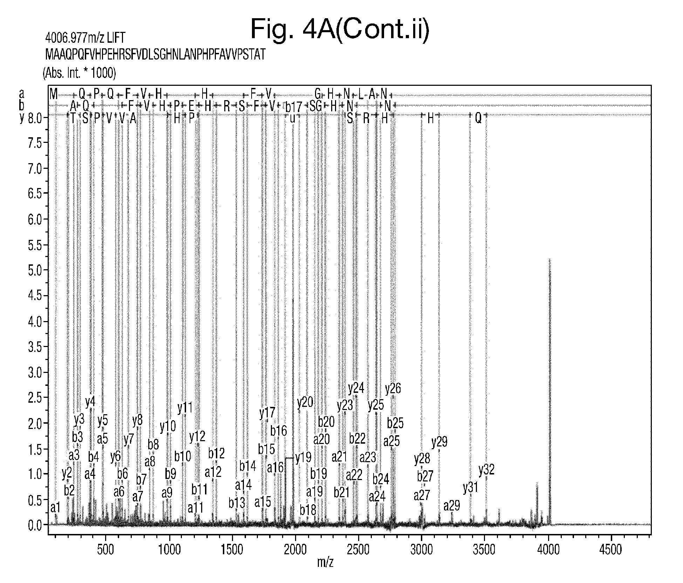

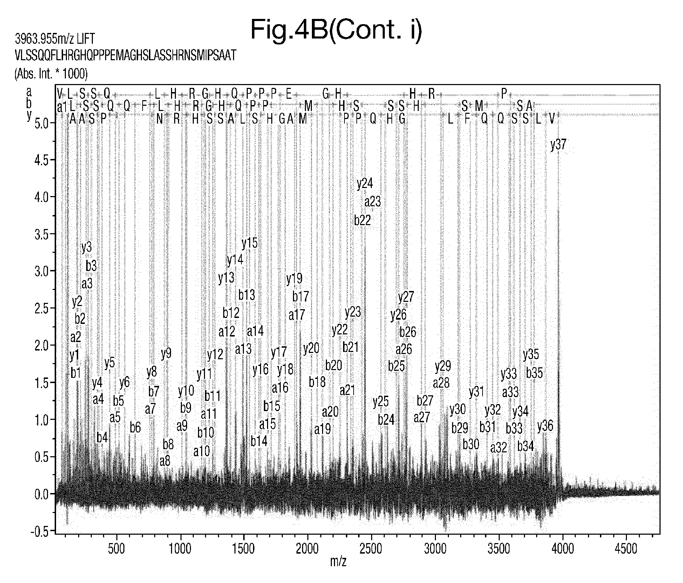

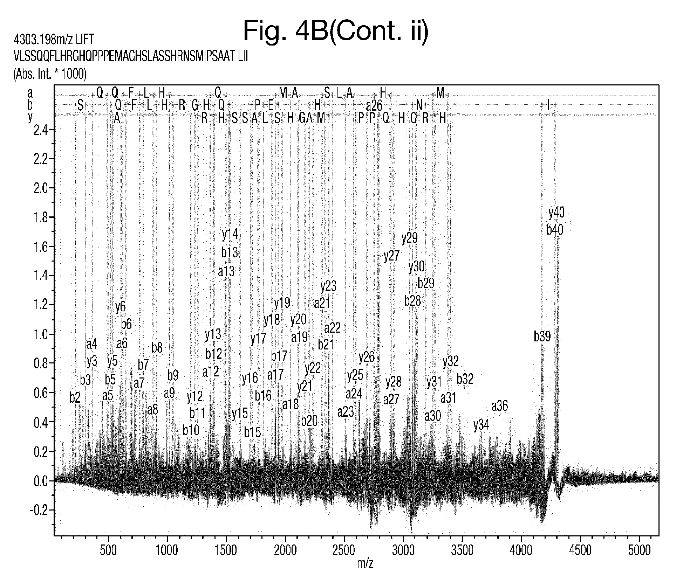

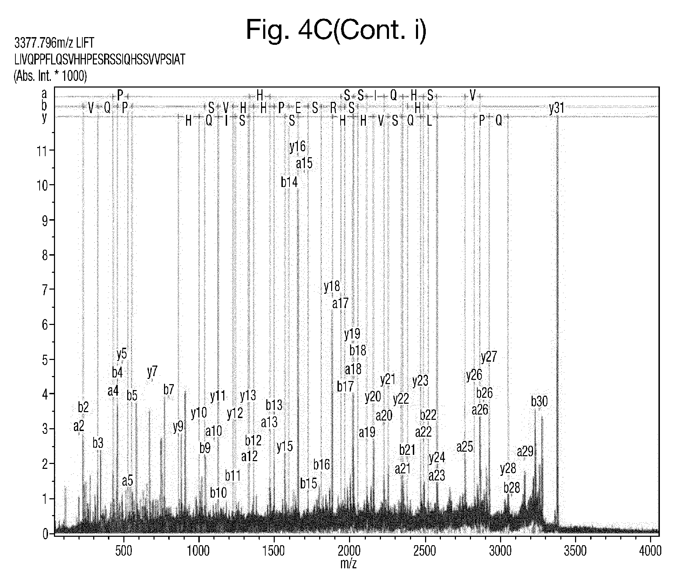

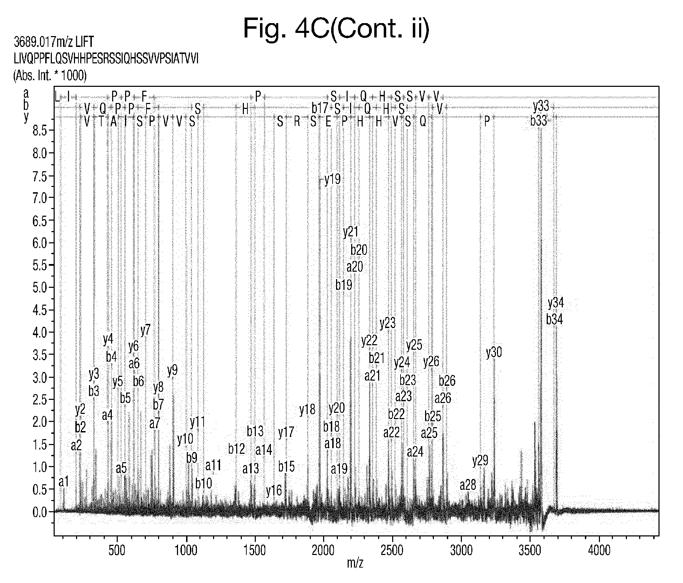

Figure 4 shows mass spectrometry results of extracellularly secreted p3-Alcₐₗₚₕₐ, p3-Alc_{beta}, and p3-Alc_{gamma}. Figure 4A shows the results of Alcₐₗₚₕₐ, Figure 4B shows the results of Alc_{beta}, and Figure 4C shows the results of Alc_{gamma}. The left diagrams show the results of MALDI-TOF/TOF analysis for determining the molecular weights. The arrows "35" and "2N+35" in Figure 4A represent p3-Alcₐₗₚₕₐ35 and p3-Alcₐₗₚₕₐ2N+35, respectively. The arrows "37" and "40" in Figure 4B represent p3-Alc_{beta}37 and p3-Alc_{beta}40, respectively. The arrows "31" and "34" in Figure 4C represent p3-Alc_{gamma}31 and p3-Alc_{gamma}34, respectively. The middle and right diagrams show the results of further analysys of the signals indicated with the arrows in the corresponding left diagrams by MALDI-MS/MS. The signal indicated with the arrow "34" in the left diagram of Figure 4C was weak. Therefore, as for p3-Alc_{gamma}34 of the right diagram of Figure 4C shows results of analysys of p3-Alc_{gamma}34 obtained from HEK293 cells expressing a L166P mutant by MALDI-MS/MS.

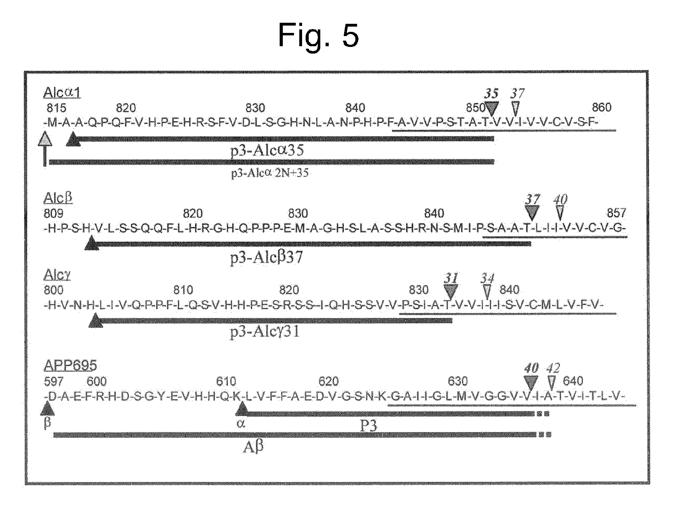

Figure 5 shows, from the top, the amino acid sequences of AlCₐₗₚₕₐ₁, Alc_{beta}, Alc_{gamma}, and APP in the neighborhood of p3. The up-pointing arrows under the sequences represent primary cleavage sites that is alpha-sites for Alcₐₗₚₕₐ₁, Alc_{beta}, and Alc_{gamma} and alpha-site (indicated with "alpha") and beta-site (indicated with "beta") for APP. The down-pointing arrows above the sequences represent secondary cleavage sites (gamma-sites). The double lines below the sequences show the amino acid sequences of p3-Alcₐₗₚₕₐ35, p3-Alcₐₗₚₕₐ2N+35, p3-Alc_{beta}37, p3-Alc_{gamma}31, A-BETA, and p3. The single lines below the sequences represent transmembrane sites.

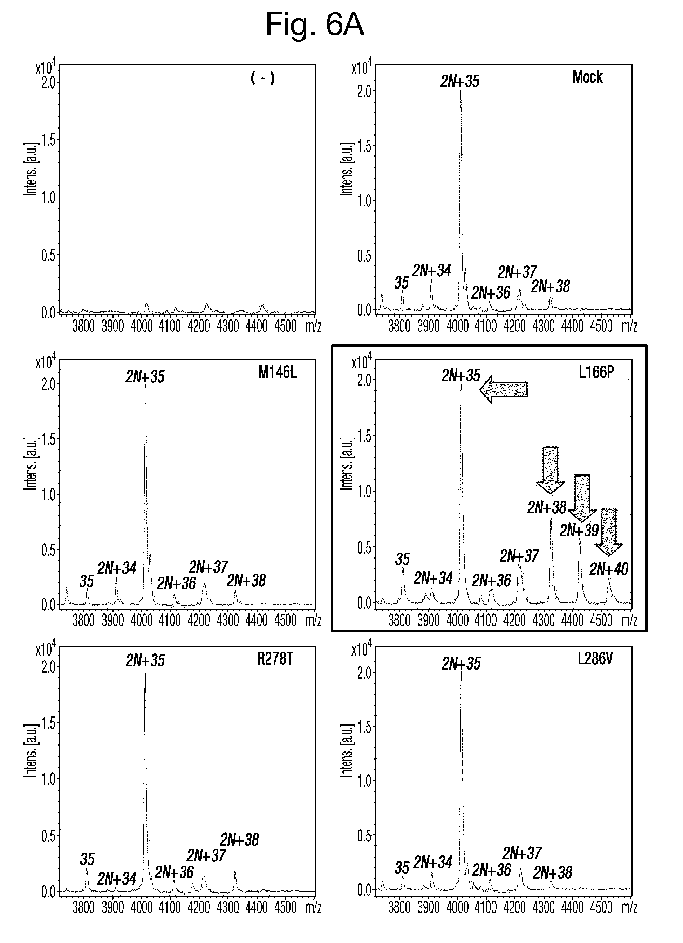

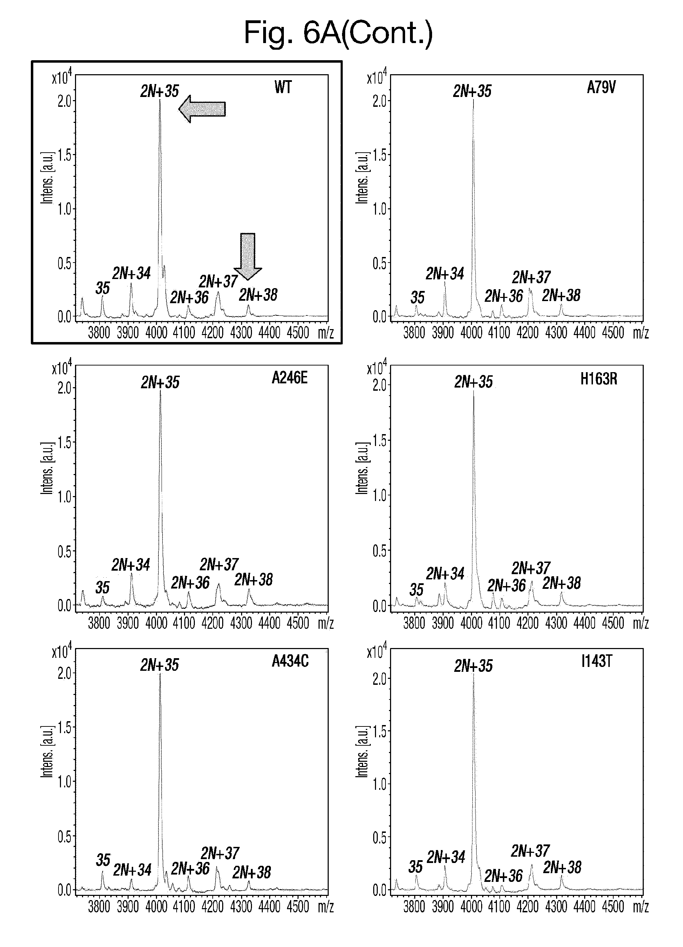

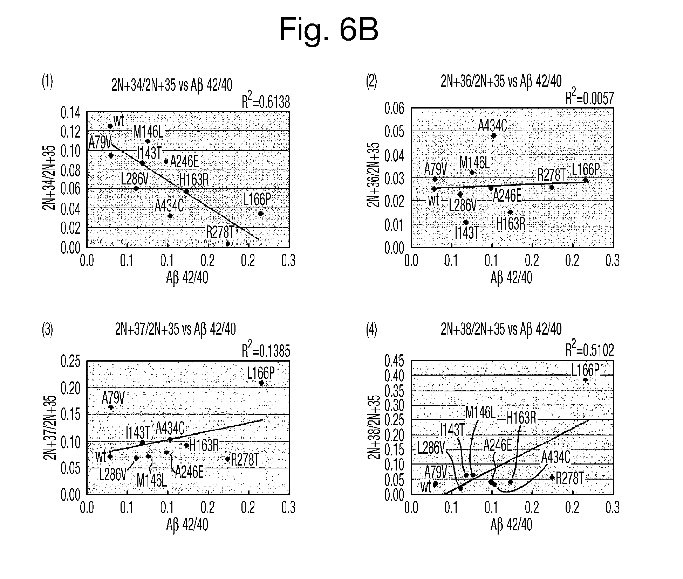

Figure 6A shows mass spectrometry results of p3-Alcₐₗₚₕₐ secreted by cells expressing wild-type PS1 or PS1 mutant linked to familial Alzheimer's disease. The cells stably expressing wild-type PS1 are indicated with "WT", and the cells non-expressing PS1 are indicated with "Mock". Other symbols represent the mutation of each PS1 mutant linked to familial Alzheimer's disease: Met146Leu (M146L), Leu166Pro (L166P), Ala246Glu (A246E), Arg278Thr (R278T), Leu286Val (L286V), Ala434Cys (A434C), Ala79Val (A79V), His163Arg (H163R), and Ile143Thr (I143T). Figure 6B shows the relationships of (1) the ratio of p3-Alcₐₗₚₕₐ2N+34 to p3-Alcₐₗₚₕₐ2N+35, (2) the ratio of p3-Alcₐₗₚₕₐ2N+36 to p3-Alcₐₗₚₕₐ2N+35, (3) the ratio of p3-Alcₐₗₚₕₐ2N+37 to p3-Alcₐₗₚₕₐ2N+35, and (4) the ratio of p3-AlCₐₗₚₕₐ2N+38 to p3-Alcₐₗₚₕₐ2N+35, with the A-BETA42/40 ratio in each cell shown in Figure 6A. vertical axis represents (1) the ratio of p3-Alcₐₗₚₕₐ2N+34 to p3-Alcₐₗₚₕₐ2N+35, (2) the ratio of p3-Alcₐₗₚₕₐ2N+36 to p3-Alcₐₗₚₕₐ2N+35, (3) the ratio of p3-Alcₐₗₚₕₐ2N+37 to p3-Alcₐₗₚₕₐ2N+35, or (4) the ratio of p3-Alcₐₗₚₕₐ2N+38 to p3-Alcₐₗₚₕₐ2N+35, and the horizontal axis represents A-BETA42/40.

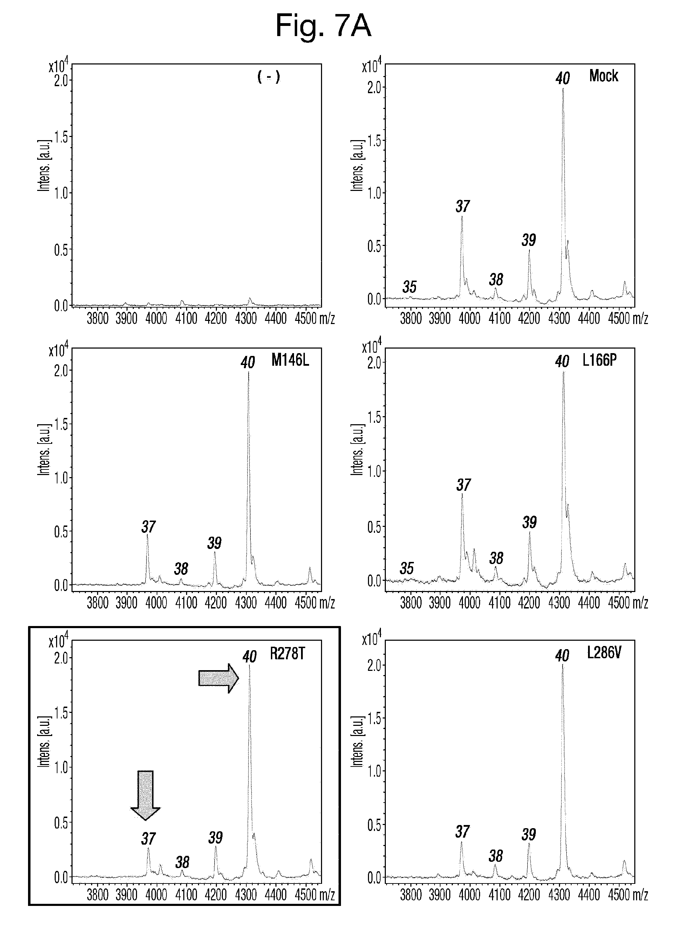

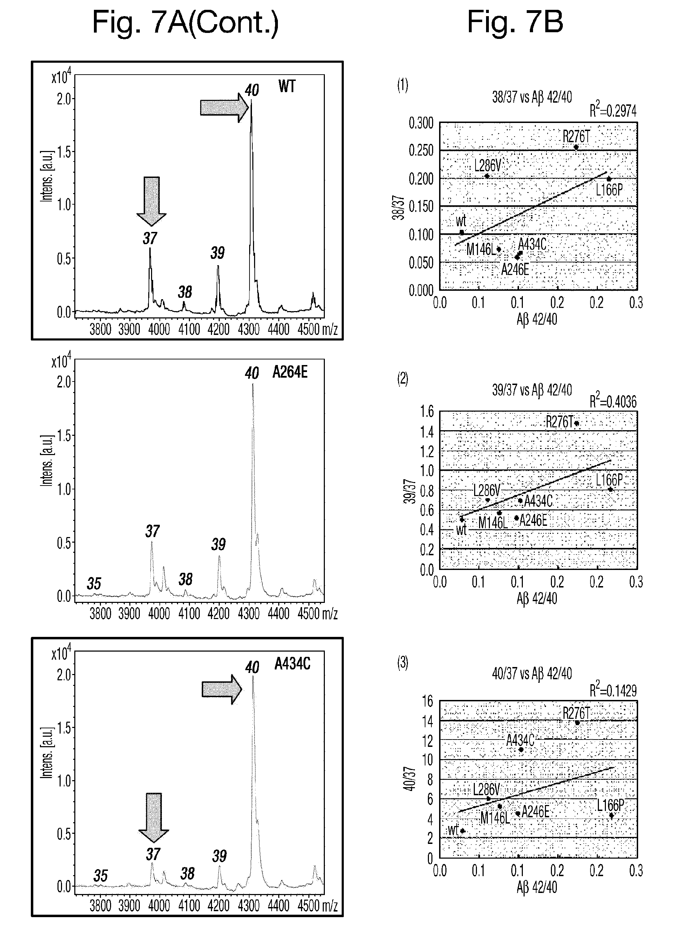

Figure 7A shows mass spectrometry results of p3-Alc_{beta} secreted by cells expressing wild-type PS1 or PS1 mutant linked to familial Alzheimer's disease. The cells stably expressing wild-type PS1 are indicated with "WT", and the cells non-expressing PS1 are indicated with "Mock". Other symbols represent the mutation of each PS1 mutant linked to familial Alzheimer's disease: Met146Leu (M146L), Leu166Pro (L166P), Ala246Glu (A246E), Arg278Thr (R278T), Leu286Val (L286V), and Ala434Cys (A434C).

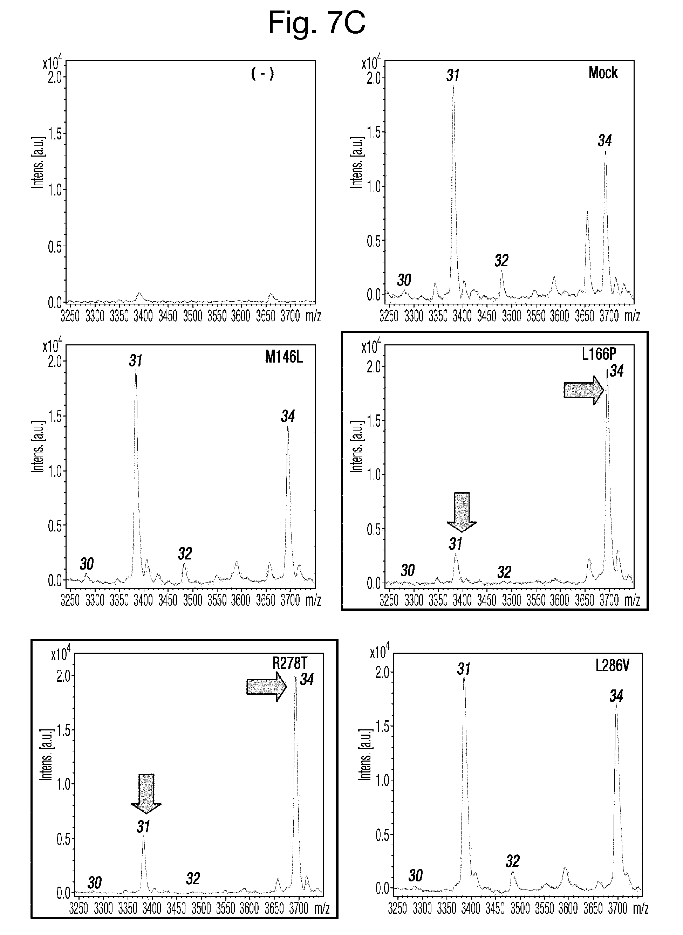

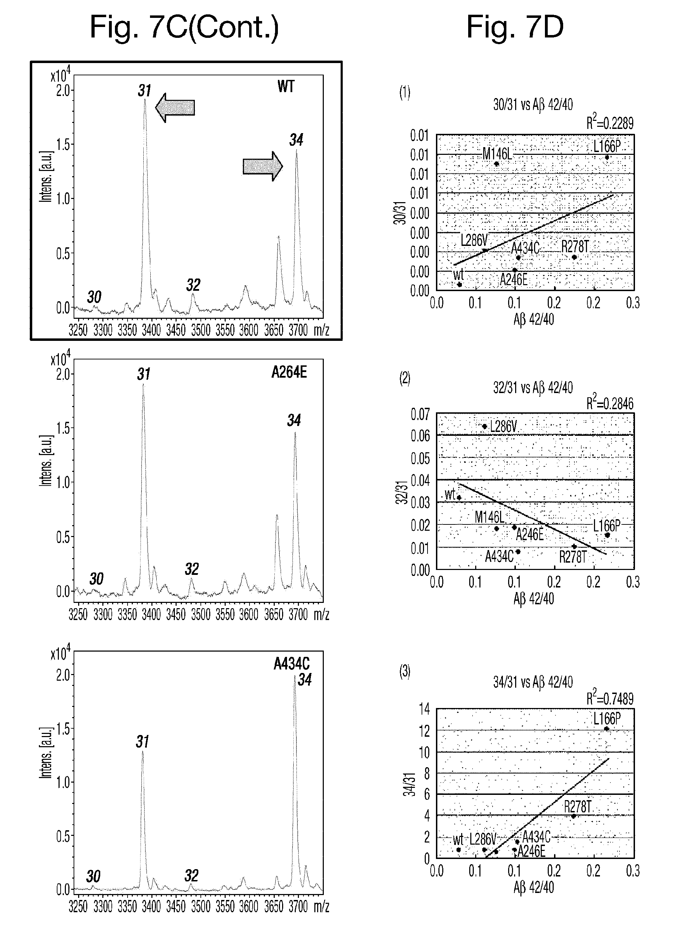

Figure 7B shows the relationships of (1) the ratio of p3-Alc_{beta}38 to p3-Alc_{beta}37, (2) the ratio of p3-Alc_{beta}39 to p3-Alc_{beta}37, and (3) the ratio of p3-Alc_{beta}40 to p3-Alc_{beta}37, with the A-BETA42/40 ratio in each cell shown in Figure 7A. The vertical axis represents (1) the ratio of p3-Alc_{beta}38 to p3-Alc_{beta}37, (2) the ratio of p3-Alc_{beta}39 to p3-Alc_{beta}37, or (3) the ratio of p3-Alc_{beta}40 to p3-Alc_{beta}37, and the horizontal axis shows A-BETA42/40. Figure 7C shows mass spectrometry results of p3-Alc_{gamma} secreted by cells expressing wild-type PS1 or PS1 mutant linked to familial Alzheimer's disease. The cells stably expressing wild-type PS1 are indicated with "WT", and the cells non-expressing PS1 are indicated with "Mock".
   Other symbols represent the mutation of each PS1 mutant linked to familial Alzheimer's disease: Met146Leu (M146L), Leu166Pro (L166P), Ala246Glu (A246E), Arg278Thr (R278T), Leu286Val (L286V), and Ala434Cys (A434C). Figure 7D shows the relationships of (1) the ratio of p3-Alc_{gamma}30 to p3-Alc_{gamma}31, (2) the ratio of p3-Alc_{gamma}32 to p3-Alc_{gamma}31, and (3) the ratio of p3-Alc_{gamma}34 to p3-Alc_{gamma}31, with the A-BETA42/40 ratio in each cell shown in Figure 7C. The vertical axis represents (1) the ratio of p3-Alc_{gamma}30 to p3-Alc_{gamma}31, (2) the ratio of p3-Alc_{gamma}32 to p3-Alc_{gamma}31, or (3) the ratio of p3-Alc_{gamma}34 to p3-Alc_{gamma}31, and the horizontal axis represents A-BETA42/40.
Figure 8 shows the A-BETA42/40 ratio in each PS1 mutant. "WT" represents cells expressing wild-type PS1, and "M146L", "L166P", "A246E", "R278T", "L286V", "A434C", "A79V", "H163R", or "I143T" represents PS1 mutants having each mutation.
Figure 9 shows mass spectrometry results of p3-Alc_{beta} in culture solutions of PS1/2 knockout mouse-derived MEFs and wild-type mouse-derived MEFs. The vertical axis represents intensity, and the horizontal axis represents m/z.

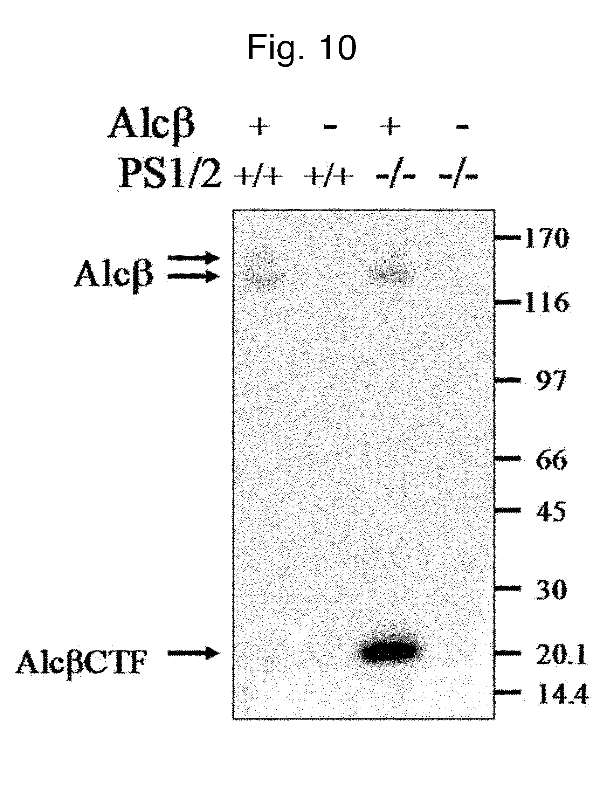

Figure 10 shows results of western blotting confirming Alc_{beta} and Alc_{beta}CTF in culture solutions of PS1/2 knockout mouse-derived MEFs and wild-type mouse-derived MEFs. The lanes show, from left to right, the results obtained from Alc_{beta}-transfected wild-type mouse-derived MEFs, Alc_{beta}-untransfected wild-type mouse-derived MEFs, Alc_{beta}-transfected PS1/2 knockout mouse-derived MEFs, and Alc_{beta}-untransfected PS1/2 knockout mouse-derived MEFs. In the drawing, the arrow indicated with AlcbetaCTF represents the Alc_{beta}CTF band, and the arrow indicated with Alcbeta represents the Alc_{beta} band.

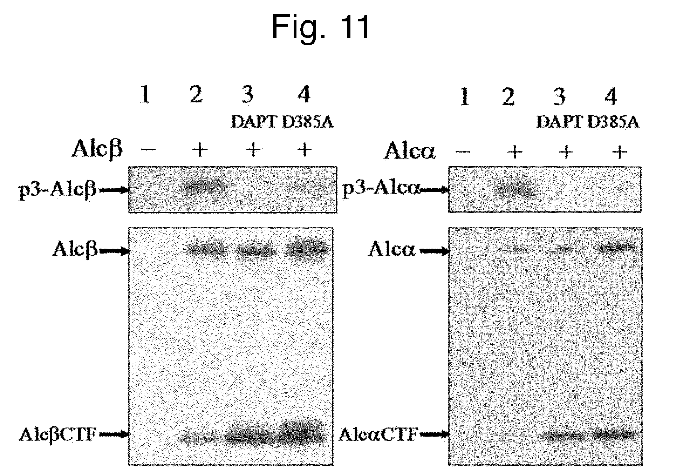

Figure 11 shows results of western blotting confirming Alc_{beta}, Alc_{beta}CTF, and p3-Alc_{beta} or Alcₐₗₚₕₐ, AlcₐₗₚₕₐCTF, and p3-Alcₐₗₚₕₐ in culture solutions obtained from Alc_{beta} - or Alcₐₗₚₕₐ-transfected cells. The lanes show, from left to right, the results obtained from Alc_{beta}- or Alcₐₗₚₕₐ-untransfected MEFs, Alc_{beta}- or Alcₐₗₚₕₐ-transfected, MEFs, DAPT-supplemented Alc_{beta}- or Alcₐₗₚₕₐ-transfected MEFs, Alc_{beta}- or Alcₐₗₚₕₐ-transfected MEFs expressing the D385A mutant of PS1. In the drawing, the arrow indicated with p3-Alcbeta represents the p3-Alc_{beta} band, the arrow indicated with AlcbetaCTF represents the Alc_{beta}CTF band, and the arrow indicated with Alcbeta represents the Alc_{beta} band. Also, in the diagram, the arrow indicated with p3-Alcalpha represents the p3-Alcₐₗₚₕₐ band, the arrow indicated with AlcalphaCTF represents the AlcₐₗₚₕₐCTF band, and the arrow indicated with Alcalpha represents the Alcₐₗₚₕₐ band;
Figure 12 is a diagram showing results of mass spectrometry of analysis of p3-Alcₐₗₚₕₐ, p3-Alc_{beta}, or p3-Alc_{gamma} in a mixture of human cerebrospinal fluids (0.3 to 1.0 mL) obtained from 5 to 10 human individuals (70 to 90 years old).
Figure 13 shows results of mass spectrometry of analysis of p3-Alcₐₗₚₕₐ, p3-Alc_{beta}, or p3-Alc_{gamma} in a mixture of human cerebrospinal fluids (0.3 to 1.0 mL) obtained from 5 to 10 human individuals (70 to 90 years old) (left diagrams), and results of MALDI-MS/MS analysis peptides indicated with the arrows which were collected to analyze their amino acid sequences by (right diagrams);
Figure 14 shows results of analyzing p3-Alc"37" consisting of 37 amino acids, by mass spectrometry conducted in reflector mode (left diagram). Amino acid sequences corresponding to p3 -Alcₐₗₚₕₐ37 and p3-Alcₐₗₚₕₐ2N+35 are also shown as a result of the analysis (right diagram);
Figure 15 shows results of analyzing the cerebrospinal fluids of 3 controls (Figures 15A to 15C) and the cerebrospinal fluids of 4 patients with Alzheimer's disease (Figures 15D to 15G) by mass spectrometry;
Figure 16 shows results of assaying p3-Alcₐₗₚₕₐ in the cerebrospinal fluids of humans without dementia (controls) (CDR=0), patients suspected of having Alzheimer's disease (CDR=0.5), sporadic Alzheimer's disease patients (CDR=1.0);

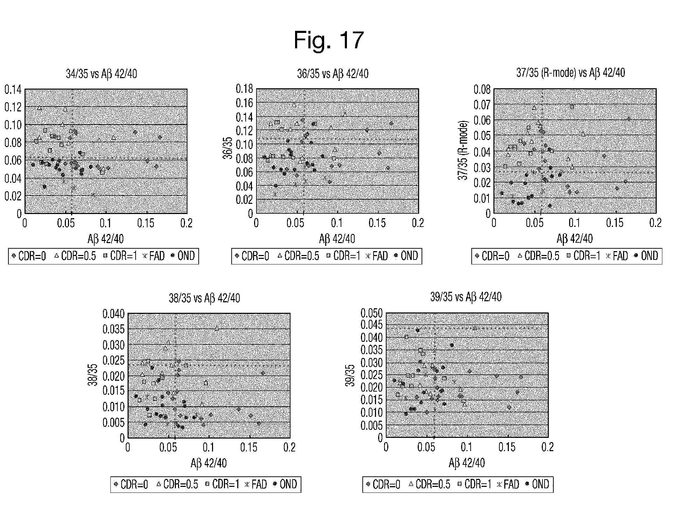

Figure 17 shows results of comparing (1) the ratio of p3-Alcₐₗₚₕₐ34 to p3-Alcₐₗₚₕₐ35, (2) the ratio of p3-Alcₐₗₚₕₐ36 to p3-Alcₐₗₚₕₐ35, (3) the ratio of p3-Alcₐₗₚₕₐ37 to p3-Alcₐₗₚₕₐ35, (4) the ratio of 3-Alcₐₗₚₕₐ38 to p3-Alcₐₗₚₕₐ35, and the ratio of p3-Alcₐₗₚₕₐ39 to p3-Alcₐₗₚₕₐ35 with the ratio of A-BETA42/40 in the cerebrospinal fluids of humans without dementia (controls) (CDR=0), patients suspected of having Alzheimer's disease (CDR=0.5), and sporadic Alzheimer's disease patients (CDR=1.0). The vertical dotted lines represent an average of the A-BETA42/40 ratio in the patients suspected having Alzheimer's disease (CDR=0.5). The horizontal dotted lines represent the maximum value of A-BETA42/40 in patients with other neurological and neurodegenerative diseases;
Figure 18 shows results of confirming the specificity of an Alc839 antibody (anti-p3-AlCₐₗₚₕₐ35 antibody) by ELISA. The rhombus represents the binding of the antibody to gamma5 (KKCVPSTATVVIV) (SEQ ID NO: 28). The square represents the binding of the antibody to TS-C (8 mer) (KCVPSTAT) (SEQ ID NO: 29). The triangle represents the binding of the antibody to TS-C (11 mer) (CPSTATVVIVV) (SEQ ID NO: 30). In the drawing, the vertical axis represents absorbance at a wavelength of 490 nm, and the horizontal axis represents a dilution ratio;
Figure 19 is a diagram showing results of preparing a standard curve using Alc817 and Alc839 antibodies. In the figure, the vertical axis represents absorbance at a wavelength of 450 nm, and the horizontal axis represents the concentration of the p3-Alcₐₗₚₕₐ35 peptide (pg/mL) ; and
Figure 20 is a diagram showing results of ELISA determing the concentration of p3-Alcₐₗₚₕₐ35 in cerebrospinal fluids obtained from humans without dementia (healthy subject), familial Alzheimer's disease patients (FAD), and patients with other neurological and neurodegenerative diseases (OND). In the figure, the vertical axis represents the concentration of the p3-AlCₐₗₚₕₐ35 peptide (pg/mL).

### Best Mode for Carrying Out the Invention

### 1. Preparation of anti-p3-Alc antibody

An antibody of the present invention can be prepared, for example, by immunizing non-human mammals or birds with p3-Alc or with a peptide having a portion of p3-Alc, such as a peptide having the any one of amino acid sequences of SEQ ID NOs: 5 to 10, 26, and 27 (hereinafter, this peptide is referred to as a "peptide having a portion of p3-Alc"), if necessary together with an immunostimulant (e.g., mineral oil or aluminum precipitates with heat-killed bacteria or lipopolysaccharide, a Freund's complete adjuvant, or a Freund's incomplete adjuvant). The p3-Alc used as an immunogen is not particularly limited as long as the p3-Alc is derived from mammals and is preferably human p3-Alc. For example, an antibody against p3-Alcₐₗₚₕₐ can be obtained using a peptide having the amino acid sequence of SEQ ID NO: 5, a peptide having the amino acid sequence of SEQ ID NO: 6, a peptide having the amino acid sequence of SEQ ID NO: 7, a peptide having the amino acid sequence of SEQ ID NO: 26, or a peptide having the amino acid sequence of SEQ ID NO: 27 as an immunogen. Particularly, an antibody against p3-Alcₐₗₚₕₐ35 can be obtained using a peptide having the amino acid sequence of SEQ ID NO: 26 or a peptide having the amino acid sequence of SEQ ID NO: 27 as an immunogen. An antibody which specifically recognizes any of other p3-Alcₐₗₚₕₐ species can be obtained by selecting an immunogen according to the way of selecting the immunogen in obtaining the antibody against p3-Alcₐₗₚₕₐ35. An antibody against p3-Alc_{beta} can be obtained using a peptide having the amino acid sequence of SEQ ID NO: 8 or a peptide having the amino acid sequence of SEQ ID NO: 9 as an immunogen. An antibody against p3-Alc_{gamma} can be obtained using a peptide having the amino acid sequence of SEQ ID NO: 10 as an immunogen. The immunogen used for preparing the antibody of the present invention can be obtained by transfecting E. coli, yeast, insect cells) animal cells, or the like with expression vectors containing cDNA encoding the p3-Alc or the peptide having a portion of p3-Alc to express the gene. When the.peptide having a portion of p3-Alc is used as an immunogen, the peptide having a portion of p3-Alc may be used directly, or one type or two or more types ofpeptides having a portion of p3-Alc may be linked via a linker for use.

The p3-Alc or the peptide having a portion of p3-Alc can be prepared by chemical synthesis using the Fmoc or Boc method or the like. For example, p3-Alc or a peptide having a portion of p3-Alc is immobilized via its C-terminal amino acid onto a polystyrene carrier, then bound with amino acids protected by a 9-fluorenylmethyloxycarbonyl (Fmoc) group or tert-butoxycarbonyl (Boc) group by using a condensing agent such as diisopropylcarbodiimide (DIC), and washed and deprotected repeatedly to obtain a peptide having the desired amino acid sequence. The p3-Alc or the peptide having a portion of p3-Alc may be synthesized using an automatic peptide synthesizer. Examples of such a peptide synthesizer include: PSSM-8 (Shimadzu Corp.); model 433A peptide synthesizer (Applied Biosystems, Inc.); and ACT-396 Apex (Advanced ChemTech Inc.).

The immunized animals are not particularly limited as long as they are animals from which hybridomas can be prepared, such as mice, rats, hamsters, rabbits, chickens, and ducks. The immunogen can be administered to the animals by, for example, subcutaneous, intraperitoneal, intravenous, intradermal, intramuscular, or footpad injection, preferably subcutaneous or intraperitoneal injection. The amount of the immunogen used is not particularly limited as long as the amount allows antibody production. The amount is preferably 0.1 to 1000 microg, more preferably 1 to 500 microg, even more preferably 10 to 100 microg. The immunization can be performed once or several times at appropriate intervals. Preferably, the immunization is preformed 2 to 5 times in total at once-α-week to every-five-week, more preferably 3 times in total at every-three-week. 1 to 2 weeks after the final immunization, blood is collected from the orbits or tail veins of the immunized animals, and the serum is used in antibody titer measurement. The antibody titer measurement can be conducted by a method well known by those skilled in the art. Examples of the method can include radioimmunoassay (RIA), enzyme-linked immunosorbent assay (ELISA), fluoroimmunoassay, and passive hemagglutination. ELISA is preferable. The antibody of the present invention can be obtained by purification from animal serum that exhibits a sufficient antibody titer.

The monoclonal antibody can be obtained by fusing myeloma cells with antibody-producing-cells obtained from the animals immunized by the method and by culturing the obtained hybridomas. Examples of the fusion method can include the method of Milstein et al. (Galfre, G. & Milstein, C., Methods Enzymol. 73: 3-46, 1981). The antibody-producing cells used can be collected from the spleens, pancreata, lymph nodes, or peripheral blood of the mice or rats immunized by the above method which exhibit a sufficient antibody titer in the serum. The myeloma cells used are, for example, cells derived from mammals such as mice, rats, guinea pigs, hamsters, rabbits, or humans, and are not particularly limited as long as the cells are capable of growing *in vitro*. Examples of such cells can include P3-X63Ag8 (X63) (Nature, 256, 495, 1975), P3/NS1/1-Ag4-1 (NS1) (Eur. J. Immunol., 6, 292, 1976), P3X63Ag8U1 (P3U1) (Curr. Top. Microbiol. Immunol., 81, 1, 1978), P3X63Ag8.653 (653) (J. Immunol., 123, 1548, 1979), Sp2/0-Ag14 (Sp2/O) (Nature, 276, 269, 1978), and Sp2/O/FO-2 (FO-2) (J. Immunol. Methods, 35, 1, 1980). The antibody-producing cells and the myeloma cells obtained according to the above method are washed with a medium, PBS (phosphate buffered saline), or the like and then fused after the addition of a cell-aggregating medium such as polyethylene glycol (hereinafter, referred to as "PEG") (Elsevier Publishing, 1988). The antibody-producing cells and the myeloma cells are fused at a ratio of, for example, 2:1 to 1:2. The cells thus fused are cultured in a medium such as a HAT (hypoxanthine-aminopterin-thymidine) medium for selective growth of hybridomas. After the culture, the culture supernatant is collected, and samples that bind to antigenic proteins but not to non-antigenic proteins are selected therefrom by ELISA or the like. The samples are made into single cells by a limiting dilution method, and cells that stably exhibit a high antibody titer are selected. The monoclonal antibody can be obtained by culturing the hybridomas obtained by the above method *in vitro*, and purifying from the culture solution. The monoclonal antibody of the present invention may be obtained by: intraperitoneally administering pristane in advance to animals of the same line as that of the donor animals used in the hybridoma production or to immunodeficient animals; transplanting the hybridomas into the animals; collecting ascitic fluids therefrom; and purifying the antibodies of interest from the collected ascitic fluids.

The purification of the monoclonal antibody can be performed by collecting IgG fractions using a protein A column, a protein G column, or the like after centrifugation. When the antibody belongs to IgY or IgM class, its purification can be achieved using a column coupled to mercaptopyridine as a ligand. The monoclonal antibody may be purified using an p3-Alc immobilized column, ion-exchange chromatography, hydrophobic interaction chromatography, or the like, regardless of antibody class.

### 2. Preparation of antibody fragment

A fragment of the antibody of the present invention, i.e., (F(ab')₂, Fab', Fab, scFv, dsFv, or polymers thereof, Diabody, or a CDR-containing peptide, can be prepared by a method shown below. The F(ab')₂ fragment of the present invention can be obtained by: treating an IgG antibody which can bind to p3-Alc with a protease pepsin; and cleaving the obtained fragment at H-chain amino acid residue 234 to obtain an antibody fragment of approximately 100,000 in molecular weight having antigen-binding activity. The F(ab')₂ fragment of the present invention may be obtained by linking Fab' fragments (described later) via a thioether or disulfide bond. The Fab' fragment of the present invention can be obtained by treating, the F(ab')₂ fragment which can bind to p3-Alc obtained by the above method with a reducing agent dithiothreitol. The Fab' fragment of the present invention may be obtained by: inserting DNA encoding the Fab' domain of the antibody of the present invention which can bind to p3-Alc into expression vectors; and transforming host cells with the vectors to express the DNA.

The Fab fragment of the present invention can be obtained by: treating an antibody which can bind to p3-Alc with a protease papain; and cleaving the obtained fragment at H-chain amino acid residue 224 to obtain an antibody fragment of approximately 50,000 in molecular weight which comprises approximately the N-terminal half region of the H chain and the whole region of the L chain linked via a disulfide bond and has antigen-binding activity. The Fab fragment of the present invention may be obtained by: inserting DNA encoding the Fab domain of the antibody of the present invention which can bind to p3-Alc, into expression vectors; and transforming host cells with the vectors to express the DNA. The scFv of the present invention can be obtained by: obtaining each cDNA encoding the VH or VL domain of an antibody which can bind to p3-Alc; inserting linker sequence-encoding DNA to between these genes to construct scFv-encoding DNA; inserting the DNA into expression vectors; and transforming host cells with the vectors to express the DNA. The length of the linker is not particularly limited as long as the length allows association between VH and VL, and is preferably 10 to 20 residues, more preferably 15 residues. The sequence of the linker is not particularly limited unless the sequence inhibits the folding of the polypeptides chains of these two domains (VH and VL), and is preferably consisting of glycine(s) and/or serine(s), more preferably GGGGS (G: glycine, S: serine) or repeats of this sequence.

The dsFv of the present invention can be obtained by: replacing each one amino acid residue in VH and VL with cysteine residues by site-directed mutagenesis; and linking the VH and VL domains via a disulfide bond between the cysteine.residues. The replaced amino acids are not particularly limited unless the amino acid residues influence antigen binding in the three-dimensional structure. The Diabody of the present invention can be obtained by: constructing the scFv-encoding DNA having a sequence encoding a linker amino acid sequence of 8 or less residues (preferably, 5 residues); inserting the DNA into expression vectors; and transforming host cells with the vectors to express the DNA. Bispecific Diabody can be obtained by preparing scFv using DNAs of two sets of scFv VH and VL domains which show different specificities in combination. The CDR-containing peptide can be obtained by: constructing DNA encoding the VH or VL CDR amino acid sequence of the antibody of the present invention which can bind to p3-Alc; inserting the DNA into expression vectors; and transforming host cells with the vectors to express the DNA.

### 3. Selection of p3-Alc-binding antibody, etc.

If necessary, an antibody having the high ability to bind to p3-Alc can be selected from the above obtained antibodies which can bind to p3-Alc or the fragments thereof. The binding of the antibody or the fragment thereof to p3-Alc can be assayed by a method well known by those skilled in the art. Examples of such a method can include western blotting, chromatography (affinity chromatography), and X-ray crystallography as well as Biacore systems (Biacore). The antibody or the fragment may bind to a particular p3-Alcₐₗₚₕₐ species or may bind to all p3-Alcₐₗₚₕₐ species. An antibody specific for a particular p3-Alcₐₗₚₕₐ species is preferably selected for assaying the particular Alcₐₗₚₕₐ species by ELISA. Alternatively, an antibody which can bind to all p3-Alcₐₗₚₕₐ species is preferable for collecting all the p3-Alcₐₗₚₕₐ species by immunoprecipitation. It is preferred that the anti-p3-Alcₐₗₚₕₐ antibody do not cross-react with the other p3-Alc species (p3-Alc_{beta} and p3-Alc_{gamma}). The same applies to p3-Alc_{beta} and p3-Alc_{gamma}.

### 4. Diagnostic agent

A diagnostic agent of the present invention can be based on a method known in the art using a p3-Alc antibody or a fragment thereof. Examples of such a method can include ELISA (Catty, Raykundalia, 1989), radioimmunoassay (Catty, Murphy, 1989), immunohistochemical method (Heider et al., 1993), and western blotting. The p3-Alc antibody or the fragment thereof is preferably an antibody specific for p3-Alc_{alpha,} p3-Alc_{beta}, or p3-Alc_{gamma}. When the quantity or concentration of a p3-Alc species is determined from the amount of the p3-Alc species which bind to the antibody as an index by using ELISA or the like, it is preferred that the diagnostic agent of the present invention comprise an antibody specific for each p3-Alc species (e.g., an antibody specific for p3-Alcₐₗₚₕₐ34, p3-Alcₐₗₚₕₐ35, p3-Alcₐₗₚₕₐ36, p3-Alcₐₗₚₕₐ37, p3-Alcₐₗₚₕₐ38, or p3-Alcₐₗₚₕₐ39). The diagnostic agent of the present invention may be a diagnostic kit used alone or may be a reagent used in combination with other instruments. The diagnostic agent of the present invention can be used for, for example, a biopsied tissue sample or liquid collected as an analyte from a subject. The biopsied analyte used is not particularly limited as long as it can be targeted by the immunoassay of p3-Alc. Examples thereof can include tissues, blood, urine, serous fluids, spinal fluids (particularly, cerebrospinal fluids), joint fluids, aqueous humor, lacrimal fluids, saliva, and fractionated or processed products thereof. The diagnostic agent of the present invention can achieve as qualitative, quantitative, or semi-quantitative analysis.

### 5. Diagnostic apparatus

A diagnostic apparatus of the present invention comprises means of separating p3-Alc in an analyte and means of detecting the separated p3-Alc species. The means of separating p3-Alc in an analyte is not particularly limited as long as the means is capable of separating p3-Alc, and examples can include immunoprecipitation, western blotting, and affinity chromatography. It is preferred that the means of separating p3-Alc from an analyte comprise a substance capable of binding to p3-Alc as a reagent for separating p3-Alc. The substance capable of binding to p3-Alc is preferably an antibody against p3-Alc or a fragment thereof, more preferably an antibody which can specifically bind to p3-Alc or a fragment thereof. The p3-Alc antibody or the fragment thereof is preferably an antibody specific for p3-Alcₐₗₚₕₐ, p3-AlC_{beta}, or p3-Alc_{gamma}. For example, an antibody obtained by using the amino acid sequence of any one of SEQ ID NOs: 5 to 7 as an antigen can be used as an anti-p3-Alcₐₗₚₕₐ antibody. An antibody obtained by using the amino acid sequence of SEQ ID NO: 8 or 9 as an antigen can be used as an anti-p3-Alc_{beta} antibody. An antibody obtained by using the amino acid sequence of SEQ ID NO: 10 as an antigen can be used as an anti-p3-Alc_{gamma} antibody.

The means of detecting the separated p3-Alc species is not particularly limited as long as the means is capable of detecting the p3-Alc species. In this context, the phrase "detecting the p3-Alc species" encompasses detecting sequence information about amino acids constituting the peptide, information about the number of amino acids constituting the peptide, the molecular weight of the peptide, physical or chemical information based on the amino acid sequence constituting the peptide, or information about the amount of the peptide. The means may detect one of these information items or may detect some of them. Examples of the means of detecting information about the amino acid sequence can include mass spectrometers and protein sequencers.

### 6. Diagnostic method and method for gathering information for diagnosis

The biomarker of the present invention serves as an index for a disease associated with the abnormality in gamma-secretase, particularly, for Alzheimer's disease. Accordingly, the biomarker of the present invention or the antibody of the present invention or the fragment thereof can be used in a diagnostic method for a disease associated with the abnormality in gamma-secretase, a method for providing information for the diagnosis of a disease associated with the abnormality in gamma-secretase, a method for monitoring the condition or the degree of progression of a disease associated with the abnormality in gamma-secretase, and a method for determining the therapeutic effect of a therapeutic agent on a disease associated with the abnormality in gamma-secretase (hereinafter, these methods are collectively referred to as a "diagnostic method and the like for a disease associated with the abnormality in gamma-secretase").

The diagnostic method and the like for a disease associated with the abnormality in gamma-secretase according to the present invention comprises the step of detecting p3-Alc in an analyte and can perform diagnosis with the amount of the p3-Alc species as an index. When the diagnostic method of the present invention performs diagnosis with the amount of the p3-Alc species as an index, the p3-Alc species to be assayed is preferably a p3-Alcₐₗₚₕₐ species such as p3-Alcₐₗₚₕₐ34, p3-Alcₐₗₚₕₐ35, p3-Alcₐₗₚₕₐ36, p3-Al_{calpha}37, p3-Alcₐₗₚₕₐ38, or p3-Alcₐₗₚₕₐ39, more preferably p3-Alcₐₗₚₕₐ34, p3-Alcₐₗₚₕₐ35, p3-Alcₐₗₚₕₐ37, and p3-Alcₐₗₚₕₐ39.

More specifically, the diagnostic method for a disease associated with the abnormality in gamma-secretase according to the present invention can be performed by the following steps:
a) preparing a sample derived from a subject;
b) contacting the sample with an antibody or a fragment thereof which can recognize at least one p3-Alc;
c) determining the level of the p3-Alc by detecting the binding of the antibody or the fragment thereof to the p3-Alc; and
d) associating the level of the p3-Alc with the presence or absence of or the severity of the disease associated with the abnormality in gamma-secretase in the subject.
For example, this method can be performed by ELISA or immunochromatography.

The diagnostic method for a disease associated with the abnormality in gamma-secretase according to the present invention can be performed by the following steps:
a) preparing a sample derived from a subject;
b) separating p3-Alc;
c) detecting the separated p3-Alc species; and
d) associating the level of the p3-Alc with the presence or absence of or the severity of the disease associated with the abnormality in gamma-secretase in the subject.
For example, this method can be performed using chromatography and mass spectrometry in combination.

In the above diagnostic methods, a peptide consisting of the amino acid sequence of any one of SEQ ID NOs: 11 to 16 and SEQ ID NOs: 18 to 25 can be used as p3-Alc. The "step of associating the level of the p3-Alc with the presence or absence of or the severity of the disease associated with the abnormality in gamma-secretase in the subject" can comprise determining a subject who have a larger amount of p3-Alc, preferably, a peptide consisting of the amino acid sequence of any one of SEQ ID NOs: 11 to 16 and SEQ ID NOs: 18 to 20, to be a patient having the disease associated with the abnormality in gamma-secretase or having the high severity of the disease.

The diagnostic method and the like for a disease associated with the abnormality in gamma-secretase according to the present invention comprises the step of detecting p3-Alc in an analyte and can perform diagnosis with the ratio of the amount of the p3-Alc species as an index. When the diagnostic method of the present invention performs diagnosis with the ratio of the amount of the p3-Alc species as an index, the p3-Alc species to be assayed is preferably a p3-Alcₐₗₚₕₐ species such as p3-Alcₐₗₚₕₐ34, p3-Alcₐₗₚₕₐ35, p3-Alcₐₗₚₕₐ36, p3-Alcₐₗₚₕₐ37, p3-Alcₐₗₚₕₐ38, or p3-Alcₐₗₚₕₐ39. When the diagnostic method of the present invention performs diagnosis with the ratio of the amount of the p3-Alc species as an index, the ratio between p3-Alcₐₗₚₕₐ35 and another p3-Alcₐₗₚₕₐ species (e.g., p3-Alcₐₗₚₕₐ34, p3-Alcₐₗₚₕₐ36, p3-Alcₐₗₚₕₐ37, p3-Alcₐₗₚₕₐ38, or p3-Alcₐₗₚₕₐ39) (e.g., p3-AlCₐₗₚₕₐ37/p3-Alcₐₗₚₕₐ35 or p3-Alcₐₗₚₕₐ35/p3-Alcₐₗₚₕₐ37) is used as an index.

More specifically, the diagnostic method for a disease associated with the abnormality in gamma-secretase according to the present invention can be performed by the following steps:
a) preparing a sample derived from a subject;
b) contacting the sample with an antibody or a fragment thereof which can recognize a peptide consisting of the amino acid sequence of SEQ ID NO: 11 or 12 and with an antibody or a fragment thereof which can recognize a peptide consisting of the amino acid sequence of any one of SEQ ID NOs: 18 to 25 or a fragment of the antibody;
c) determining the levels of the peptide consisting of the amino acid sequence of SEQ ID NO: 11 or 12 and the peptide consisting of the amino acid sequence of any one of SEQ ID NOs: 18 to 25 by detecting the bindings of the antibodies or the fragments thereof to the peptide consisting of the amino acid sequence of SEQ ID NO: 11 or 12 and the peptide consisting of the amino acid sequence of any one of SEQ ID NOs: 18 to 25;
d) calculating the quantitative ratio between the peptide consisting of the amino acid sequence of any one or SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
e) associating the calculated ratio with the presence or absence of or the severity of the disease associated with the abnormality in gamma-secretase in the subject.

The diagnostic method for a disease associated with the abnormality in gamma-secretase according to the present invention can be performed by the following steps:
a) preparing a sample derived from a subject;
b) separating a peptide consisting of the amino acid sequence of SEQ ID NO: 11 or 12 and a peptide consisting of the amino acid sequence of any one of SEQ ID NOs: 18 to 25;
c) detecting information about the amino acid sequences of the separated peptide consisting of the amino acid sequence of SEQ ID NO: 11 or 12 and the separated peptide consisting of the amino acid sequence of any one of SEQ ID NOs: 18 to 25;
d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
e) associating the calculated ratio with the presence or absence of or the severity of the disease associated with the abnormality in gamma-secretase in the subject.

In the diagnostic method, a peptide consisting of the amino acid sequence of any one of SEQ ID NOs: 11 to 16 or SEQ ID NOs: 18 to 25 can be used as p3-Alc. The "step of associating the calculated value with the presence or absence of or the severity of the disease associated with the abnormality in gamma-secretase in the subject" can be performed by determining a subject having a larger quantitative ratio of the peptide consisting of the amino acid sequence of any one of SEQ ID NOs: 18 to 20 to the peptide consisting of the amino acid sequence of SEQ ID NO: 12 or a larger quantitative ratio of the peptide consisting of the amino acid sequence of any one of SEQ ID NOs: 21 to 25 to the peptide consisting of the amino acid sequence of SEQ ID NO: 11, to be a patient having the disease associated with the abnormality in gamma-secretase or having the high severity of the disease.

The diagnostic method and the like for a disease associated with the abnormality in gamma-secretase according to the present invention can be performed in combination with the assay of A-BETA already used as an index for Alzheimer's disease. Specifically, the diagnostic method and the like for a disease associated with the abnormality in gamma-secretase according to the present invention comprises the steps described above and further comprises the steps of:
i) detecting amyloid beta42 and amyloid beta40 in the analyte;
ii) calculating the ratio of the amyloid beta42 to the amyloid beta40 detected;
iii) calculating the quantitative ratio between the peptide consisting of the amino acid sequence of any one of SEQ ID NOs: 18 to 25 and the peptide consisting of the amino acid sequence of SEQ ID NO: 11; and
iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the severity of the disease associated with the abnormality in gamma-secretase in the subject.

The patient-derived analyte used in the method of the present invention is not particularly limited as long as the expression of the biomarker can be detected from the analyte. For example, a biopsied tissue sample or liquid collected from a subject can be used. The analyte used is not particularly limited as long as it can be a subject in the assay of the present invention. Examples thereof can include tissues, blood, plasma, serum, lymph, urine, serous fluids, spinal fluids (particularly, cerebrospinal fluids), joint fluids, aqueous humor, lacrimal fluids, saliva, and fractionated or processed products thereof. The analyte is preferably blood (containing plasma or serum). The patient-derived sample used in the method of the present invention may be the patient-derived analyte pretreated prior to the assay test or the analyte collected from the patient may be used directly as a sample. The diagnostic method of the present invention can achieve qualitative, quantitative, or semi-quantitative analysis. In the diagnostic method of the present invention, the disease associated with the abnormality in gamma-secretase is preferably Alzheimer's disease. The diagnostic method for a disease associated with the abnormality in gamma-secretase may be a method for providing information for the diagnosis of a disease associated with the abnormality in gamma-secretase or a method for monitoring the condition or the degree of progression of a disease associated with the abnormality in gamma-secretase.

The biomarker of the present invention serves as an index for the disease and thus, can be used as an index for therapeutic effect on a patient in the screening of a disease associated with the abnormality in gamma-secretase. For example, whether or not a therapeutic agent can treat a disease associated with the abnormality in gamma-secretase can be determined by: adding the test agent to a cell expressing mutant gamma-secretase, administering the test agent to an abnormal gamma-secretase model animal, or administering the test agent to a patient with a disease associated with the abnormality in gamma-secretase; and determining the amount of the biomarker of the present invention after a given time. The screening method of the present invention comprises the step of detecting p3-Alc in an analyte and can be performed with the amount of the p3-Alc species as an index.

Specifically, the method for screening a therapeutic or preventive agent for a disease associated with the abnormality in gamma-secretase according to the present invention, comprising the step of detecting p3-Alc in an analyte, can be performed by the following steps:
a) contacting a test agent with a cell expressing mutant gamma-secretase;
b) preparing a culture solution of the cell as a sample;
c) contacting the sample with an antibody or a fragment thereof which can recognize at least one p3-Alc;
d) determining the level of the p3-Alc by detecting the binding of the antibody or the fragment thereof to the p3-Alc; and
e) associating the level of the p3-Alc with the presence or absence of or the degree of the therapeutic or
preventive effect of the test agent on the disease associated with the abnormality in gamma-secretase.

The method for screening a therapeutic or preventive agent for a disease associated with the abnormality in gamma-secretase according to the present invention, comprising the step of detecting p3-Alc in an analyte, can be performed by the following steps:
a) administering a test agent to an animal or a human having the abnormality in gamma-secretase;
b) preparing the body fluid of the recipient as a sample;
c) contacting the sample with an antibody or a fragment thereof which can recognize at least one p3-Alc;
d) determining the level of the p3-Alc by detecting the binding of the antibody or the fragment thereof to the p3-Alc; and
d) associating the level of the p3-Alc with the presence or absence of or the degree of the therapeutic or
preventive effect of the test agent on the disease associated with the abnormality in gamma-secretase.

The method for screening a therapeutic or preventive agent for a disease associated with the abnormality in gamma-secretase according to the present invention, comprising the step of detecting p3-Alc in an analyte, can be performed by the following steps:
a) contacting a test agent with a cell expressing mutant gamma-secretase;
b) preparing a culture solution of the cell as a sample;
c) separating p3-Alc:
d) detecting the separated p3-Alc species; and
e) associating the level of the p3-Alc with the presence
or absence of or the degree of the therapeutic or preventive effect of the test agent on the disease associated with the abnormality in gamma-secretase.

Also, the method for screening a therapeutic or preventive agent for a disease associated with the abnormality in gamma-secretase according to the present invention, comprising the step of detecting p3-Alc in an analyte, can be performed by the following steps:
a) administering a test agent to an animal or a human having the abnormality in gamma-secretase;
b) preparing the body fluid of the recipient as a sample;
c) separating p3-Alc:
d) detecting the separated p3-Alc species; and
e) associating the level of the p3-Alc with the presence
or absence of or the degree of the therapeutic or preventive effect of the test agent on the disease associated with the abnormality in gamma-secretase.

The method for screening a therapeutic or preventive agent for a disease associated with the abnormality in gamma-secretase according to the present invention comprises the step of detecting p3-Alc in an analyte and can perform diagnosis with the ratio of the amount of the p3-Alc species as an index. For example, in the method for screening a therapeutic or preventive agent for a disease associated with the abnormality in gamma-secretase according to the present invention, the step of associating the level of the p3-Alc with the presence or absence of or the degree of the therapeutic or preventive effect of the test agent on the disease associated with the abnormality in gamma-secretase can be performed by the following steps:
e-1) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
e-2) associating the calculated ratio with the presence or absence of or the degree of the therapeutic or preventive effect of the test agent on the disease associated with the abnormality in gamma-secretase.

The method for screening a therapeutic or preventive agent for a disease associated with the abnormality in gamma-secretase according to the present invention can be performed in combination with the assay of A-BETA which is already used as an index for Alzheimer's disease. Specifically, the method for screening a therapeutic or preventive agent for a disease associated with the abnormality in gamma-secretase according to the present invention comprises the steps described above and may further comprise the steps of:
i) detecting amyloid beta42 and amyloid beta40 in the analyte;
ii) calculating the ratio of the amyloid beta42 to the amyloid beta40 detected;
iii) calculating the quantitative ratio between the peptide consisting of the amino acid sequence of any one of SEQ ID NOs: 18 to 25 and the peptide consisting of the amino acid sequence of SEQ ID NO: 11; and
iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the degree of the therapeutic or preventive effect of the test agent on the disease associated with
the abnormality in gamma-secretase.

In the method for screening a therapeutic or preventive agent for a disease associated with the abnormality in gamma-secretase according to the present invention, the p3-Alc may be any of p3-Alc species p3-Alcₐₗₚₕₐ, p3-Alc_{beta}, and p3-Alc_{gamma}. The p3-Alc may be, for example, p3-Alcₐₗₚₕₐ2N+34, p3-Alcₐₗₚₕₐ2N+35, p3-Alcₐₗₚₕₐ2N+36, p3-AlCₐₗₚₕₐ2N+37, p3-Alcₐₗₚₕₐ2N+38, p3-Alcₐₗₚₕₐ2N+39, p3-AlCₐₗₚₕₐ34, p3-Alcₐₗₚₕₐ35, p3-Alcₐₗₚₕₐ36, p3-Alcₐₗₚₕₐ37, p3-Alcₐₗₚₕₐ38, or p3-Alcₐₗₚₕₐ39 and may be specifically a peptide consisting of the amino acid sequence of any one of SEQ ID NOs: 11 to 16 and SEQ ID NOs: 18 to 25. The "step of associating the level of the p3-Alc with the presence or absence of or the degree of the therapeutic or preventive effect of the test agent on the disease associated with the abnormality in gamma-secretase" can comprise determining a test agent decreasing the amount of p3-Alc, preferably, decreasing the amount of a peptide consisting of the amino acid sequence of any one of SEQ ID NOs: 11 to 16 and SEQ ID NOs: 18 to 20, to have therapeutic or preventive effect on the disease associated with the abnormality in gamma-secretase or have higher therapeutic or preventive effect on this disease. The "step of associating the calculated ratio with the presence or absence of or the degree of the therapeutic or preventive effect of the test agent on the disease associated with the abnormality in gamma-secretase" can comprise determining a test agent decreasing the ratio of the quantity of the peptide consisting of the amino acid sequence of any one of SEQ ID NOs: 18 to 20 to the quantity of the peptide consisting of the amino acid sequence of SEQ ID NO: 12 or the quantitative ratio between the peptide consisting of the amino acid sequence of any one of SEQ ID NOs: 21 to 25 to the quantity of the peptide consisting of the amino acid sequence of SEQ ID NO: 11, to have therapeutic or preventive effect on the disease associated with the abnormality in gamma-secretase or have higher therapeutic or preventive effect on this disease.

In the method for screening a therapeutic or preventive agent according to the present invention, a culture solution or, for example, a biopsied tissue sample or liquid collected from a subject can be used as an analyte. The biopsied analyte used is not particularly limited as long as it can be targeted by the immunoassay of p3-Alc. Examples thereof can include tissues, blood, serum, plasma, urine, serous fluids, spinal fluids (particularly, cerebrospinal fluids), joint fluids, aqueous humor, lacrimal fluids, saliva, and fractionated or processed products thereof. The analyte is preferably a body fluid, more preferably blood, serum, plasma, or a spinal fluid (particularly, a cerebrospinal fluid). The method for screening a therapeutic or preventive agent according to the present invention can achieve qualitative, quantitative, or semi-quantitative analysis. In the method for screening a therapeutic or preventive agent according to the present invention, the disease associated with the abnormality in gamma-secretase is preferably Alzheimer's disease.

In the above methods, the "step of detecting p3-Alc in an analyte" can be performed by a method that is capable of detecting p3-Alc in an analyte and is well known by those skilled in the art. The detection of p3-Alc can encompass the identification and quantification of each p3-Alc species. For example, the p3-Alc in an analyte can also be detected by, for example, ELISA, radioimmunoassay, immunohistochemical method, or western blotting, using an antibody specific for each p3-Alc species. The "step of detecting p3-Alc in an analyte" may be performed by separating the desired p3-Alc from the analyte and then detecting the separated p3-Alc species. The p3-Alc separation from the analyte can be performed by a method that is used in peptide separation and is well known by those skilled in the art. The p3-Alc can be separated from the analyte by, for example, immunoprecipitation, western blotting, or affinity chromatography. The method for detecting the separated p3-Alc species is not limited as long as the method provides information about the amino acid sequence, molecular weight, physical property, or chemical property of the peptide, which enables the p3-Alc species identification, and can be performed by a method well known by those skilled in the art. Examples of such a method can include mass spectrometry, protein sequencers, western blotting, and ELISA.

The "step of calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11" can be performed by the steps of: obtaining quantitative information of the peptide consisting of the amino acid sequence of SEQ ID NO: 11 or 12 and quantitative information of the peptide consisting of the amino acid sequence of any one of SEQ ID NOs: 18 to 25 based on the information about the p3-Alc determined in the preceding step; and calculating the quantitative ratio of the peptide consisting of the amino acid sequence of any one of SEQ ID NOs: 18 to 20 to the peptide consisting of the amino acid sequence of SEQ ID NO: 12 or the quantitative ratio of the peptide consisting of the amino acid sequence of any one of SEQ ID NOs: 21 to 25 and the peptide consisting of the amino acid sequence of SEQ ID NO: 11 based on the obtained quantitative information (the denominator and the numerator are interchangeable).

When the p3-Alc species is assayed in combination with A-BETA, diagnosis can be performed by associating the quantitative ratio of the peptide consisting of the amino acid sequence of any one of SEQ ID NOs: 18 to 20 to the peptide consisting of the amino acid sequence of SEQ ID NO: 12 or the quantitative ratio of the peptide consisting of the amino acid sequence of any one of SEQ ID NOs: 21 to 25 to the peptide consisting of the amino acid sequence of SEQ ID NO: 11 and the A-BETA42/40 ratio, with the presence or absence of or the severity of the disease associated with the abnormality in gamma-secretase in the subject. Specifically, a subject having a larger quantitative ratio of the peptide consisting of the amino acid sequence of any one of SEQ ID NOs: 18 to 20 to the peptide consisting of the amino acid sequence of SEQ ID NO: 11 and a larger A-BETA42/40 ratio can be diagnosed as a patient having the disease associated with the abnormality in gamma-secretase or having the high severity of the disease.

In the present specification, the term "associating" used in diagnosing the disease associated with the abnormality in gamma-secretase in the patient relating to the secretion of the assayed p3-Alc species and the presence or absence of or the severity of the disease associated with the abnormality in gamma-secretase means comparing the presence, level, or abundance ratio of the p3-Alc species in the subject with that of the p3-Alc species in a patient with the disease associated with the abnormality in gamma-secretase, a patient known to be likely to have the disease associated with the abnormality in gamma-secretase, a patient already determined to have no disease associated with the abnormality in gamma-secretase, or a patient believed to have no disease associated with the abnormality in gamma-secretase. The level or ratio of the p3-Alc species in the patient used as a control can be determined, for example: according to the disclosure of the present invention; by determining the level of the p3-Alc species in an analyte derived from a patient whose disease associated with the abnormality in gamma-secretase or severity of the disease has already been determined; or by evaluating the p3-Alc species in combination with evaluation of another index for the disease associated with the abnormality in gamma-secretase. The level or ratio of the p3-Alc species can be used to determine the likelihood of the patient having the disease associated with the abnormality in gamma-secretase or the severity of the disease in the patient. The association of the level or ratio of the p3-Alc species with the disease associated with the abnormality in gamma-secretase can be performed by statistical analysis. Statistical significance is determined by comparing two or more populations and determining a confidence interval and/or a p value (Dowdy and Wearden, Statistics for Research, John Wiley & Sons, New York, 1983). The confidence interval of the present invention may be, for example, 90%, 95%, 98%, 99%, 99.5%, 99.9%, or 99.99%. The p value of the present invention may be, for example, 0.1, 0.05, 0.025, 0.02, 0.01, 0.005, 0.001, 0,0005, 0.0002, or 0.0001.

Preferably, the abundance or abundance ratio of the p3-Alc species can be associated with the disease associated with the abnormality in gamma-secretase or the severity thereof. For example, the threshold level of each p3-Alc species such as the peptide consisting of the amino acid sequence of SEQ ID NO: 12, 18, 19, or 20 may be set as an index for the occurrence of the disease associated with the abnormality in gamma-secretase or as an index for each stage of severity of the disease, and the level of each p3-Alc in the patient-derived sample can be compared with the threshold level for the association. Such a threshold level can be set to, for example, sensitivity of 50, 55, 60, 65, 70, 75, 80, 85, 90, 91, 92, 93, 94, 95, 96, 97, or 98% or higher. The threshold level can be set to specificity of 40, 45, 50, 55, 60, 65, 70, 75, 80, 85, 90, 91, 92, 93, 94, 95, 96, 97, or 98% or higher. The threshold level of the ratio between one or more types of p3-Alc species (e.g., p3-Alc, whose secretion is observed in healthy humans such as p3-Alcₐₗₚₕₐ35, p3-Alc_{beta}37, and p3-Alc_{gamma}31) and other p-Alc species may be set as an index for the occurrence of the disease associated with the abnormality in gamma-secretase or as an index for each stage of severity of the disease, and compared with the ratio of each p-Alc in the patient-derived sample can be compared with the threshold level for the association. Such a threshold level can be set to, for example, sensitivity of 50, 55, 60, 65, 70, 75, 80, 85, 90, 91, 92, 93, 94, 95, 96, 97, or 98% or higher. The threshold level can be set to specificity of 40, 45, 50, 55, 60, 65, 70, 75, 80, 85, 90, 91, 92, 93, 94, 95, 96, 97, or 98% or higher.

In the present specification, the term "associating" used in screening a therapeutic or preventive agent for the disease associated with the abnormality in gamma-secretase relating of the secretion of the assayed p3-Alc species with the presence or absence of or the degree of therapeutic or preventive effect on the disease associated with the abnormality in gamma-secretase means comparing the presence, level, or abundance ratio of the p3-Alc species in a culture solution of a cell, an animal model, or a human (patient) dosed with the test agent, with that of the p3-Alc species in a culture solution of a cell, an animal model, or a human (patient) undosed with the test agent or in a culture solution of a cell, an animal model, or a human (patient) dosed with a control agent. Specifically, the association can be performed according to the diagnostic method described above.

### Examples

Hereinafter, the present invention will be described more specifically with reference to Examples. However, the present invention is not intended to be limited to them. All documents cited herein are incorporated herein by reference in their entirety.

### Example 1. Construction of plasmids

As human alcadein cDNAs, hAlcₐₗₚₕₐ₁ cDNA (SEQ ID NO: 1: GenBank Accession No. AY753301), hAlc_{beta} cDNA (SEQ ID NO: 2: GenBank Accession No. NM_014718), and hAlc_{gamma} cDNA (SEQ ID NO: 3: GenBank Accession No. NM_022131) were used. By inserting a FLAG sequence between leucine at residue 38 and glutamic acid at residue 39 in hAlcₐₗₚₕₐ, pcDNA3-FLAG-hAlcₐₗₚₕₐ was prepared; by inserting a FLAG sequence between lysine at residue 26 and,proline at residue 27 in hAlc_{beta}, pCDNA3.1-FLAG-hAlc_{beta} was prepared; and by inserting a FLAG sequence between glutamine at residue 29 and arginine at residue 30 in hAlc_{gamma}, pcDNA3.1-FLAG-hAlc_{gamma} was prepared. pcDNA3-FLAG-APP₆₉₅ was prepared according to the method described in Ando, et al., (1999) J. Neurosci. 19, 4421-4427. pcDNA3-ADAM10-HA and pcDNA3-ADAM17-HA were prepared according to the method described in Endres, et al., (2005) FEBS J. 272, 5808-5820.

A plasmid pcDNA3-PS1wt containing cDNA encoding human presenilin 1 (hereinafter, referred to as "PS1") (SEQ ID NO: 4) was prepared according to the method described in Araki, et al., (2004) J. Biol. Chem. 279, 24343-24354. A mutation linked to familial Alzheimer's disease (FAD) was introduced in PS1 using PCR. Specifically, the following plasmids were prepared: a plasmid pcDNA4-PS1M146L encoding a mutant PS1 wherein methionine is replaced with leucine at residue 146 of the wequence of PS1; a plasmid pcDNA4-PS1L166P encoding a mutant PS1 wherein leucine is replaced with proline at residue 166 of the sequence of PS1; a plasmid pcDNA4-PS1A246E encoding a mutant PS1 wherein alanine is replaced with glutamic acid at residue 246 of the sequence of PS1; a plasmid pcDNA4-PS1R278T encoding a mutant of the PS1 wherein arginine is replaced with threonine at residue 278 of the sequence of PS1; a plasmid pcDNA4-PS1L286V encoding a mutant PS1 wherein leucine is replaced with valine at residue 286 of the sequence of PS1; a plasmid pcDNA4-PS1A434C encoding a mutant PS1 wherein alanine is replaced with cysteine at residue 434of the sequence of PS1; and a plasmid pcDNA4-PS1D385A encoding a mutant PS1 wherein aspartic acid is replaced with alanine at residue 385 of the sequence of PS1. HEK293 cells were transfected with these plasmids and used in experiments described below. Cells stably expressing PS1 were cloned.

### Example 2. Preparation of antibodies

Rabbit anti-Alcₐₗₚₕₐ polyclonal antibodies UT134 and UT135 were obtained as antibodies against a peptide consisting of an amino acid sequence from residues 821 to 843 in human Alcₐₗₚₕₐ₁ and cysteine added thereto (FVHPEHRSFVDLSGHNLANPHPF+C: SEQ ID NO: 5) and as antibodies against a peptide consisting of an amino acid sequence from residues 839 to 851 in human Alcₐₗₚₕₐ₁ and cysteine added thereto (NPHPFAVVPSTAT+C: SEQ ID NO: 6), respectively. An anti-Alcₐₗₚₕₐ monoclonal antibody 3B5 was obtained as an antibody against a peptide consisting of an amino acid sequence from residues 821 to 826 in human Alcₐₗₚₕₐ₁ and cysteine added thereto (C+FVHPEH: SEQ ID NO: 7). Rabbit anti-Alc_{beta} antibodies UT142 and UT143 were obtained as antibodies against a peptide consisting of an amino acid sequence from residues 837 to 849 in human Alc_{beta} and cysteine added thereto (SSHRNSMIPSAAT+C: SEQ ID NO: 8) and as antibodies against a GST fusion protein comprising an amino acid sequence from residues 819 to 847 in human Alc_{beta} (FLHRGHQPPPEMAGHSLASSHRNSMIPSA: SEQ ID NO: 9), respectively. A rabbit anti-Alc_{gamma} polyclonal antibody UT166 was obtained as an antibody against a peptide consisting of an amino acid sequence from residues 823 to 834 in human Alc_{gamma} and cysteine added thereto (C+IQHSSWPSIAT: SEQ ID NO: 10). These Alc-specific antibodies were prepared against the extracellular juxtamembrane region of each Alc family protein and were specific for their respective corresponding p3-Alc targets. These antibodies were used in the isolation and detection of p3-Alc. An anti-Alcₐₗₚₕₐ cytoplasmic domain antibody UT83 and an anti-Alc_{beta} cytoplasmic domain antibody UT99 were prepared according to the known method (Araki et al., (2004) J. Biol. Chem. 279, 24343-24354). Commercially available products were used as an anti-FLAG monoclonal antibody (M2, Sigma-Aldrich, Inc.) and an anti-HA antibody (12CA5, BD Biosciences). Anti-mouse and anti-rabbit peroxidase-linked species-specific whole antibodies were purchased from GE Healthcare.

### Example 3. Cleavage of Alc and APP in ADAM10 gene knockout mouse-derived embryonic fibroblasts

### (1) Preparation of cells

ADAM10 gene homozygous knockout mouse (-/-)-derived mouse embryonic fibroblasts (MEFs) (hereinafter, referred to as "ADAM (-/-) MEFs") and ADAM10 gene heterozygous knockout mouse (+/-)-derived mouse embryonic fibroblasts (hereinafter, referred to as "ADAM (+/-) MEFs") were prepared according to the known method (Hartmann et al., (2002) Hum. Mol. Gen. 11, 2615-2624).

### (2) Gene transfer

FLAG-Alcₐₗₚₕₐ, FLAG-Alc_{beta}, FLAG-Alc_{gamma}, or FLAG-APP was introduced and expressed in ADAM (-/-) MEFs and ADAM (+/-) MEFs. The gene transfer was performed by transfecting MEFs (0.3 to 1.0×10⁶ cells) with the pcDNA3-FLAG-hAlcₐₗₚₕₐ, pcDNA3.1-FLAG-hAlc_{beta}, pcDNA3.1-FLAG-hAlc_{gamma}, or pcDNA3-FLAG-APP₆₉₅ plasmid prepared in Example 1 with Lipofectamine 2000 or LipofectAMINE (both Invitrogen Corp.) according to the protocol of the manufacturer. 24 hours after the transfection, the medium was replaced with a fresh one, and the cells were cultured for additional 24 hours.

### (3) Collection of immunoprecipitate in culture solution and cell lysate and western blotting

sAlc and sAPP were collected from the conditioned medium by immunoprecipitation using the anti-FLAG antibody and protein G Sepharose. To analyze cellular proteins, the cells were collected and dissolved in Hepes-buffered saline with Triton X-100 (HBST) (Araki et al., (2004) J. Biol. Chem. 279, 24343-24354). The cell lysate and the immunoprecipitate were subjected to western blotting using the antibodies prepared in Example 2. The proteins were detected using ECL (GE Healthcare) and quantified using a VersaDoc Imaging System (Bio-Rad Laboratories, Inc.).

### (4) Results

The results are shown in Figure 1. The primary cleavage of APP could not be confirmed in ADAM (-/-) MEFs but was confirmed in ADAM (+/-) MEFs (Figure 1D). ADAM (-/-) MEFs secreted sAPP (lane 2 of Figure 1D) 40% lower than ADAM (+/-) MEFs (lane 1 of Figure 1D). The amounts of sAlcₐₗₚₕₐ, sAlc_{beta}, and sAlc_{gamma} secreted by ADAM (-/-) MEFs were as low as that of APP (Figures 1A to 1C). These results demonstrated that as with APP, Alcₐₗₚₕₐ, Alc_{beta}, and Alc_{gamma} undergo primary cleavage by ADAM10.

### Example 4. Cleavage of Alc and APP by transfecting ADAM10 gene into ADAM10 gene knockout mouse-derived mouse embryonic fibroblasts

FLAG-Alcₐₗₚₕₐ, FLAG-Alc_{beta}, FLAG-Alc_{gamma}, or FLAG-APP and ADAM10 were introduced and expressed in ADAM (-/-) MEFs obtained in the same way as in Example 3. The gene transfer was performed by transfecting MEFs (0.3 to 1.0×10⁶ cells) with the plasmids pcDNA3-FLAG-hAlcₐₗₚₕₐ (0.5 microg), pcDNA3.1-FLAG-hAlc_{beta} (0.25 microg), pcDNA3.1-FLAG-hAlc_{gamma} (0.5 microg), or pcDNA3-FLAG-APP₆₉₅ (0.5 microg) and pcDNA3-ADAM10-HA (1.5 microg or 4.5 microg) prepared in Example 1 with Lipofectamine 2000 or LipofectAMINE (both Invitrogen Corp.) according to the protocol of the manufacturer. 24 hours after the transfection, the medium was replaced with a fresh one, and the cells were cultured for additional 24 hours. Then, an immunoprecipitate from the culture solution and a cell lysate were collected and subjected to western blotting in the same way as in Example 3.

The results are shown in Figure 2. The expression of exogenous ADAM10 restored sAPP secretion (Figure 2D). The amount of sAPP secreted by ADAM (-/-) MEFs secreting exogenous ADAM10 was about 1.7 fold over that of sAPP secreted by ADAM (-/-) MEFs. In ADAM MEFs, the expression level of intracellular mature APP (mAPP) that serves as a substrate for ADAM10 and generates sAPP was reduced in response to the expression of exogenous ADAM10. The amounts of sAlcₐₗₚₕₐ, sAlc_{beta}, and sAlc_{gamma} secreted by ADAM (-/-) MEFs were restored in response to the expression of exogenous ADAM10 (Figures 2A to 2C). The amount of sAlc secreted by ADAM (-/-) MEFs exogenous ADAM10 was 1.8 to 2.2 times larger than that of sAlc secreted by ADAM (-/-) MEFs. These results demonstrated that ADAM10 is a main enzyme digesting Alcₐₗₚₕₐ, Alc_{beta}, and Alc_{gamma}.

### Example 5. Cleavage of Alc and APP by transfecting ADAM17 gene into N2a cells

FLAG-Alcₐₗₚₕₐ, FLAG-Alc_{beta}, FLAG-Alc_{gamma}, or FLAG-APP and ADAM17 were introduced and expressed in N2a cells (purchased from ATCC). The gene transfer was performed by transfecting N2a (0.3 to 1.0×10⁶ cells) with the plasmids pcDNA3-FLAG-hAlcₐₗₚₕₐ (0.5 microg), pcDNA3.1-FLAG-hAlc_{beta} (0.5 microg), pcDNA3.1-FLAG-hAlc_{gamma} (1.0 microg), or pcDNA3-FLAG-APP₆₉₅ (0.5 microg), and pcDNA3-ADAM17-HA (0.5 microg) or 1.5 microg) prepared in Example 1 with Lipofectamine 2000 or LipofectAMINE (both Invitrogen Corp.) according to the protocol of the manufacturer. 24 hours after the transfection, the medium was replaced with a fresh one, and the cells were cultured for additional 24 hours. Then, an immunoprecipitate from the culture solution and a cell lysate were collected and subjected to western blotting in the same way as in Example 3.

The results are shown in Figure 3. In the N2a cells, ADAM17 cleaved APP (Figure 3D). The amount of secreted sAPP increased by increasing expression level of ADAM17. This showed that exogenously expressed ADAM17 has APP-cleaving activity. The amount of sAlc (sAlcₐₗₚₕₐ, sAlc_{beta}, and sAlc_{gamma}) secreted by the N2a cells expressing exogenous ADAM17 increased in response to the expression level of exogenous ADAM17. Similarly to the results of ADAM10, these results demonstrated that ADAM17 cleaves Alcₐₗₚₕₐ, Alc_{beta}, and Alc_{gamma}, as with APP.

### Example 6. Identification and analysis of p3-Alc secreted into culture solution

6 microg of pcDNA3-hAlcₐₗₚₕₐ₁, pcDNA3.1-hAlc_{beta}, or pcDNA3.1-hAlc_{gamma} was introduced to 8 to 9×10⁶ HEK293 cells for 24 hours with Lipofectamine 2000. p3-Alcₐₗₚₕₐ, p3-Alc_{beta}, and p3-Alc_{gamma} secreted into 6 mL each of the media were collected by immunoprecipitation using 25 microg of the affinity-purified anti-p3-Alcₐₗₚₕₐ polyclonal antibody UT135, 100 microL serum containing the anti-p3-Alc_{beta} polyclonal antibody UT143, and 100 microL serum containing the anti-p3-Alc_{gamma} polyclonal antibody UT166, respectively, and protein G Sepharose beads.

The beads were subsequently washed with a washing buffer I (10 mM Tris-HCl (pH 8.0), 140 mM NaCl, 0.1% (w/v) n-octyl-D-glucose, 0.025% (w/v) sodium azide) and a washing buffer II (10 mM Tris-HCl (pH 8.0), 0.025% (w/v) sodium azide). Then, the analytes were eluted with sinapinic acid saturated trifluoroacetic acid/acetonitrile/water (1:20:20). The eluted analytes were dried on target plates and analyzed by MALDI-TOF/MS using Voyager-DE PRO MALDI-TOF Mass Spectrometer (PerSeptive Biosystems, Inc.) or Ultraflex II TOF/TOF (Bruker Daltonics Inc.). The molecular weights were calibrated using ProteoMass ACTH Fragment 18-39 MALDI-MS Standard and Insulin for Voyager-DE PRO MALDI-TOF Mass Spectrometer (Sigma-Aldrich, Inc.) or Peptide Calibration Standard for Ultraflex II TOF/TOF (Bruker Daltonics Inc.). The amino acid sequence of peptide in each major peak was determined by MALDI-MS/MS analysis.

The results are shown in Figure 4. The p3-Alcₐₗₚₕₐ spectrum showed a major peak at molecular weights of 3804 and 4007 (Figure 4A). The p3-Alc_{beta} spectrum showed a major peak at molecular weights of 3964 and 4303 (Figure 4B). The p3-Alc_{gamma} spectrum showed a major peak at a molecular weight of 3377 and a minor peak at a molecular weight of 3689 (Figure 4C). The amino acid analysis showed the major peaks of p3-Alcₐₗₚₕₐ consisted of 35 and 37 amino acids; the major peaks of p3-Alc_{beta} consisted of 37 and 40 amino acids; and the major peak of p3-Alc_{gamma} consisted of 31 and 34 amino acids (middle and right diagrams of Figure 4). Each p3-Alc sequence elucidated by the present experiment is shown in Figure 5. p3-Alcₐₗₚₕₐ35 and p3-Alcₐₗₚₕₐ2N+35 secreted by the HEK293 cells expressing Alcₐₗₚₕₐ₁ are major p3-Alcₐₗₚₕₐ species and were a peptide comprising a sequence from alanine at residue 817 (Ala⁸¹⁷) to threonine at residue 851 (Thr⁸⁵¹) in human Alcₐₗₚₕₐ (SEQ ID NO: 11) and a peptide comprising a sequence from alanine at residue 815 (Ala⁸¹⁵) to threonine at residue 851 (Thr⁸⁵¹) in human Alcₐₗₚₕₐ (SEQ ID NO: 12), respectively. p3-Alc_{beta}37 and p3-Alc_{beta}40 secreted by the HEK293 cells expressing Alc_{beta} are major p3-Alc_{beta} species and were a peptide comprising a sequence from valine at residue 813 (Val⁸¹³) to threonine at residue 849 (Thr⁸⁴⁹) in human Alc_{beta} (SEQ ID NO: 13) and a peptide comprising a sequence from valine at residue 813 (Val⁸¹³) to isoleucine at residue 852 (Ile⁸⁵²) in human Alc_{beta} (SEQ ID NO: 14), respectively. p3-Alc_{gamma}31 and p3-Alc_{gamma}34 secreted by the HEK293 cells expressing Alc_{gamma} are major p3-Alc_{gamma} species and were a peptide comprising a sequence from leucine at residue 804 (Leu⁸⁰⁴) to threonine at residue 834 (Thr⁸³⁴) in human Alc_{gamma} (SEQ ID NO: 15) and a peptide comprising a sequence from leucine at residue 804 (Leu⁸⁰⁴) to isoleucine at residue 837 (Ile⁸³⁷) in human Alc_{gamma} (SEQ ID NO: 16), respectively.

### (Primary cleavage site)

The present inventors had identified alanine at residue 816 (Ala⁸¹⁶) (number in the Alcₐₗₚₕₐ₁ isoform) as the terminal amino acid of AlcₐₗₚₕₐCTF using a gas-phase protein sequencer in the previous report. From this, the present inventors deduced that ADAM10 and ADAM17 cleave the peptide bond between methionine at residue 815 (Met⁸¹⁵) and alanine at residue 816 (Ala⁸¹⁶). Thus, the N-terminal amino acids of p3-Alcₐₗₚₕₐ35 and p3-Alcₐₗₚₕₐ2N+35 were analyzed by MALDI-MS/MS and consequently confirmed to be alanine at residue 817 (Ala⁸¹⁷) and methionine at residue 815 (Met⁸¹⁵), respectively (Figure 4A). Accordingly, the N-terminal Met⁸¹⁵ and/or Ala⁸¹⁶ of AlcₐₗₚₕₐCTF formed by primary cleavage were shown to be removed by amino-terminal exopeptidase during the course of p3-Alcₐₗₚₕₐ secretion.

Although the terminal amino acid of Alc_{beta}CTF had been unsuccessfully identified using the gas-phase protein sequencer, the N-terminal amino acid of p3-Alc_{beta}37 was analyzed by MALDI-MS/MS and consequently confirmed to be valine at residue 813 (Val⁸¹³) in human Alc_{beta} (Figure 4B). This suggests that human Alc_{beta}CTF is a peptide of 15.5 kDa in molecular weight which consists of 143 amino acids and contains valine at residue 813 in human Alc_{beta} as the N-terminal amino acid.

Likewise, the present inventors had identified the amino-terminal amino acid of Alc_{gamma}CTF (824-LIVQPPFLQ-832: SEQ ID NO: 17) using the gas-phase protein sequencer. This result was consistent with the results of MALDI-MS/MS analysis (Figure 4C). Accordingly, the primary cleavage site in Alc_{gamma} was confirmed to be the peptide bond between histidine at residue 803 (His⁸⁰³) and leucine at residue 804 (Leu⁸⁰⁴).
The primary cleavage sites in the Alc family proteins are indicated in Figure 5 with the up-pointing arrows under each sequence. For comparison, primary cleavage sites (alpha- and beta-sites) in APP₆₉₅ are also indicated in Figure 5 in the same way as above.

### (Secondary cleavage site)

Also, secondary cleavage sites in Alcₐₗₚₕₐ, Alc_{beta}, and Alc_{gamma} were identified by MALDI-TOF/MS. The major secondary cleavage site in Alcₐₗₚₕₐ was the peptide bond between threonine at residue 851 (Thr⁸⁵¹) and valine at residue 852 (Val⁸⁵²), at which the cleavage formed p3-Alcₐₗₚₕₐ35. The additional secondary cleavage site in Alcₐₗₚₕₐ was the peptide bond between valine at residue 853 (Val⁸⁵³) and isoleucine at residue 854 (IIe⁸⁵⁴), at which the cleavage formed p3-Alcₐₗₚₕₐ2N+35 (Figure 4A). The major secondary cleavage site in Alc_{beta} was the peptide bond between threonine at residue 849 (Thr⁸⁴⁹) and leucine at residue 850 (Leu⁸⁵⁰), at which the cleavage formed p3-Alc_{beta}37. The additional secondary cleavage site in Alc_{beta} was the peptide bond between isoleucine at residue 851 (IIe⁸⁵¹) and isoleucine at residue 852 (Ile⁸⁵²), at which the cleavage formed p3-Alc_{beta}40 (Figure 4B). The major secondary cleavage site in Alc_{gamma} was the peptide bond between threonine at residue 834 (Thr⁸³⁴) and valine at residue 835 (Val⁸³⁵), at which the cleavage formed p3-Alc_{gamma}31. The additional secondary cleavage site in Alc_{gamma} was the peptide bond between isoleucine at residue 837 (Ile⁸³⁷) and isoleucine at residue 838 (Ile⁸³⁸), at which the cleavage formed p3-Alc_{gamma}34 (Figure 4C).

The secondary cleavage sites in the Alc family proteins determined by MALDI-MS/MS analysis are indicated in Figure 5 with the down-pointing arrows above each sequence. For comparison, gamma-cleavage sites in APP₆₉₅, which form A-BETA40 (arrow 40) and A-BETA42 (arrow 42), are also indicated in Figure 5 in the same way as above.

### Example 7. Regulation of gamma-cleavage site in Alc by familial Alzheimer's disease-linked mutant presenilin

### (1) Preparation of cells

HEK293 cells expressing wild-type PS1 (WT) (SEQ ID NO: 4), PS1 having a substitution of methionine at residue 146 by leucine (Met146Leu: M146L), a substitution of leucine at residue 166 by proline (Leu166Pro: L166P), a substitution of alanine at residue 246 by glutamic acid (Ala246Glu: A246E), a substitution of arginine at residue 278 by threonine (Arg278Thr: A278T), a substitution of leucine at residue 286 by valine (Leu286Val: L286V), or a substitution of alanine at residue 434 by cysteine (Ala434Cys: A434C) as a mutation linked to familial Alzheimer's disease, or a vector (Mock) was transformed with pcDNA3-FLAG-hAlcₐₗₚₕₐ, pcDNA3.1-FLAG-hAlc_{beta}, pcDNA3.1-FLAG-hAlc_{gamma}, or pcDNA3-FLAG-APP₆₉₅ or in the absence of these plasmids. HEK293 cells expressing a PS1 mutant having a substitution of alanine at residue 79 by valine (Ala79Val: A79V), a PS1 mutant having a substitution of histidine at residue 163 by arginine (His163Arg), or a PS1 mutant having a substitution of isoleucine at residue 143 by threonine (Ile143Thr: I143T) was transformed with using pcDNA3-FLAG-hAlcₐₗₚₕₐ.

### (2) Collection of immunoprecipitate in culture solution

p3-Alcₐₗₚₕₐ, p3-Alc_{beta}, and p3-Alc_{gamma} secreted into media were collected by immunoprecipitation using 25 microg of the affinity-purified anti-p3-Alcₐₗₚₕₐ polyclonal antibody UT135, 100 microL serum containing the anti-p3-Alc_{beta} polyclonal antibody UT143, and 100 microL serum containing the anti-p3-Alc_{gamma} polyclonal antibody UT166, respectively, and protein G Sepharose beads.

### (3) Mass spectrometry

The beads were subsequently washed with a washing buffer I (10 mM Tris-HCl (pH 8.0), 140 mM NaCl, 0.1% (w/v) n-octyl-D-glucose, 0.025% (w/v) sodium azide) and a washing buffer II (10 mM Tris-HCl (pH 8.0), 0.025% (w/v) sodium azide). Then, the analytes were eluted with sinapinic acid saturated in trifluoroacetic acid/acetonitrile/water (1:20:20). The eluted analytes were dried on target plates and analyzed by MALDI-TOF/MS using Voyager-DE PRO MALDI-TOF Mass Spectrometer (PerSeptive Biosystems, Inc.) or Ultraflex II TOF/TOF (Bruker Daltonics Inc.). The molecular weights were calibrated using ProteoMass ACTH Fragment 18-39 MALDI-MS Standard and Insulin for Voyager-DE PRO MALDI-TOF Mass Spectrometer (Sigma-Aldrich, Inc.) or Peptide Calibration Standard for Ultraflex II TOF/TOF (Bruker Daltonics Inc.). The amino acid sequence of a peptide in each major peak was determined by MALDI-MS/MS analysis.

The expression of PS1 was confirmed by western blotting using anti-PS1 N-terminus and anti-PS1 C-terminus antibodies (not shown). The effects of the mutations linked to familial Alzheimer's disease were confirmed based on characteristics to increase the A-BETA42/A-BETA40 ratio in the culture solution of the HEK293 cells stably expressing PS and APP₆₉₅.

### (4) Results

The results are shown in Figures 6 and 7. Figure 6 shows the results of p3-Alcₐₗₚₕₐ. The HEK293 cells stably expressing wild-type PS1 (WT) or vector (Mock) produced a p3-Alcₐₗₚₕₐ species p3-Alcₐₗₚₕₐ"37" which is with molecular weight of 4002 and consisting of 37 amino acids (Figure 6A). As a result of analyzing the amino acid sequence of this p3-Alcₐₗₚₕₐ by MALDI-MS/MS it was confirmed to be not p3-Alcₐₗₚₕₐ37, but p3-Alcₐₗₚₕₐ2N+35 comprising a sequence from methionine at residue 815 (Met⁸¹⁵) to threonine at residue 851 (Thr⁸⁵¹) in human Alcₐₗₚₕₐ₁. The HEK293 cells expressing wild-type PS1 (WT) or vector (Mock) produced p3-Alcₐₗₚₕₐ"35" which is with molecular weight of 3804 and consisting of 35 amino acids (Figure 6A). As a result of analyzing the amino acid sequence, the amino acid sequence of this p3-Alcₐₗₚₕₐ"35" was identical with that of p3-Alcₐₗₚₕₐ35 shown in Figure 5. Accordingly, the HEK293 cells expressing wild-type PS1 (WT) produced p3-Alcₐₗₚₕₐ species having Alcₐₗₚₕₐ₁ Thr⁸⁵¹ at the C-terminus.

On the other hand, the HEK293 cells expressing the PS1 mutation linked to familial Alzheimer's disease produced abnormal p3-Alcₐₗₚₕₐ species, in addition to p3-Alcₐₗₚₕₐ35 and p3-Alcₐₗₚₕₐ2N+35. Analysys of these abnormal p3-Alcₐₗₚₕₐ species showed the levels of a peptide p3-Alcₐₗₚₕₐ2N+38 (SEQ ID NO: 18) consisting of a sequence from methionine at residue 815 (Met⁸¹⁵) to isoleucine at residue 854 (Ile⁸⁵⁴) in human Alcₐₗₚₕₐ, a peptide p3-Alcₐₗₚₕₐ2N+39 (SEQ ID NO: 19) consisting of a sequence from methionine at residue 815 (Met⁸¹⁵) to valine at residue 855 (Val⁸⁵⁵) in human Alcₐₗₚₕₐ, and a peptide p3-Alcₐₗₚₕₐ2N+40 (SEQ ID NO: 20) consisting of a sequence from methionine at residue 815 (Met⁸¹⁵) to valine at residue 856 (Val⁸⁵⁶) in human Alcₐₗₚₕₐ were elevated, particularly, in the culture solution of the cells expressing PS1 having the L166P mutation (Figure 6A). The correlation of the ratios of these minor p3-Alcₐₗₚₕₐ species to p3-Alcₐₗₚₕₐ2N+35 with the ratio of A-BETA42 to A-BETA40 (hereinafter, referred to as "A-BETA42/40") is shown in Figure 6B. Specifically, Figure 6B(1) shows p3-Alcₐₗₚₕₐ2N+34/p3-Alcₐₗₚₕₐ2N+35; Figure 6B(2) shows p3-Alcₐₗₚₕₐ2N+36/p3-Alcₐₗₚₕₐ2N+35; Figure 6B(3) shows p3-Alcₐₗₚₕₐ2N+37/p3-Alcₐₗₚₕₐ2N+35; and Figure 6B(4) shows p3-Alcₐₗₚₕₐ2N+38/p3-Alcₐₗₚₕₐ2N+35. The p3-Alcₐₗₚₕₐ2N+34/p3-Alcₐₗₚₕₐ2N+35 ratio and the p3-Alcₐₗₚₕₐ2N+38/p3-Alcₐₗₚₕₐ2N+35 ratio correlated with A-BETA42/40 (R²>0.5). No remarkable production of p3-Alcₐₗₚₕₐ was observed in the exogenous Alcₐₗₚₕₐ-untransfected HEK293 cells or the HEK293 cells expressing PS1 having the D385A mutation (not shown). Accordingly, it was demonstrated that some PS1 mutations linked to familial Alzheimer's disease influence the digestion of Alcₐₗₚₕₐ by gamma-secretase.

Figure 7C shows the results of p3-Alc_{gamma}. The HEK293 cells stably expressing wild-type PS1 (WT) or vector (Mock) and Alc_{gamma} produced p3-Alc_{gamma}31 which is with molecular weight of 3377 and consisting of 31 amino acids and/or p3-Alc_{gamma}34. The amino acid sequence of p3-Alc_{gamma}31 was identical with that of p3-Alc_{gamma}31 shown in Figure 5.

Figure 7C also shows results of analysis of p3-Alc_{gamma} produced from the HEK293 cells coexpressing Alc_{gamma} and PS1 having the mutation linked to familial Alzheimer's disease. In the HEK293 cells expressing the PS1 mutant linked to familial Alzheimer's disease, the L166P and R278T mutations caused decrease in p3-Alc_{gamma}31 level and increase in p3-Alc_{gamma}34 level. Results of correlating the ratio of p3-Alc_{gamma}30, p3-Alc_{gamma}32, or p3-Alc_{gamma}34 to p3-Alc_{gamma}31 with the A-BETA42/40 ratio are shown in Figure 7D. In the PS1 mutants, the degree of change in the cleavage of p3-Alc_{gamma} by gamma-secretase correlated with increase in the ratio of A-BETA42 production (A-BETA42/40) (R²>0.5). No remarkable production of p3-Alc_{gamma} was confirmed in the HEK293 cells non-expressing exogenous Alc_{gamma} or the cells expressing PS1 having the D385A mutation. These results demonstrated that Alc_{gamma} also undergoes secondary cleavage by PS-dependent gamma-secretase. It was also demonstrated that p3-Alc_{gamma} can be used as a reliable surrogate for evaluating the incorrect processing of A-BETA because some PS1 mutations linked to familial Alzheimer's disease cause generation of C-terminus extended p3-Alc_{gamma}, as with p3-Alcₐₗₚₕₐ and A-BETA.

Figure 7B shows the results of p3-Alc_{beta}. The cells (WT) expressing wild-type PS1 and Alc_{beta} (WT)and the HEK293 cells stably expressing vector and Alc_{beta} (Mock) produced one minor p3-Alc_{beta} species p3-Alc_{beta}39, in addition to two major p3-Alc_{beta} species p3-Alc_{beta}37 and p3-Alc_{beta}40 (Figure 7A).

Figure 7A also shows results of analysis of p3-Alc_{beta} produced from the HEK293 cells coexpressing Alc_{beta} and PS1 having the mutation linked to familial Alzheimer's disease. In the HEK293 cells expressing the PS1 mutant linked to familial Alzheimer's disease, the R278T and A434C mutations caused decrease in p3-Alc_{beta}37 level and increase in p3-Alc_{beta}40 level. Results of correlating the ratio of p3-Alc_{beta}38, p3-Alc_{beta}39, or p3-Alc_{beta}40 to p3-Alc_{beta}37 with the A-BETA42/40 ratio are shown in Figure 7B. In the PS1 mutants, the degree of change in the cleavage of p3-Alc_{beta} by gamma-secretase correlated with increase in the ratio of A-BETA42 production (A-BETA42/40).

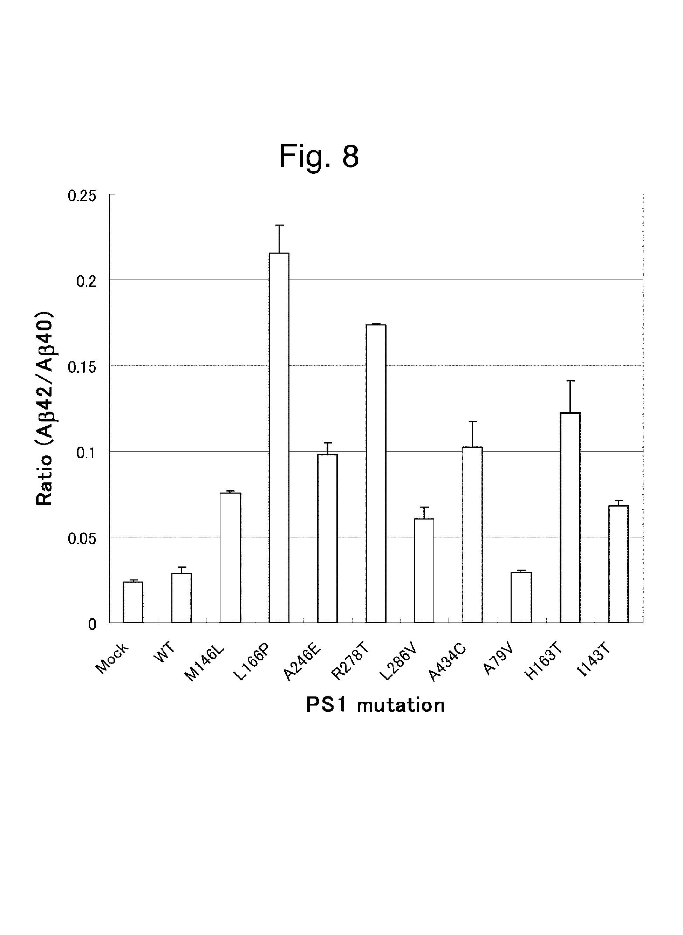

The values of A-BETA42/40 determined in the present experiment are shown in Figure 8. The familial Alzheimer's disease-linked PS1 mutants L166P, R278T, A434C, A246E, and M146L, which increase the A-BETA42/40 ratio, also changed the gamma-cleavage of Alcalpha. The L166P mutant changed the ratios p3-Alcₐₗₚₕₐ2N+38/p3-Alcₐₗₚₕₐ35, p3-Alcₐₗₚₕₐ2N+37/p3-Alcₐₗₚₕₐ35, p3-Alc_{beta}39/p3-Alc_{beta}37, p3-Alc_{gamma}34/p3-Alc_{gamma}31, and p3-Alc_{gamma}30/p3-Alc_{gamma}31. The R278T mutant changed the ratios p3-Alc_{beta}39/p3-Alc_{beta}37, p3-Alc_{beta}40/p3-Alc_{beta}37, and p3-Alc_{gamma}34/p3-Alc_{gamma}31. The A434 mutant changed the ratios p3-Alcₐₗₚₕₐ2N+36/p3-Alcₐₗₚₕₐ35 and p3-Alc_{beta}40/p3 - Alc_{beta}37. The A246E mutant changed the ratios p3-Alc_{beta}39/p3-Alc_{beta}37 and p3-Alc_{beta}40/p3-Alc_{beta}37. The M146L mutant changed the ratios p3-Alcₐₗₚₕₐ2N+38/p3-Alcₐₗₚₕₐ35 and p3-Alc_{gamma}30/p3-Alc_{gamma}31. These results demonstrated that as with APP, Alc was cleaved by a PS-dependent gamma-secretase complex, and that PS1 mutations linked to familial Alzheimer's disease cause gamma-cleavage at incorrect sites of Alc family proteins. Accordingly, it was confirmed that p3-Alc can be used as a surrogate marker for the incorrect cleavage of A-BETA by PS1 mutants.

### Example 8. Assay on secretion of p3-Alc_{beta} by PS1-deficient cells

### (1) Preparation of cells

PS1/PS2 gene double knockout mouse (PS1/2 -/-)-derived MEFs (hereinafter, referred to as "PS1/2 (-/-) MEFs") and wild-type mouse (PS1/2 +/+)-derived MEFs (hereinafter, referred to as "PS1/2 (+/+) MEFs") were prepared according to the known method (Herreman et al., (2000) Nat. Cell Biol., 2, 461-462).

### (2) Gene transfer

FLAG-Alc_{beta} was introduced and expressed in PS1/2 (-/-) MEFs and PS1/2 (+/+) MEFs. The gene transfer was performed by transfecting MEFs (0.3 to 1.0×10⁶ cells) with the plasmid pcDNA3.1-FLAG-hAlc_{beta} prepared in Example 1 with Lipofectamine 2000 or LipofectAMINE (both Invitrogen Corp.) according to the protocol of the manufacturer. 24 hours after the transfection, the medium was replaced by a fresh one, and the cells were cultured for additional 24 hours.

### (3) Collection of immunoprecipitate in culture solution and cell lysate and western blotting

p3-Alc_{beta} secreted into the medium was collected by immunoprecipitation using 100 microL serum containing the anti-p3-Alc_{beta} polyclonal antibody UT143 and protein G Sepharose beads. To analyze cellular proteins, the cells were collected and dissolved in Hepes-buffered saline with Triton X-100 (HBST) (Araki et al., (2004) J. Biol. Chem. 279, 24343-24354). The cell lysate and the immunoprecipitate were subjected to western blotting using the antibodies prepared in Example 2. The proteins were detected using ECL (GE Healthcare) and quantified using VersaDoc Imaging System (Bio-Rad Laboratories, Inc.).

### (4) Mass spectrometry

The beads were subsequently washed with a washing buffer I (10 mM Tris-HCl (pH 8.0), 140 mM NaCl, 0.1% (w/v) n-octyl-D-glucose, 0.025% (w/v) sodium azide) and a washing buffer II (10 mM Tris-HCl (pH 8.0), 0.025% (w/v) sodium azide). Then, the analytes were eluted with sinapinic acid saturated in trifluoroacetic acid/acetonitrile/water (1:20:20). The eluted analytes were dried on target plates and analyzed by MALDI-TOF/MS using Voyager-DE PRO MALDI-TOF Mass Spectrometer (PerSeptive Biosystems, Inc.) or Ultraflex II TOF/TOF (Bruker Daltonics Inc.). The molecular weights were calibrated using ProteoMass ACTH Fragment 18-39 MALDI-MS Standard and Insulin for Voyager-DE PRO MALDI-TOF Mass Spectrometer (Sigma-Aldrich, Inc.) or Peptide Calibration Standard for Ultraflex II TOF/TOF (Bruker Daltonics Inc.). The amino acid sequence of a peptide in each major peak was determined by MALDI-MS/MS analysis.

### (5) Results

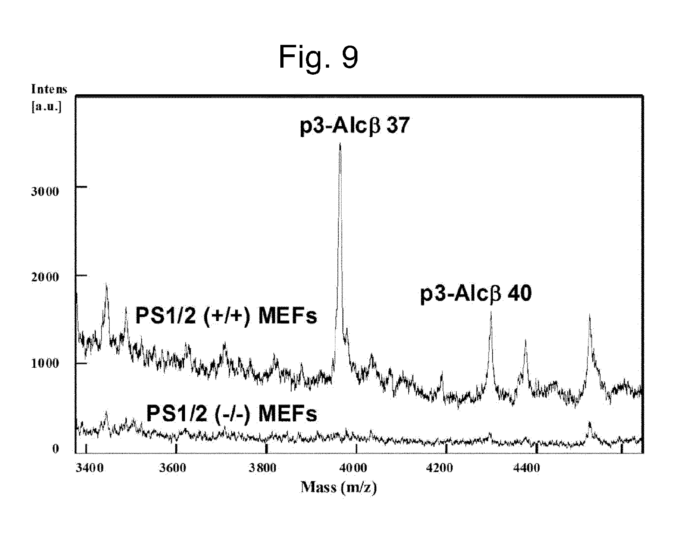

The results are shown in Figures 9 and 10. Alc_{beta} was expressed in PS1/2 (-/-) MEFs and the corresponding wild-type control PS1/2 (+/+) MEFs. Although PS1/2 (-/-) MEFs and the corresponding wild-type control PS1/2 (+/+) MEFs expressed the same levels of Alc_{beta}. Nevertheless, mass spectrometry detected both p3-Alc_{beta}37 and p3-Alc_{beta}40 were detected from the culture solution of the control PS1/2 (+/+) MEFs but not from the culture solution of PS1/2 (-/-) MEFs (Figure 9).

Figure 10 shows results of assaying the accumulation of Alc_{beta}CTF (C-terminal fragment of primary cleavage production of Alc_{beta}) in PS1/2 (-/-) MEFs. Figure 10 shows results of analyzing the cell lysates of PS1/2 (-/) MEFs and the corresponding wild-type control PS1/2 (+/+) MEFs by western blotting. Western blotting using the anti-Alc_{beta} cytoplasm antibody UT99 detected both approximately 21.5-kDa Alc_{beta}CTF and full-length Alc_{beta} from the cell lysate of the wild-type control PS1/2 (+/+) MEFs. This showed that these cells express primary cleavage enzymes such as ADAM10 and ADAM17. Alc_{beta}CTF accumulated in PS1/2 (-/-) MEFs. These results demonstrated that Alc_{beta} is a substrate for a PS-dependent gamma-secretase complex.

### Example 9. Cleavage by PS1 having D385A mutation

p3-Alc_{beta} and p3-Alcₐₗₚₕₐ in culture solutions were collected by immunoprecipitation and confirmed by western blotting together with intracellular Alc_{beta} and Alc_{beta}CTF or with intracellular Alcₐₗₚₕₐ and AlcₐₗₚₕₐCTF. Specifically, Alc_{beta}-untransfected HEK293 cells, Alc_{beta}-transfected HEK293 cells expressing wild-type PS1, or Alc_{beta}-transfected HEK293 cells expressing PS1 having the D385A mutation were cultured in the presence or absence of a gamma-secretase inhibitor DAPT (1 microM). After cultivation, a p3-Alc_{beta} immunoprecipitate in the culture solution of the cells and a cell lysate were prepared according to the method described above and subjected to western blotting.

Likewise, Alcₐₗₚₕₐ-untransfected HEK293 cells, Alcₐₗₚₕₐ-transfected HEK293 cells expressing wild-type PS1, or Alcₐₗₚₕₐ-transfected HEK293 cells expressing PS1 having the D385A mutation were cultured in the presence or absence of a gamma-secretase inhibitor DAPT (1 microM). After cultivation, a p3-Alcₐₗₚₕₐ immunoprecipitate in the culture solution of the cells thus cultured and a cell lysate were prepared according to the method described above and subjected to western blotting.

The results are shown in Figure 11. The lane 1 shows the Alc_{beta} (left diagram) or Alcₐₗₚₕₐ (right diagram) untransfected HEK293 cells expressing wild-type PS1 (DAPT not added); the lane 2 shows the Alc_{beta} (left diagram) or Alcₐₗₚₕₐ (right diagram) transfected HEK293 cells expressing wild-type PS1 (DAPT not added); the lane 3 shows the Alc_{beta} (left diagram) or Alcₐₗₚₕₐ (right diagram) transfected HEK293 cells expressing wild-type PS1 (DAPT added); and the lane 4 shows the Alc_{beta} (left diagram) or Alcₐₗₚₕₐ (right diagram) transfected HEK293 cells expressing PS1 having the D385A mutation (DAPT not added). The expression of Alc_{beta} or Alcₐₗₚₕₐ was observed regardless of the presence or absence of PS1 having the D385A mutation or addition/non-addition of DAPT. In comparison between cells expressing the same levels of Alc_{beta}, the cells expressing PS1 having the D385A mutation secreted p3-Alc_{beta} approximately 24% of that by the control cells non-expressing PS1 having the D385A mutation (lane 4 in the left diagram of Figure 11). DAPT inhibited the production of p3-Alc_{beta} (lane 3 in the left diagram of Figure 11). The intracellular accumulation of Alc_{beta}CTF was observed both in the cells expressing PS1 having the D385A mutation and in the cells treated with DAPT (lanes 3 and 4 in the left diagram of Figure 11). In the similar experiment using Alcₐₗₚₕₐ expression, the production of p3-Alcₐₗₚₕₐ was completely inhibited in the cells expressing PS1 having the D385A mutation and the cells treated with DAPT (lanes 3 and 4 in the right diagram of Figure 11). AlcₐₗₚₕₐCTF accumulated both in the cells expressing PS1 having the D385A mutation and in the cells treated with DAPT. These results demonstrated that as with APP or other Alc family proteins (Alcₐₗₚₕₐ and Alc_{gamma}), Alc_{beta} undergoes main cleavage by a PS-dependent gamma-secretase complex.

Thus, in contrast to Alcₐₗₚₕₐ and Alc_{gamma}, Alc_{beta} exhibited unique properties during the course of production of p3-Alc_{beta} in cells expressing PS1 having the mutation linked to familial Alzheimer's disease. p3-Alcₐₗₚₕₐ and p3-Alc_{gamma} secreted from cells expressing the disease-linked PS1 mutant are extended at the C-terminus, whereas p3-Alc_{beta} secreted from the cells are shortened at the C-terminus. This suggested that the secondary cleavage of Alc_{beta} may be regulated by a mechanism different from that of for p3-Alcₐₗₚₕₐ and p3-Alc_{gamma}, although Alc_{beta}, as with the other Alc family proteins, undergoes coordinated metabolism with APP. In spite of these differences, the secondary cleavage was shown to be induced by primary cleavage. It was demonstrated that the formation of soluble Alc, p3-Alc, and AlcICD from Alc family proteins in neurons starts with primary cleavage by ADAM10 and/ ADAM17.

### Example 10. Secretion of p3-Alc into human cerebrospinal fluid

### (1) Obtainment of analytes and immunoprecipitation

In the first experiment using human analytes, p3-Alcₐₗₚₕₐ, p3-Alc_{beta}, and p3-Alc_{gamma} were collected from a mixture of human cerebrospinal fluids (0.3 to 1.0 mL) obtained from 5 to 10 human individuals (70 to 90 years old), by immunoprecipitation using the 150 microg of the affinity-purified 3B5 antibody (IgG fraction), 100 microL serum containing the anti-p3-Alc_{beta} polyclonal antibody UT143, and 100 microL serum containing the anti-p3-Alc_{gamma} polyclonal antibody UT166, respectively, and protein G Sepharose beads.

In the second experiment, the analytes used were cerebrospinal fluids obtained from 4 human patients with sporadic Alzheimer's disease and cerebrospinal fluids obtained from 3 humans of similar age without dementia as cohort controls. To detect p3-Alcₐₗₚₕₐ species in the cerebrospinal fluids, 800 microL each of the cerebrospinal fluids was individually subjected to immunoprecipitation in the same way as above.
The levels of amyloid beta42 (A-BETA42: Immuno-Biological Laboratories Co., Ltd.) and tau phosphorylated at threonine 181 (pTau) (Innogenetics) in the cerebrospinal fluids were quantified using an ELISA system (Sunderland, et al., (2003) JAMA 289, 2094-103).

In the third experiment, the analytes used were cerebrospinal fluids obtained from humans without dementia (controls) (CDR=0), patients suspected to have Alzheimer's disease (CDR=0.5), patients with sporadic Alzheimer's disease (CDR=1.0), patients with familial Alzheimer's disease having an A79V, H163R, or I143T mutation, and patients with other neurological and neurodegenerative diseases (OND) such as frontotemporal dementia (FTD), amyotrophic lateral sclerosis (ALS), and cerebral apoplexy. These analytes were individually subjected to immunoprecipitation in the same way as above.

### (2) Mass spectrometry

The beads were subsequently washed with a washing buffer I (10 mM Tris-HCl (pH 8.0), 140 mM NaCl, 0.1% (w/v) n-octyl-D-glucose, 0.025% (w/v) sodium azide) and a washing buffer II (10 mM Tris-HCl (pH 8.0), 0.025% (w/v) sodium azide). Then, the analytes were eluted with sinapinic acid saturated in trifluoroacetic acid/acetonitrile/water (1:20:20). The eluted analytes were dried on target plates and analyzed by MALDI-TOF/MS using Voyager-DE PRO MALDI-TOF Mass Spectrometer (PerSeptive Biosystems, Inc.) or Ultraflex II TOF/TOF (Bruker Daltonics Inc.). The molecular weights were calibrated using ProteoMass ACTH Fragment 18-39 MALDI-MS Standard and Insulin for Voyager-DE PRO MALDI-TOF Mass Spectrometer (Sigma-Aldrich, Inc.) or Peptide Calibration Standard for Ultraflex II TOF/TOF (Bruker Daltonics Inc.). The amino acid sequence of a peptide in each major peak was determined by MALDI-MS/MS analysis.

### (3) Addendum information

The human cerebrospinal fluids were used in the present study after obtainment of informed consent from the patients or patients' family members.

### (4) Results

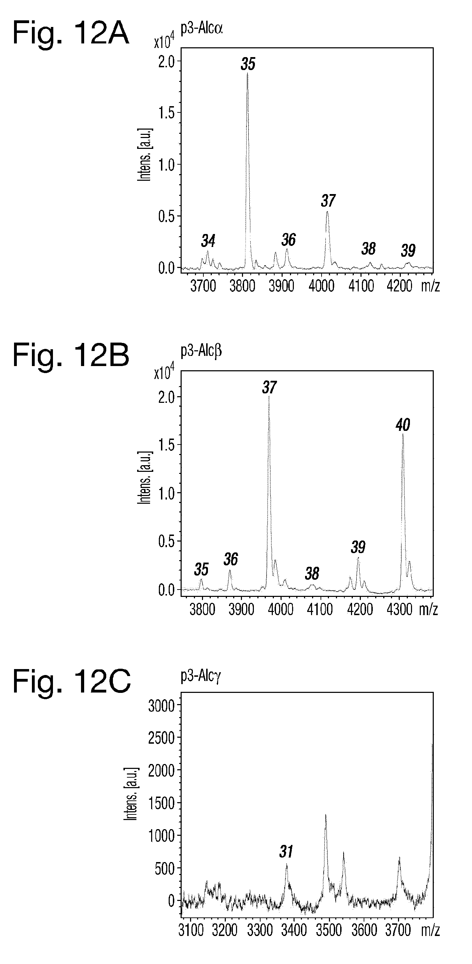

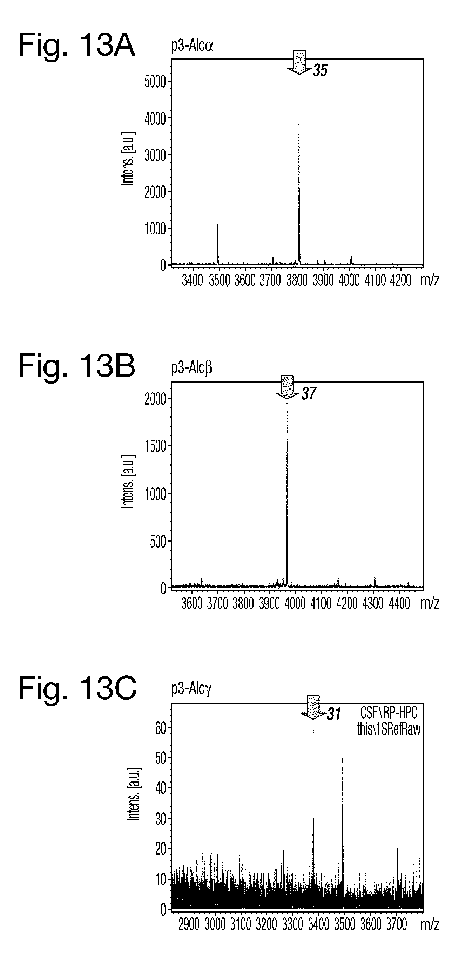

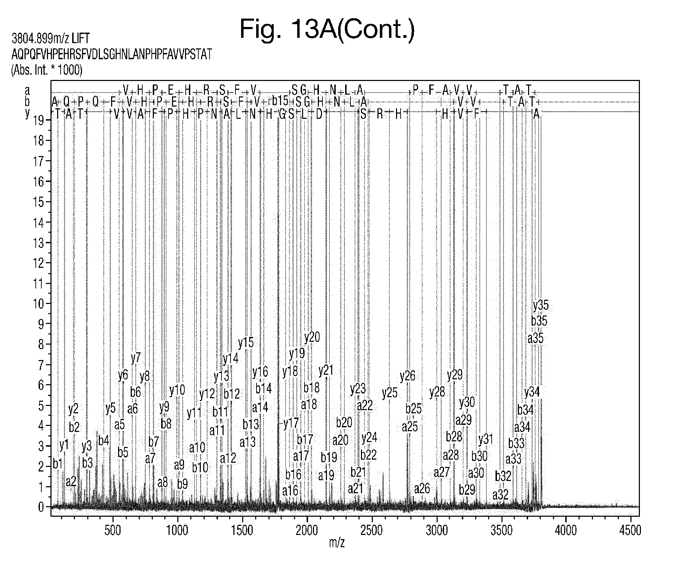

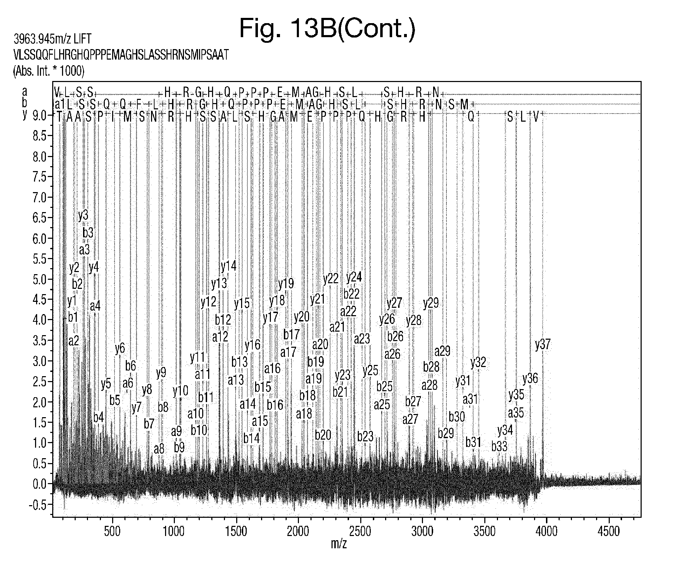

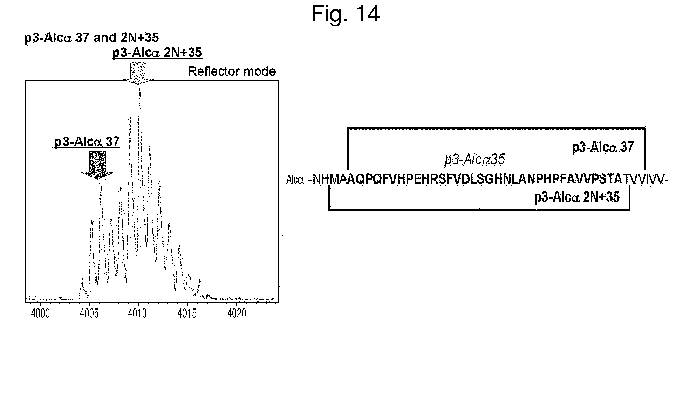

The mass spectrometry results of the first experiment are shown in Figure 12. A peptide with molecular weight of 3804.6 was collected by immunoprecipitation using the 3B5 antibody (Figure 12A). The collected peptide was confirmed by MALDI-MS/MS analysis to be p3-Alcₐₗₚₕₐ35 (Figure 13A) . Peptides collected with molecular weight of around 4000 were confirmed by MALDI-MS/MS analysis to be a peptide p3-Alcₐₗₚₕₐ37 comprising a sequence from alanine at residue 817 (Ala⁸¹⁷) to valine at residue 853 (Val⁸⁵³) and a peptide p3-AlcₐₗₚₕₐN2+35 comprising a sequence from methionine at residue 815 (Met⁸¹⁵) to threonine at residue 851 (Thr⁸⁵¹). These two peptides were distinguishable by analysis conducted in reflector mode (Figure 14). In the subsequent experiments, p3-Alcₐₗₚₕₐ37 was distinguished from p3-AlcₐₗₚₕₐN2+35 and assayed as an independent component, unless otherwise specified.
A peptide with molecular weight of 3963.9 was collected by immunoprecipitation using the UT143 antibody (Figure 12B). The collected peptide was confirmed by MALDI-MS/MS analysis to be p3-Alc_{beta}37 (Figure 13B). A peptide with molecular weight of 3377.6 was collected by immunoprecipitation using the UT166 antibody (Figure 12C). This species with molecular weight of 3377.6 was collected in a small amount by immunoprecipitation and therefore, could not be analyzed by MALDI-MS/MS. Its molecular weight was the same as that of p3-Alc_{gamma}31 (Figure 13C) .

These results demonstrated that cleavage sites in human Alc are the same as the sites determined by the *in-vitro* experiments described in the above Examples, and that p3-Alcₐₗₚₕₐ35, p3-Alcₐₗₚₕₐ37, p3-Alc_{beta}37, p3-Alc_{beta}40, and p3-Alc_{gamma}31 are formed as major species in humans.

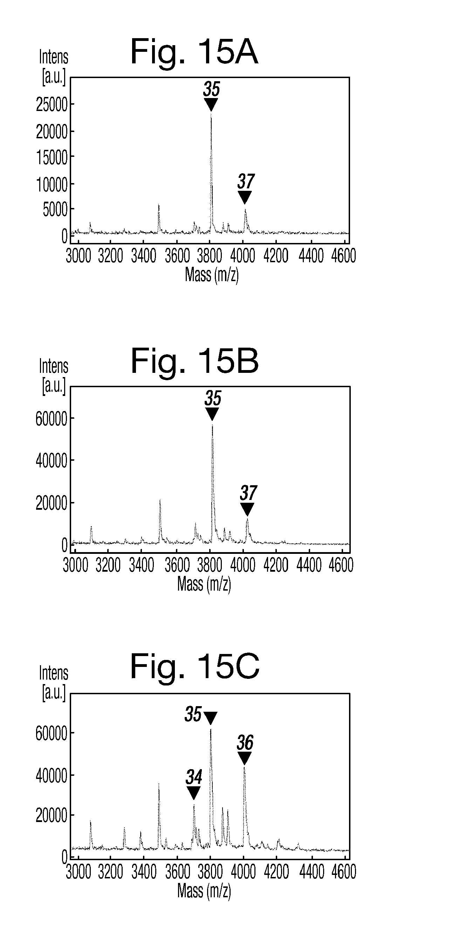

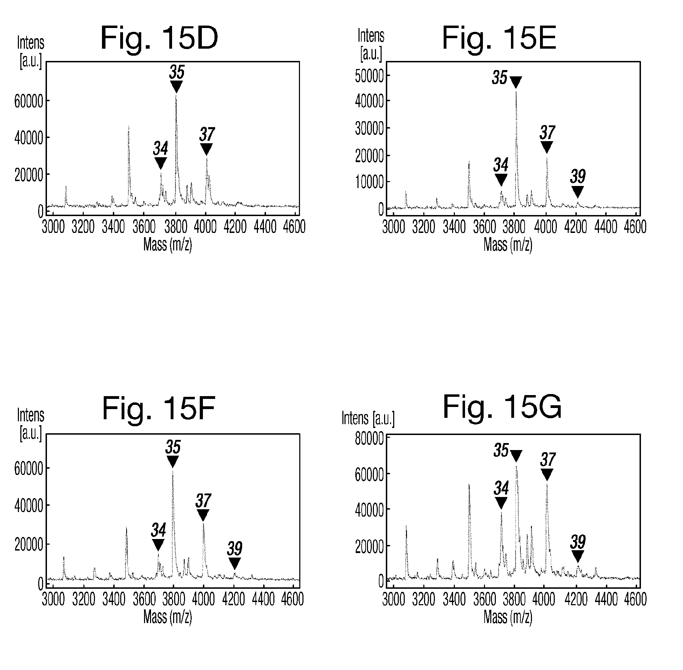

In the second experiment, the level of A-BETA42 in the cerebrospinal fluids obtained from 4 patients with Alzheimer's disease was lower than that observed in the control cerebrospinal fluids. The cerebrospinal fluids obtained from the Alzheimer's disease patients had A-BETA42 levels of 299 to 467 pg/mL and levels of tau phosphorylated at Thr¹⁸¹ (hereinafter, referred to as "pTau") of 68 to 135 pg/mL. On the other hand, the control cerebrospinal fluids had A-BETA42 levels of 760 to 898 pg/mL and pTau levels of 42 to 52 pg/mL. The major p3-Alcₐₗₚₕₐ species p3-Alcₐₗₚₕₐ35 and p3-Alcₐₗₚₕₐ37 were detected in the cerebrospinal fluids of 3 controls (Figures 15A to 15C) and the cerebrospinal fluids of 4 patients with Alzheimer's disease (Figures 15D to 15G). Although the peak "37" was presumed from the molecular weight to be p3-Alcₐₗₚₕₐ37, still this peak might contain p3-AlcₐₗₚₕₐN2+35. The peak "34" was detected in the cerebrospinal fluids of all the patients with Alzheimer's disease (Figures 15D to 15G) and the cerebrospinal fluid of one control (Figure 15C) and confirmed to be p3-Alcₐₗₚₕₐ34 having C-terminal shortening. The peak "39" was detected in the cerebrospinal fluids of 3 patients with Alzheimer's disease (Figures 15E to 15G) and determined to be p3-Alcₐₗₚₕₐ39 containing C-terminal extension. These p3-Alcₐₗₚₕₐ species shortened or extended at the C-terminus were not detected in the untransfected cells or the cells overexpressing wild-type PS1 (see Figure 6A). From these results, the presence of abnormal p3-Alcₐₗₚₕₐ species in a relatively large amount and/or increase in the levels of abnormal p3-Alcₐₗₚₕₐ species indicate that sporadic Alzheimer's disease is associated with the dysfunction of gamma-secretase. Particularly p3-Alcₐₗₚₕₐ34 and p3-Alcₐₗₚₕₐ39 exhibited remarkable peaks in the patients with Alzheimer's disease, demonstrating that they can be used as indexes for Alzheimer's disease.

The p3-Alc species obtained above were not observed to accumulate (not shown). Accordingly, it was demonstrated that change in the formation of p3-Alc species can be used as a surrogate marker for incorrectly processed A-BETA in the diagnosis of Alzheimer's disease. Specifically, the dysfunction of gamma-secretase characterizes the onset of sporadic Alzheimer's disease, and the formation of abnormal p3-Alc fragments serves as a surrogate marker for the dysfunction of gamma-secretase. Particularly, p3-Alcₐₗₚₕₐ34 and p3-Alcₐₗₚₕₐ39 were p3-Alcₐₗₚₕₐ species extended at the C-terminus and detected from the cerebrospinal fluids of sporadic Alzheimer's disease patients, which can be used as diagnostic markers for Alzheimer's disease.

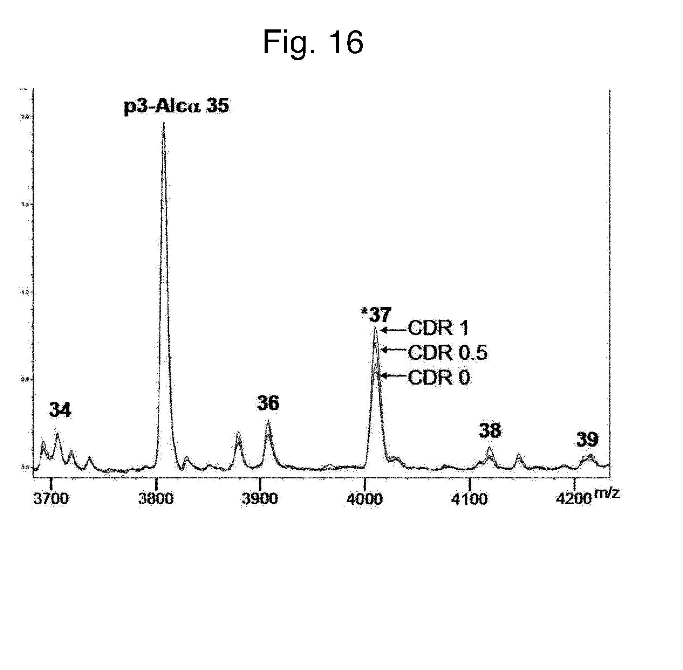

The results of assaying p3-Alcₐₗₚₕₐ in the third experiment are shown in Figure 16. As indicated with the arrows in Figure 16, the level of p3-Alcₐₗₚₙₐ37 was decreased in the following order: the sporadic Alzheimer's disease patients (CDR=1.0), the patients suspected to have Alzheimer's disease (CDR=0.5), and the humans without dementia (controls) (CDR=0). This demonstrates that p3-Alcₐₗₚₕₐ37 can be used as a diagnostic marker for Alzheimer's disease. According to the present experiment, the ratios of p3-Alcₐₗₚₕₐ34, p3-Alcₐₗₚₕₐ36, p3-Alcₐₗₚₕₐ37, p3-Alcₐₗₚₕₐ38, and p3-Alcₐₗₚₕₐ39 to p3-Alcₐₗₚₕₐ35 compared with the A-BETA42/40 ratio are shown in Figure 17. The A-BETA42/40 ratio in the cerebrospinal fluids obtained from the Alzheimer's disease patients (CDR=0.5 and CDR=1.0) and the patients having the PS1 mutation was lower than that observed in the cerebrospinal fluids of the controls (CDR=0). In Figure 17, the vertical dotted lines represent an average of the A-BETA42/40 ratio in the patients suspected to have Alzheimer's disease (CDR=0.5). The A-BETA42/40 ratio derived from the OND patients was also low, that demonstrates that reduction in A-BETA42 level can serve as an index for Alzheimer's disease but cannot distinguish between OND and Alzheimer's disease. The patients suspected to have Alzheimer's disease (CDR=0.5) and the sporadic Alzheimer's disease patients (CDR=1.0) exhibited high p3-Alcₐₗₚₕₐ34/p3-Alcₐₗₚₕₐ35 and p3-Alcₐₗₚₙₐ37/p3-Alcₐₗₚₕₐ35 ratios, compared to the OND patients. The horizontal dotted lines represent the maximum value of A-BETA42/40 in the humans without Alzheimer's disease. p3-Alcₐₗₚₕₐ34/p3-Alcₐₗₚₕₐ35 and p3-Alcₐₗₚₕₐ37/p3-Alcₐₗₚₕₐ35 in the patients suspected to have Alzheimer's disease (CDR=0.5) and the sporadic Alzheimer's disease patients (CDR=1.0) were distinguishable from those in the OND patients. This result suggests that patients having a low A-BETA42/40 ratio and a high p3-Alcₐₗₚₕₐ34/p3-Alcₐₗₚₕₐ35 or p3-Alcₐₗₚₕₐ37/p3-Alcₐₗₚₕₐ35 ratio do not have OND but possibly have Alzheimer's disease or will have it in the future. The patients having A79V, H163R, or I143T as a PS1 mutation exhibited the A-BETA42/40 ratio at an intermediate level on the vertical line and did not have high p3-Alcₐₗₚₕₐ34/p3-Alcₐₗₚₕₐ35 and p3-Alcₐₗₚₕₐ31/p3-Alcₐₗₚₕₐ35 ratios. The presence of abnormal p3-Alcₐₗₚₕₐ species and/or increase in the levels thereof revealed that sporadic Alzheimer's disease is associated with the general dysfunction of gamma-secretase, and this dysfunction influences not only APP but also the Alc family.

### Example 11. Assay of p3-Alcₐₗₚₕₐ35 using ELISA system

### (1) Obtainment of antigenic polypeptides

Antigenic polypeptides used were HPLC chromatography-purified polypeptides (a) and (b) consisting of an amino acid sequence shown below, which were purchased from Chinese Peptide Company (CPC, China). This polypeptide (a) comprises an amino acid sequence corresponding to residues 839 to 851 in p3-Alcₐₗₚₕₐ and cysteine (C) added to the N-terminus thereof. The polypeptide (b) comprises an amino acid sequence corresponding to residues 817 to 822 in p3-Alcₐₗₚₕₐ and cysteine (C) added to the C-terminus thereof.
Polypeptide (a): CNPHPFAVVPSTAT (SEQ ID NO: 26)
Polypeptide (b): AQPQFVC (SEQ ID NO: 27)

### (2) Preparation of polyclonal antibodies

A conjugate of the polypeptide (a) obtained in the above paragraph (1) and thyroglobulin was used as an antigen for immunization. Rabbits were immunized by administering 50 microl (100 mg/ml) of the conjugate solution at 1- or 2-week intervals. The antigen was mixed with a Freund's complete adjuvant only for the first immunization and mixed with a Freund's incomplete adjuvant for the rest of immunization. Then, the whole blood was collected from the rabbits and centrifuged at 1,500 rpm for 15 minutes to separate antiserum to obtain a rabbit polyclonal antibody (hereinafter, this antibody is referred to as an "Alc839 antibody").
A mouse polyclonal antibody was obtained in the same way as in the method of Alc839 antibody preparation except that: a conjugate of the polypeptide (b) obtained in the paragraph (1) and thyroglobulin was used as an antigen for immunization; and immunized animals used were mice (hereinafter, this antibody is referred to as an "Alc817 antibody").

### (3) Confirmation of antibody specificity by ELISA

To confirm the specificity of the Alc839 antibody obtained in the paragraph (2), ELISA was conducted. Specifically, antigenic peptides shown below were immobilized on a 96-well plate. The plate was reacted with the serially diluted antibody and then with horseradish peroxidase (HRP)-labeled anti-rabbit IgG antibodies and subsequently washed, followed by color development. The absorbance was detected at 490 nm to confirm the specificity.
Antigenic peptides:
gamma5 (KKCVPSTATVVIV) (SEQ ID NO: 28)
TS-C (8 mer) (KCVPSTAT) (SEQ ID NO: 29)
TS-C (11 mer) (CPSTATVVIVV) (SEQ ID NO: 30)

### (4) Results

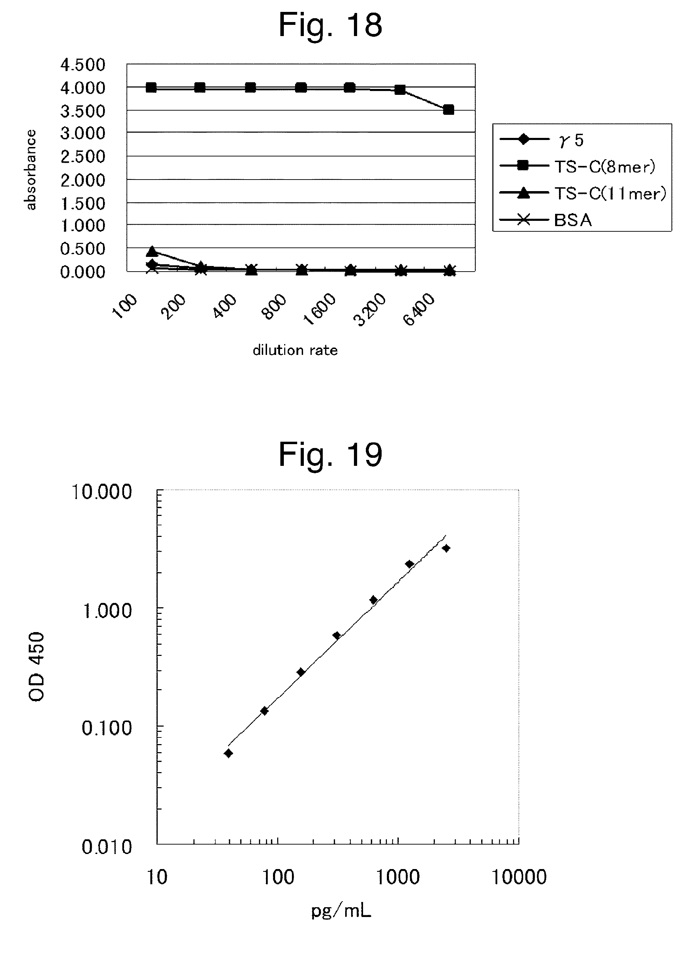

The results are shown in Figure 18. The present antibody strongly reacted with the peptide TS-C (8 mer) having the C-terminal fragment sequence of p3-Alcₐₗₚₕₐ35 but not with the other peptides gamma5 and TS-C (11 mer). This demonstrated that the antibody specifically reacts with the C-terminal of p3-Alcₐₗₚₕₐ35.

### Example 12. Construction of sandwich ELISA

### (1) Preparation of conjugate of Alc817 antibody and HRP

A conjugate of the Alc817 antibody obtained in Example 11 and HRP was prepared as follows. A necessary amount of HRP was dissolved in distilled water, then oxidized with NaIO₄, and then dialyzed overnight against a 1 mM acetate buffer at pH 4.4. 2 mg of the Alc817 antibody was also dialyzed overnight against a 0.1 M carbonate buffer at pH 9.5. The dialyzed Alc817 antibody and HRP were mixed in an amount of 0.4 mg of HRP with respect to 1 mg of the antibody and reacted at room temperature for 2 hours. To this reaction mixture, NaBH₄ was subsequently added, and the mixture was reacted for 2 hours in ice and then dialyzed overnight against PBS. Furthermore, this reaction product was gel-filtered to prepare a conjugate of the Alc817 antibody and HRP (hereinafter, this conjugate is referred to as a "labeled Alc817 antibody").

### (2) Preparation of sandwich ELISA plate

The Alc839 antibody (10 microg/ml) was added at a concentration of 100 microl/well to a 96-well ELISA plate. Subsequently, this plate was reacted overnight at 4°C and then blocked with a 1% BSA/PBS/NaN₃ solution to prepare an Alc839 antibody-immobilized sandwich ELISA plate. The conjugate of the Alc817 antibody and HRP prepared in the preceding paragraph (1) was used as a labeled antibody.

### (3) Preparation of standard curve and results

p3-Alcₐₗₚₕₐ35 was assayed using the 96-well ELISA plate prepared in the paragraph (2), the labeled Alc817 antibody prepared in the paragraph (1), and 35-mer alcadein peptides (SEQ ID NO: 11) as standards. The results are shown in Figure 19. The p3-Alcₐₗₚₕₐ35 assay with the Alc839 antibody and the labeled Alc817 antibody exhibited favorable linearity in each concentration-dependent manner.

### Example 12. p3-Alcₐₗₚₕₐ35 in cerebrospinal fluids of familial AD patients

The ELISA system prepared in Example 11 was used to determine the quantity of p3-Alcₐₗₚₕₐ35 in the cerebrospinal fluids of 5 patients with familial Alzheimer's disease (one patient having an I143T mutation in gamma-secretase, two patients having an A79V mutation in gamma-secretase, and two patients having an H163R mutation in gamma-secretase), 16 patients with OND (10 patients with Parkinson's disease, two patients with cerebral apoplexy, 3 patients with FTD, and one patient with ALS), and 26 healthy subjects.

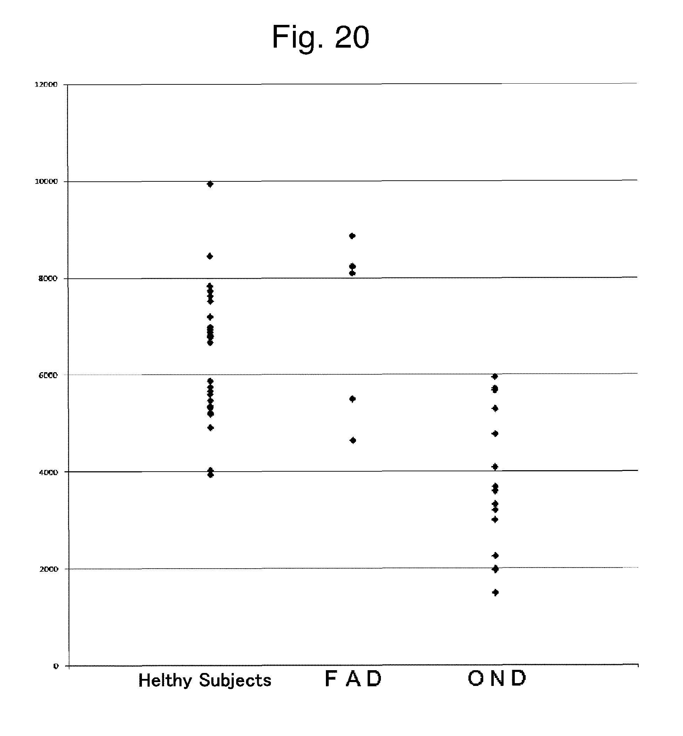

The results are shown in Figure 20. As shown in Figure 20, the familial Alzheimer's disease patients showed tendency to secrete a large amount of p3-Alcₐₗₚₕₐ35. This tendency was not observed in the healthy sebujects or the OND patients, demonstrating that p3-Alcₐₗₚₕₐ35 can serve as a biomarker for Alzheimer's disease.

In this context, SEQ ID NOs and sequences used herein are as shown below.

**[Table 1]**

| SEQ ID NO | NAME | SEQUENCE |
|---|---|---|
| 1 | hAlcₐ₁ (GenBank Accesion NO AY75330 1) | MLRRPAPALAPAARLLLAGLLCGGGVWAARVNKHKPWLEPTYHGIVTENDNTV LLDPPLIALDKDAPLRFAGEICGFKIHGQNVPFDAVVVDKSTGEGVIRSKEKLDC ELQKDYSFTIQAYDCGKGPDGTNVKKSHKATVHIQVNDVNEYAPVFKEKSYKA TVIEGKQYDSILRVEAVDADCSPQFSQICSYEIITPDVPFTVDKDGYIKNTEKLNY GKEHQYKLTVTAYDCGKKRATEDVLVKISIKPTCTPGWQGWNNRIEYEPGTGAL AVFPNIHLETCDEPVASVQATVELETSHIGKGCDRDTYSEKSLHRLCGAAAGTAE LLPSPSGSLNWTMGLPTDNGHDSDQVFEFNGTQAVRIPDGVVSVSPKEPFTISVW MRHGPFGRsKETILCSSDKTDMNRHHYSLYVHGCRLIFLFRQDPSEEKKYRPAEF HWKLNQVCDEEWHHYVLNVEFPSVTLYVDGTSHEPFSVTEDYPLHPSKIETQLV VGACWQEFSGVENDNETEPVTVASAGGDLHMTQFFRGNLAGLTLRSGKLADK KVIDCLYTCKEGLDLQVLEDSGRGVQIQAHPSQLVLTLEGEDLGELDKAMQHIS YLNSRQFPTPGIRRLKITSTIKCFNEATCISVPPVDGYVMVLQPEEPKISLSGVHHF ARAASEFESSEGVFLFPELRIISTITREVEPEGDGAEDPTVQESLVSEEIVHDLDTC EVTVEGEELNHEQESLEVDMARLQQKGIEVSSSELGMTFTGVDTMASYEEVLII LLRYRNWHARSLLDRsFKLICSELNGRYISNEFKVEVNVIHTANPMEHANHMAA QPQFVHPEHRSFVDLSGHNLANPHPFAVVPSTATVVIVVCVSFLVFMIILGVFRIR AAHRRTMRDQDTGKENEMDWDDSALTITVNPMETYEDQHSSEEEEEEEEEEES EDGEEEDDITSAESESSEEEEGEQGDPQNATRQQQLEWDDSTLSY |
| 2 | hA1c_{β} (GenBank Accesion NO NM._014 718) | MTLLLLPLLLASLLASCSCNKANKHKPWIEAEYQGIVMENDNTVLLNPPLFALD KDAPLRYAGEICGFRLHGSGVPFEAVILDKATGEGLIRAKEPVDCEAQKEHTFTIQ AYDCGEGPDGANTKKSHKATVHVRVNDVNEFAPVFVERLYRAAVTEGKLYDRI LRVEAIDGDCSPQYSQICYYEILTPNTPFLIDNDGNIENTEKLQYSGERLYKFTVTA YDCGKKRAADDAEVEIQVKPTCKPSWQGWNKRIEYAPGAGSLALFPGIRLETC DEPLWNIQATIELQTSHVAKGCDRDNYSERALRKLCGAATGEVDLLPMPGPNAN WTAGLSVHYSQDSSLIYWFNGTQAVQVPLGGPSGLGSGPQDSLSDHFTLSFWM KHGVTPNKGKKEEETIVCNTVQNEDGFSHYSLTVHGCRIAFLYWPLLESARPVK FLWKLEQVCDDEWHHYALNLEFPTVTLYTDGISFDPALIHDNGLIHPPRREPALM IGACWTEEKNKEKEKGDNSTDTTQGDPLSIHHYFHGYLAGFSVRSGRLESREVI ECLYACREGLDYRDFESLGKGMKVHVNPSQSLLTLEGDDVETFNHALQHVAYM NTLRFATPGVRPLRLTTAVKCFSEESCVSIPEVEGYVVVLQPDAPQILLSGTAHFA RPAVDFEGTNGVPLFPDLQITCSISHQVEAKKDESWQGTVTDTRMSDEIVHNLD GCEISLVGDDLDPERESLLLDTTSLQQRGLELTNTSAYLTIAGVESITVYEEILRQA RYRLRHGAALYTRKFRLSCSEMNGRYSSNEFIVEVNVLHSMNRVAHPSHVLSSQ QFLHRGHQPPPEMAGHSLASSHRNSMIPSAATLIIVVCVGFLVLMVVLGLVRIHS LHRRVSGAGGPPGASSDPKDPDLFWDDSALTIIVNPMESYQNRQSCVTGAVGGQ QEDEDSSDSEVADSPSSDERRHETPPHRY |
| 3 | hAlc_{γ} (GenBank Accesion NO NM_022 131) | MLPGRLCWVPLLLALGVGSGSGGGGDSRQRRLLAAKVNKHKPWIETSYHGVIT ENNDTVILDPPLVALDKDAPVPFAGEICAFKIHGQELPFEAVVLNKTSGEGRLRA KSPIDCELQKEYTFIIQAYDCGAGPHETAWKKSHKAVVHIQVKDVNEFAPTFKEP AYKAVVTEGKIYDSILQVEAIDEDCSPQYSQICNYEIVTTDVPFAIDRNGNIRNTE KLSYDKQHQYEILVTAYDCGQKPAAQDTLVQVDVKPVCKPGWQDWTKRIEYQP GSGSMPLFPSIHLETCDGAVSSLQIVTELQTNYIGKGCDRETYSEKSLQKLCGASS GIIDLLPSPSAATNWTAGLLVDSSEMIFKFDGRQGAKIPDGIVPKNLTDQFTITMW MKHGPSPGVRAEKETILCNSDKTEMNRHHYALYVHNCRLVFLLRKDFDQADTF RPAEFHWKLDQICDKEWHYYVINVEFPVVTLYMDGATYEPYLVTNDWPIHPSHI AMQLTVGACWQGGEVTKPQFAQFFHGSLASLTIRPGKMESQKVISCLQACKEGL DINSLESLGQGIKYHFNPSQSILVMEGDDIGNINRALQKVSYINSRQFPTAGVRRL KVSSKVQCFGEDVCISIPEVDAYVMVLQAIEPRITLRGTDHFWRPAAQFESARGV TLFPDIKIVSTFAKTEAPGDVKTTDPKSEVLEEMLHNLDFCDILVIGGDLDPRQEC LELNHSELHQRHLDATNSTAGYSIYGVGSMSRYEQVLHHIRYRNWRPASLEARR FRIKCSELNGRYTSNEFNLEVSILHEDQVSDKEHVNHLIVQPPFLQSVHHPESRSS IQHSSVVPSIATVVIIISVCMLVFVVAMGVYRVRIAHQHFIQETEAAKESEMDWD DSALTITVNPMEKHEGPGHGEDETEGEEEEEAEEEMSSSSGSDDSEEEEEEEGMG RGRHGQNGARQAQLEWDDSTLPY |

**[Table 2]**

| SEQ ID NO | NAME | SEQUENCE |
|---|---|---|
| 4 | Human Presenilin 1cDNA (GenBank Accesion NO NM_000 021) | MTELPAPLSYFQNAQMSEDNHLSNTVRSQNDNRERQEHNDRRSLGHPE PLSNGRPQGNSRQVVEQDEEEDEELTLKYGAKHVIMLFVPVTLCMVVV VATIKSVSFYTRKDGQLIYTPFTEDTETVGQRALIISILNAAIMISVIVVM TILLVVLYKYRCYKVIHAWLIISSLLLLFFFSFIYLGEVFKTYNVAVDYIT VALLIWNFGVVGMISIHWKGPLRLQQAYLIMISALMALVFIKYLPEWTA WLILAVISVYDLVAVLCPKGPLRMLVETAQERNETLFPALIYSSTMVWL VNMAEGDPEAQRRVSKNSKYNAESTERESQDTVAENDDGGFSEEWEA QRDSHLGPHRSTPESRAAVQELSSSILAGEDPEERGVKLGLGDFIFYSVL VGKASATASGDWNTTIACFVAILIGLCLTLLLLAIFKKALPALPISITFGL VFYFATDYLVQPFMDQLAFHQFYI |
| 5 | UT134 Antigen | FVHPEHRSFVDLSGHNLANPHPFC |
| 6 | UT135 Antigen | NPHPFAVVPSTATC |
| 7 | 3B5 Antigen | CFVHPEH |
| 8 | UT142 Antigen | SSHRNSMIPSAATC |
| 9 | UT143 antigen | FLHRGHQPPPEMAGIISLASSIIRNSMIPSA |
| 10 | UT166 Antigen | CIQHSSVVPSIAT |
| 11 | p3-Alc _{α}35 | AQPQFVHPEHRSFVDLSGHNLANPHPFAVVPSTAT |
| 12 | p3-A1c _{α}2N+35 | MAAQPQFVHPEHRSFVDLSGHNLANPHPFAVVPSTAT |
| 13 | p3-A1c _{β}37 | VLSSQQFLHRGHQPPPEMAGIISLASSHRNSMIPSAAT |
| 14 | p3 A1c _{β}40 | VLSSQQFLIIRGIIQPPPEMAGHSLASSHRNSMIPSAATLH |
| 15 | p3 - A1c _{γ}31 | LIVQPPFLQSVHSVHIIPESRSSIQHSSVVPSIAT |
| 16 | p3 - A1c _{γ}34 | LIVQPPFLQSVHHPESRSSIQHSSVVPSIATVVI |
| 17 | A1c_{γ}CT F | LIVQPPFLQ |
| 18 | p3-A1c _{α}2N + 38 | MAAQPQFVHPEHRSFVDLSGHNLANPHPFAVVPSTAVVI |
| 19 | p3-A1c _{α}2 N + 39 | MAAQPQFVHPEHRSFVDLSGHNLANPHPFAVVPSTATV |
| 20 | p3-A1c _{α}2 N + 40 | MAAQPQFVIIPEHRSFVDLSGHNLANPHPFAVVIVV |
| 21 | p3 - A1c _{α}34 | AQPQFVHPEHRSFVDLSGHNLANPHPFAVVPSTA |
| 22 | p3 - A1c _{α}36 | AQPQFVHPEHRSFVDLSGHNLANPHPFAVVPSTATV |
| 23 | p3 - A1c _{α}37 | AQPQFVHPEHRSFVDLSGHNLANPHPFAVVPSTATVV |
| 24 | p3 - A1c _{α}38 | AQPQFVHPEHRSFVDLSGHNLANPHPFAVVPSTATVVI |
| 25 | p 39 - A1c _{α}39 | AQPQFVHPEHRSFVDLSGHNLANPHPFAVVPSTATVVIV |

**[Table 3]**

| SEQ ID NO | NAME | SEQUENCE |
|---|---|---|
| 26 | A1c839 Antigen | CNPHPFAVVPSTAT |
| 27 | A1c817 Antigen | AQPQFVC |
| 28 | Antigen Peptide γ5 | KKCVPSTATVVIV |
| 29 | Antigen PeptideTS-C (8mer) | KCVPSTAT |
| 30 | Antigen PeptideTS-C (11mer) | CPSTATVVIVV |

### Industrial Applicability

A biomarker of the present invention serves as an index for a disease associated with the abnormality in gamma-secretase. Therefore an agent, an apparatus and a method for diagnosis of the present invention can be used for diagnosing a disease associated with the abnormality in gamma-secretase, providing information for the diagnosis of a disease associated with the abnormality in gamma-secretase, monitoring the condition or the degree of progression of a disease associated with the abnormality in gamma-secretase, and determining the therapeutic effect of a therapeutic agent on a disease associated with the abnormality in gamma-secretase.

The present invention also relates to the following aspects:
1. A diagnostic agent for a disease associated with the abnormality in gamma-secretase, comprising an antibody or a fragment thereof which recognizes an alpha-secretase and gamma-secretase digestion product of an alcadein.
2. The diagnostic agent for a disease associated with the abnormality in gamma-secretase of aspect 1, wherein the diagnostic agent is used for assaying the alpha-secretase and gamma-secretase digestion product of an alcadein in the body fluid of a subject.
3. The diagnostic agent for a disease associated with the abnormality in gamma-secretase of aspect 1 or 2, wherein the alpha-secretase and gamma-secretase digestion product of an alcadein is a peptide consisting of the amino acid sequence of any one of SEQ ID NOs: 11 to 16 and SEQ ID NOs: 18 to 25.
4. The diagnostic agent for a disease associated with the abnormality in gamma-secretase of any one of aspects 1 to 3, wherein the antibody is an antibody which recognizes the amino acid sequence of SEQ ID NO: 5, the amino acid sequence of SEQ ID NO: 6, the amino acid sequence of SEQ ID NO: 7, the amino acid sequence of SEQ ID NO: 8, the amino acid sequence of SEQ ID NO: 9, the amino acid sequence of SEQ ID NO: 10, the amino acid sequence of SEQ ID NO: 26, or the amino acid sequence of SEQ ID NO: 27 as an epitope.
5. The diagnostic agent of any one of aspects 1 to 4, wherein the disease associated with the abnormality in gamma-secretase is Alzheimer's disease.
6. A diagnostic apparatus for a disease associated with the abnormality in gamma-secretase, comprising means of separating an alpha-secretase and gamma-secretase digestion product of an alcadein from an analyte and means of detecting information about the amino acid sequence of the separated alpha-secretase and gamma-secretase digestion product of an alcadein.
7. A peptide consisting of the amino acid sequence of any one of SEQ ID NOs: 11 to 16 and SEQ ID NOs: 18 to 25.
8. An antibody or a fragment thereof which recognizes the amino acid sequence of SEQ ID NO: 5, the amino acid sequence of SEQ ID NO: 6, the amino acid sequence of SEQ ID NO: 7, the amino acid sequence of SEQ ID NO: 8, the amino acid sequence of SEQ ID NO: 9, the amino acid sequence of SEQ ID NO: 10, the amino acid sequence of SEQ ID NO: 26, or the amino acid of SEQ ID NO: 27 as an epitope.
9. A diagnostic method for a disease associated with the abnormality in gamma-secretase, comprising the step of detecting an alpha-secretase and gamma-secretase digestion product of an alcadein in an analyte.
10. A diagnostic method for a disease associated with the abnormality in gamma-secretase, comprising the following steps:
   a) preparing a sample derived from a subject;
   b) contacting the sample with an antibody or a fragment thereof which recognizes at least one alpha-secretase and gamma-secretase digestion product of an alcadein;
   c) determining the level of the alpha-secretase and gamma-secretase digestion product of an alcadein by detecting the binding of the antibody or the fragment thereof to the alpha-secretase and gamma-secretase digestion product of an alcadein; and
   d) associating the level of the alpha-secretase and gamma-secretase digestion product of an alcadein with the presence or absence of or the severity of the disease associated with the abnormality in gamma-secretase in the subject.
11. A diagnostic method for a disease associated with the abnormality in gamma-secretase, comprising the following steps:
   a) preparing a sample derived from a subject;
   b) separating an alpha-secretase and gamma-secretase digestion product of an alcadein;
   c) detecting information about the amino acid sequence of the separated alpha-secretase and gamma-secretase digestion product of an alcadein; and
   c) associating the level of the alpha-secretase and gamma-secretase digestion product of an alcadein with the presence or absence of or the severity of the disease associated with the abnormality in gamma-secretase in the subject.
12. The diagnostic method for a disease associated with the abnormality in gamma-secretase of any one of aspects 9 to 11, wherein the alpha-secretase and gamma-secretase digestion product of an alcadein is a peptide consisting of the amino acid sequence of any one of SEQ ID NOs: 11 to 16 or SEQ ID NOs: 18 to 25.
13. A diagnostic method for a disease associated with the abnormality in gamma-secretase, comprising the following steps:
   a) preparing a sample derived from a subject;
   b) contacting the sample with an antibody or a fragment thereof which recognizes a peptide consisting of the amino acid sequence of SEQ ID NO: 11 or 12 and with an antibody or a fragment thereof which recognizes a peptide consisting of the amino acid sequence of any one of SEQ ID NOs: 18 to 25;
   c) determining the levels of the peptide consisting of the amino acid sequence of SEQ ID NO: 11 or 12 and the peptide consisting of the amino acid sequence of any one of SEQ ID NOs: 18 to 25 by detecting the binding of the antibody or the fragment thereof to the peptide consisting of the amino acid sequence of SEQ ID NO: 11 or 12 and the peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
14. A diagnostic method for a disease associated with the abnormality in gamma-secretase, comprising the following steps:
   a) preparing a sample derived from a subject;
   b) separating a peptide consisting of the amino acid sequence of SEQ ID NO: 11 or 12 and a peptide consisting of the amino acid sequence of any one of SEQ ID NOs: 18 to 25;
   c) detecting information about the amino acid sequences of the separated peptide consisting of the amino acid sequence of SEQ ID NO: 11 or 12 and the separated peptide consisting of the amino acid sequence of any one of SEQ ID NOs: 18 to 25;
   d) calculating the quantitative ratio between the peptide consisting of the amino acid sequence of any one of SEQ ID NOs: 18 to 20 and the peptide consisting of the amino acid sequence of SEQ ID NO: 12 or the quantitative ratio between the peptide consisting of the amino acid sequence of any one of SEQ ID NOs: 21 to 25 and the peptide consisting of the amino acid sequence of SEQ ID NO: 11; and
   e) associating the calculated ratio with the presence or absence of or the severity of the disease associated with the abnormality in gamma-secretase in the subject.
15. The diagnostic method for a disease associated with the abnormality in gamma-secretase of aspect 11 or 12, further comprising the following steps:
   i) detecting amyloid beta42 and amyloid beta40 in the analyte;
   ii) calculating the ratio of the amyloid beta42 to the amyloid beta40 detected;
   iii) calculating the quantitative ratio between the peptide consisting of the amino acid sequence of any one of SEQ ID NOs: 18 to 25 and the peptide consisting of the amino acid sequence of SEQ ID NO: 11; and
   iv) associating the ratio of the amyloid beta42 to the amyloid beta40 and the quantitative ratio between the peptide consisting of the amino acid sequence of any one of SEQ ID NOs: 18 to 25 and the peptide consisting of the amino acid sequence of SEQ ID NO: 11 with the presence or absence of or the severity of the disease associated with the abnormality in gamma-secretase in the subject.
16. The diagnostic method for a disease associated with the abnormality in gamma-secretase of any one of aspects 9 to 15, wherein the analyte is the body fluid of the subject.
17. The diagnostic method for a disease associated with the abnormality in gamma-secretase to any one of aspects 9 to 16, wherein the disease associated with the abnormality in gamma-secretase is Alzheimer's disease.
18. A method for screening a therapeutic or preventive agent for a disease associated with the abnormality in gamma-secretase, comprising the steps of: administering a test agent; and detecting an alpha-secretase and gamma-secretase digestion product of an alcadein in an analyte.

## Claims

1. A method for diagnosing Alzheimer's disease, comprising the step of detecting an alpha-secretase and gamma-secretase digestion product of an alcadein-beta in an analyte from a subject, wherein the alpha-secretase and gamma-secretase digestion product of an alcadein-beta is a peptide consisting of the amino acid sequence of SEQ ID NO: 13 or SEQ ID NO: 14.

2. The method of claim 1, wherein said method uses a diagnostic agent comprising an antibody or a fragment thereof which specifically recognizes the peptide consisting of the amino acid sequence of SEQ ID NO: 13 or SEQ ID NO: 14.

3. The method of claim 2, comprising the following steps:
(a) contacting a sample from a subject with an antibody or a fragment thereof which recognizes the peptide consisting of the amino acid sequence of SEQ ID NO: 13 or SEQ ID NO: 14;
(b) determining the level of the peptide consisting of the amino acid sequence of SEQ ID NO: 13 or SEQ ID NO: 14 by detecting the binding of the antibody or the fragment thereof to the peptide consisting of the amino acid sequence of SEQ ID NO: 13 3 or SEQ ID NO: 14; and
(c) associating the level of the peptide consisting of the amino acid sequence of SEQ ID NO: 13 or SEQ ID NO: 14 with the presence or absence of or the severity of Alzheimer's disease in the subject.

4. The method of claim 1, comprising the following steps:
(a) separating the amino acid sequence of SEQ ID NO: 13 or SEQ ID NO: 14 in a sample from a subject;
(b) detecting information about the amino acid sequence of SEQ ID NO: 13 or SEQ ID NO: 14; and
(c) associating the level of the amino acid sequence of SEQ ID NO: 13 or SEQ ID NO: 14 with the presence or absence of or the severity of Alzheimer's disease in the subject.

5. The method of claim 1, comprising the following steps:
(a) contacting a sample from a subject with an antibody or a fragment thereof which recognizes a peptide consisting of the amino acid sequence of SEQ ID NO: 13 and with an antibody or a fragment thereof which recognizes a peptide consisting of the amino acid sequence of SEQ ID NO: 14;
(b) determining the levels of the peptide consisting of the amino acid sequence of SEQ ID NO: 13 and the peptide consisting of the amino acid sequence of SEQ ID NO:14. by detecting the binding of the antibody or the fragment thereof to the peptide consisting of the amino acid sequence of SEQ ID NO: 13 and the peptide consisting of the amino acid sequence of SEQ ID NO: 14;
(c) calculating the quantitative ratio between the peptide consisting of the amino acid sequence of any one of SEQ ID NO: 14 and the peptide consisting of the amino acid sequence of SEQ ID NO: 13; and
(d) associating the calculated ratio with the presence or absence of or the severity of Alzheimer's disease in the subject.

6. The method of claim 1, comprising the following steps:
(a) separating a peptide consisting of the amino acid sequence of SEQ ID NO: 13 and a peptide consisting of the amino acid sequence of SEQ ID NO: 14 in a sample from a subj ect;
(b) detecting information about the amino acid sequences of the separated peptide consisting of the amino acid sequence of SEQ ID NO: 13 and the separated peptide consisting of the amino acid sequence of any one of SEQ ID NO: 14;
(c) calculating the quantitative ratio between the peptide consisting of the amino acid sequence of SEQ ID NO: 14 and the peptide consisting of the amino acid sequence of SEQ ID NO: 13; and
(d) associating the calculated ratio with the presence or absence of or the severity of Alzheimer's disease in the subject.

7. The method of claim 5, further comprising the following steps:
(i) detecting amyloid beta42 and amyloid beta40 in the analyte;
(ii) calculating the ratio of the amyloid beta42 to the amyloid beta40 detected;
(iii) calculating the quantitative ratio between the peptide consisting of the amino acid sequence of SEQ ID NOs: 14 and the peptide consisting of the amino acid sequence of SEQ ID NO: 13; and
(iv) associating the ratio of the amyloid beta42 to the amyloid beta40 and the quantitative ratio between the peptide consisting of the amino acid sequence of SEQ ID NO: 14 and the peptide consisting of the amino acid sequence of SEQ ID NO: 13 with the presence or absence of or the severity of Alzheimer's disease in the subject.

8. The method of any one of claims 1 to 7, wherein the analyte is the body fluid of the subj ect.

9. A diagnostic apparatus for use in a method according to any of claims 1 to 8, comprising means of separating the peptide consisting of the amino acid sequence of SEQ ID NO: 13 or SEQ ID NO: 14 from an analyte and means of detecting information about the peptide consisting of the amino acid sequence of SEQ ID NO: 13 or SEQ ID NO: 14.

10. A peptide consisting of the amino acid sequence of SEQ ID NOs: 13 or SEQ ID NO: 14.

11. An antibody or a fragment thereof which recognizes the amino acid sequence of SEQ ID NO: 13, or the amino acid sequence of SEQ ID NO: 14 as an epitope.

12. A method for screening a therapeutic or preventive agent for Alzheimer's disease, comprising detecting the peptide consisting of the amino acid sequence of SEQ ID NO: 13 or SEQ ID NO: 14 in an analyte from an animal that has been administered a test agent.
